# EUROPEAN PATENT SPECIFICATION

(11) **EP 2 989 893 B1**
(45) Date of publication and mention of the grant of the patent: **19.06.2019**
(21) Application number: 15186515.1
(22) Date of filing: 24.04.2012
(51) Int. Cl.: A01K 67/027, C07K 16/00, C07K 16/46, C12N 15/85

(54) **NON-HUMAN ANIMALS EXPRESSING ANTIBODIES HAVING A COMMON LIGHT CHAIN**
NICHT-MENSCHLICHE TIERE, DIE ANTIKÖRPER MIT EINER GEMEINSAMEM LEICHTE KETTE EXPRIMIEREN
ANIMAUX NON HUMAINS EXPRIMANT DES ANTICORPS PRÉSENTANT UNE CHAÎNE LÉGÈRE COMMUNE

(30) Priority: 25.04.2011 US 201113093156
(43) Date of publication of application: 02.03.2016
(62) Divisional of application: 12717033.0
(73) Proprietor: Regeneron Pharmaceuticals, Inc., Tarrytown, NY 10591 (US)
(72) Inventor: MCWHIRTER, John, Tarrytown, NY 10591 (US); MACDONALD, Lynn, Harrison, NY 10528 (US); STEVENS, Sean, San Francisco, CA 94158 (US); DAVIS, Samuel, New York, NY 10024 (US); BUCKLER, David R., Sleepy Hollow, NY 10591 (US); HOSIAWA, Karolina A., Yorktown Heights, NY 10598 (US); MURPHY, Andrew J., Croton-on-Hudson, NY 10520 (US)
(74) Representative: J A Kemp

(56) References cited:
- WO-A1-2011/097603
- WO-A2-2004/009618
- US-A1- 2006 015 957
- JAKOBOVITS AYA ET AL: "From XenoMouse technology to panitumumab, the first fully human antibody product from transgenic mice", NATURE BIOTECHNOLOGY, NATURE PUBLISHING GROUP, NEW YORK, NY, US, vol. 25, no. 10, 1 October 2007 (2007-10-01), pages 1134-1143, XP002556637, ISSN: 1087-0156, DOI: 10.1038/NBT1337 [retrieved on 2007-10-05]
- MARVIN JONATHAN S ET AL: "Recombinant approaches to IgG-like bispecific antibodies", ACTA PHARMACOLOGICA SINICA, NATURE PUBLISHING GROUP, US, CN, vol. 26, no. 6, 1 June 2005 (2005-06-01), pages 649-658, XP002412036, ISSN: 1671-4083, DOI: 10.1111/J.1745-7254.2005.00119.X
- NICHOLSON I C ET AL: "Antibody repertoires of four- and five-feature translocus mice carrying human immunoglobulin heavy chain and kappa and lambda light chain yeast artificial chromosomes", THE JOURNAL OF IMMUNOLOGY, THE AMERICAN ASSOCIATION OF IMMUNOLOGISTS, US, vol. 163, 1 January 1999 (1999-01-01), pages 6898-6906, XP002974255, ISSN: 0022-1767

### SEQUENCE LISTING

<110> MCWHIRTER, John MACDONALD, Lynn STEVENS, Sean DAVIS, Samuel BUCKLER, David R. HOSIAWA, Karolina Anna MURPHY, Andrew J.
<120> Non-Human Animals Expressing
   Antibodies Having A Common Light Chain
<130> 802B-WO
<140> To be assigned
   <141>
<150> 13/093,156
   <151> 2012-04-25
<160> 33
<170> FastSEQ for Windows Version 4.0
<210> 1
   <211> 3155
   <212> DNA
   <213> Artificial Sequence
<220>
   <223> Synthetic
<400> 1
   ggcgcgccgt agctttgaat tttaaacatc tatttgacaa gaaatgcata gttccttctc 60
   tttaaaataa tgtaatgttt ctttcaagaa taagcttggt ttgatgcctc tctccccaac 120
   atgatagaag tgtagcataa atctatgaaa aattccattt ccctgtgcct acaacaacta 180
   cctgggattg aaaacttctt cccttgctct agtcctttct tctacaccta cttccacatc 240
   atctgtgact caaaacaata cttgtcagga aagatcccgg aaagagcaaa aaagacttcc 300
   ttagaggtgt cagagattcc tatgccacta tctgtcatct ctagaagggg ttgtgagtat 360
   gaggaagagc agagcttgta aattttctac ttgctttgac ttccactgta tttcctaaca 420
   acaacaacca cagcaacacc cataacatca caggacaaac ttctagtact tccaaggctt 480
   tagtctcagt aaatcttctc tacctccatc acagcagcta gaaggtttga tactcataca 540
   aatagtactg tagctttctg ttcataattg gaaaaataga caagacccaa tgtaatacag 600
   gctttccttc agccagttag cgttcagttt ttggatcacc attgcacaca tatacccagc 660
   atatgtctaa tatatatgta gaaatccgtg aagcaagagt tataatagct tgtgttttct 720
   attgtattgt attttcctct tatatcatct tcttcttcgt tcattaaaaa aaaaccgttc 780
   aagtaggtct aaattaatta ttggatcata agtagataaa atattttatt tcataacaca 840
   ttgacccgat gaatatgttt ctttgccaga catagtcctc atttccaagg taacaagcct 900
   gaaaaaatta tactggagca agtcaacagg taatgatggt agcttttcct tattgtcctg 960
   gggcaagaat aagacaaaag ataacagggt agaataaaga ttgtgtaaga aagaaggaca 1020
   gcaacaggac atgggaacct tttatagagt aacattttga taatggatga tgagaattaa 1080
   tgagttagac agggatgggt gggaatgatt gaaggtgtga gtactttagc acagattaag 1140
   accaaatcat taggatttaa agagttgtgt agagttagtg aaggaaaagc cttagaatta 1200
   aatttggctg cggataaaac attcttggat tagactgaag actcttttct gtgctaagta 1260
   agtatattta tgataatgat gatgactgta gtgctgaata tttaataaat aaaaacaaaa 1320
   ttaattgccg catacataat gtcctgaata ctattgtaaa tgttttatct tatttccttt 1380
   aaactgtcta cagcactata aggtaggtac cagtattgtc acagttacac agatatggaa 1440
   accgagacac agggaagtta agttacttga tcaatttcaa gcaatcggca agccatggag 1500
   catctatgtc agggctgcca ggacatgtga ctgtaaacag aagtttttca ctttttaact 1560
   caaagagggt atgtggctgg gttaatggaa agcttcagga ccctcagaaa acattactaa 1620
   caagcaaatg aaaggtgtat ctggaagatt aagttttaac agactcttca tttccatcga 1680
   tccaataatg cacttaggga gatgactggg catattgagg ataggaagag agaagtgaaa 1740
   acacagcttt ttatattgtt cttaacaggc ttgtgccaaa catcttctgg gtggatttag 1800
   gtgattgagg agaagaaaga cacaggagcg aaattctctg agcacaaggg aggagttcta 1860
   cactcagact gagccaacag acttttctgg cctgacaacc agggcggcgc aggatgctca 1920
   gtgcagagag gaagaagcag gtggtctttg cagctgaaag ctcagctgat ttgcatatgg 1980
   agtcattata caacatccca gaattcttta agggcagctg ccaggaagct aagaagcatc 2040
   ctctcttcta gctctcagag atggagacag acacactcct gctatgggtg ctgctgctct 2100
   gggttccagg tgagggtaca gataagtgtt atgagcaacc tctgtggcca ttatgatgct 2160
   ccatgcctct ctgttcttga tcactataat tagggcattt gtcactggtt ttaagtttcc 2220
   ccagtcccct gaattttcca ttttctcaga gtgatgtcca aaattattct taaaaattta 2280
   aatgaaaagg tcctctgctg tgaaggcttt taaagatata taaaaataat ctttgtgttt 2340
   atcattccag gtgccagatg tgacatccag atgacccagt ctccatcctc cctgtctgca 2400
   tctgtaggag acagagtcac catcacttgc cgggcaagtc agagcattag cagctattta 2460
   aattggtatc agcagaaacc agggaaagcc cctaagctcc tgatctatgc tgcatccagt 2520
   ttgcaaagtg gggtcccatc aaggttcagt ggcagtggat ctgggacaga tttcactctc 2580
   accatcagca gtctgcaacc tgaagatttt gcaacttact actgtcaaca gagttacagt 2640
   acccctccga tcaccttcgg ccaagggaca cgactggaga ttaaacgtaa gtaatttttc 2700
   actattgtct tctgaaattt gggtctgatg gccagtattg acttttagag gcttaaatag 2760
   gagtttggta aagattggta aatgagggca tttaagattt gccatgggtt gcaaaagtta 2820
   aactcagctt caaaaatgga tttggagaaa aaaagattaa attgctctaa actgaatgac 2880
   acaaagtaaa aaaaaaaagt gtaactaaaa aggaaccctt gtatttctaa ggagcaaaag 2940
   taaatttatt tttgttcact cttgccaaat attgtattgg ttgttgctga ttatgcatga 3000
   tacagaaaag tggaaaaata cattttttag tctttctccc ttttgtttga taaattattt 3060
   tgtcagacaa caataaaaat caatagcacg ccctaagatc tagatgcatg ctcgagtgcc 3120
   atttcattac ctctttctcc gcacccgaca tagat                            3155
<210> 2
   <211> 28
   <212> DNA
   <213> Artificial Sequence
<220>
   <223> Synthetic
<400> 2
   aggtgagggt acagataagt gttatgag 28
<210> 3
   <211> 27
   <212> DNA
   <213> Artificial Sequence
<220>
   <223> Synthetic
<400> 3
   tgacaaatgc cctaattata gtgatca 27
<210> 4
   <211> 21
   <212> DNA
   <213> Artificial Sequence
<220>
   <223> Synthetic
<400> 4
   gggcaagtca gagcattagc a 21
<210> 5
   <211> 21
   <212> DNA
   <213> Artificial Sequence
<220>
   <223> Synthetic
<400> 5
   tgcaaactgg atgcagcata g 21
<210> 6
   <211> 19
   <212> DNA
   <213> Artificial Sequence
<220>
   <223> Synthetic
<400> 6
   ggtggagagg ctattcggc 19
<210> 7
   <211> 17
   <212> DNA
   <213> Artificial Sequence
<220>
   <223> Synthetic
<400> 7
   gaacacggcg gcatcag 17
<210> 8
   <211> 23
   <212> DNA
   <213> Artificial Sequence
<220>
   <223> Synthetic
<400> 8
   tgggcacaac agacaatcgg ctg 23
<210> 9
   <211> 29
   <212> DNA
   <213> Artificial Sequence
<220>
   <223> Synthetic
<400> 9
   ccattatgat gctccatgcc tctctgttc 29
<210> 10
   <211> 26
   <212> DNA
   <213> Artificial Sequence
<220>
   <223> Synthetic
<400> 10
   atcagcagaa accagggaaa gcccct 26
<210> 11
   <211> 3166
   <212> DNA
   <213> Artificial Sequence
<220>
   <223> Synthetic
<400> 11
   ggcgcgccgt agctttgaat tttaaacatc tatttgacaa gaaatgcata gttccttctc 60
   tttaaaataa tgtaatgttt ctttcaagaa taagcttggt ttgatgcctc tctccccaac 120
   atgatagaag tgtagcataa atctatgaaa aattccattt ccctgtgcct acaacaacta 180
   cctgggattg aaaacttctt cccttgctct agtcctttct tctacaccta cttccacatc 240
   atctgtgact caaaacaata cttgtcagga aagatcccgg aaagagcaaa aaagacttcc 300
   ttagaggtgt cagagattcc tatgccacta tctgtcatct ctagaagggg ttgtgagtat 360
   gaggaagagc agagcttgta aattttctac ttgctttgac ttccactgta tttcctaaca 420
   acaacaacca cagcaacacc cataacatca caggacaaac ttctagtact tccaaggctt 480
   tagtctcagt aaatcttctc tacctccatc acagcagcta gaaggtttga tactcataca 540
   aatagtactg tagctttctg ttcataattg gaaaaataga caagacccaa tgtaatacag 600
   gctttccttc agccagttag cgttcagttt ttggatcacc attgcacaca tatacccagc 660
   atatgtctaa tatatatgta gaaatccgtg aagcaagagt tataatagct tgtgttttct 720
   attgtattgt attttcctct tatatcatct tcttcttcgt tcattaaaaa aaaaccgttc 780
   aagtaggtct aaattaatta ttggatcata agtagataaa atattttatt tcataacaca 840
   ttgacccgat gaatatgttt ctttgccaga catagtcctc atttccaagg taacaagcct 900
   gaaaaaatta tactggagca agtcaacagg taatgatggt agcttttcct tattgtcctg 960
   gggcaagaat aagacaaaag ataacagggt agaataaaga ttgtgtaaga aagaaggaca 1020
   gcaacaggac atgggaacct tttatagagt aacattttga taatggatga tgagaattaa 1080
   tgagttagac agggatgggt gggaatgatt gaaggtgtga gtactttagc acagattaag 1140
   accaaatcat taggatttaa agagttgtgt agagttagtg aaggaaaagc cttagaatta 1200
   aatttggctg cggataaaac attcttggat tagactgaag actcttttct gtgctaagta 1260
   agtatattta tgataatgat gatgactgta gtgctgaata tttaataaat aaaaacaaaa 1320
   ttaattgccg catacataat gtcctgaata ctattgtaaa tgttttatct tatttccttt 1380
   aaactgtcta cagcactata aggtaggtac cagtattgtc acagttacac agatatggaa 1440
   accgagacac agggaagtta agttacttga tcaatttcaa gcaatcggca agccatggag 1500
   catctatgtc agggctgcca ggacatgtga ctgtaaacag aagtttttca ctttttaact 1560
   caaagagggt atgtggctgg gttaatggaa agcttcagga ccctcagaaa acattactaa 1620
   caagcaaatg aaaggtgtat ctggaagatt aagttttaac agactcttca tttccatcga 1680
   tccaataatg cacttaggga gatgactggg catattgagg ataggaagag agaagtgaaa 1740
   acacagcttt ttatattgtt cttaacaggc ttgtgccaaa catcttctgg gtggatttag 1800
   gtgattgagg agaagaaaga cacaggagcg aaattctctg agcacaaggg aggagttcta 1860
   cactcagact gagccaacag acttttctgg cctgacaacc agggcggcgc aggatgctca 1920
   gtgcagagag gaagaagcag gtggtctttg cagctgaaag ctcagctgat ttgcatatgg 1980
   agtcattata caacatccca gaattcttta agggcagctg ccaggaagct aagaagcatc 2040
   ctctcttcta gctctcagag atggagacag acacactcct gctatgggtg ctgctgctct 2100
   gggttccagg tgagggtaca gataagtgtt atgagcaacc tctgtggcca ttatgatgct 2160
   ccatgcctct ctgttcttga tcactataat tagggcattt gtcactggtt ttaagtttcc 2220
   ccagtcccct gaattttcca ttttctcaga gtgatgtcca aaattattct taaaaattta 2280
   aatgaaaagg tcctctgctg tgaaggcttt taaagatata taaaaataat ctttgtgttt 2340
   atcattccag gtgccagatg tataccaccg gagaaattgt gttgacgcag tctccaggca 2400
   ccctgtcttt gtctccaggg gaaagagcca ccctctcctg cagggccagt cagagtgtta 2460
   gcagcagcta cttagcctgg taccagcaga aacctggcca ggctcccagg ctcctcatct 2520
   atggtgcatc cagcagggcc actggcatcc cagacaggtt cagtggcagt gggtctggga 2580
   cagacttcac tctcaccatc agcagactgg agcctgaaga ttttgcagtg tattactgtc 2640
   agcagtatgg tagctcacct tggacgttcg gccaagggac caaggtggaa atcaaacgta 2700
   agtaattttt cactattgtc ttctgaaatt tgggtctgat ggccagtatt gacttttaga 2760
   ggcttaaata ggagtttggt aaagattggt aaatgagggc atttaagatt tgccatgggt 2820
   tgcaaaagtt aaactcagct tcaaaaatgg atttggagaa aaaaagatta aattgctcta 2880
   aactgaatga cacaaagtaa aaaaaaaaag tgtaactaaa aaggaaccct tgtatttcta 2940
   aggagcaaaa gtaaatttat ttttgttcac tcttgccaaa tattgtattg gttgttgctg 3000
   attatgcatg atacagaaaa gtggaaaaat acatttttta gtctttctcc cttttgtttg 3060
   ataaattatt ttgtcagaca acaataaaaa tcaatagcac gccctaagat ctagatgcat 3120
   gctcgagtgc catttcatta cctctttctc cgcacccgac atagat                3166
<210> 12
   <211> 19
   <212> DNA
   <213> Artificial Sequence
<220>
   <223> Synthetic
<400> 12
   tccaggcacc ctgtctttg 19
<210> 13
   <211> 26
   <212> DNA
   <213> Artificial Sequence
<220>
   <223> Synthetic
<400> 13
   aagtagctgc tgctaacact ctgact 26
<210> 14
   <211> 24
   <212> DNA
   <213> Artificial Sequence
<220>
   <223> Synthetic
<400> 14
   aaagagccac cctctcctgc aggg 24
<210> 15
   <211> 3187
   <212> DNA
   <213> Artificial Sequence
<220>
   <223> Synthetic
<400> 15
   ggcgcgccgt agctttgaat tttaaacatc tatttgacaa gaaatgcata gttccttctc 60
   tttaaaataa tgtaatgttt ctttcaagaa taagcttggt ttgatgcctc tctccccaac 120
   atgatagaag tgtagcataa atctatgaaa aattccattt ccctgtgcct acaacaacta 180
   cctgggattg aaaacttctt cccttgctct agtcctttct tctacaccta cttccacatc 240
   atctgtgact caaaacaata cttgtcagga aagatcccgg aaagagcaaa aaagacttcc 300
   ttagaggtgt cagagattcc tatgccacta tctgtcatct ctagaagggg ttgtgagtat 360
   gaggaagagc agagcttgta aattttctac ttgctttgac ttccactgta tttcctaaca 420
   acaacaacca cagcaacacc cataacatca caggacaaac ttctagtact tccaaggctt 480
   tagtctcagt aaatcttctc tacctccatc acagcagcta gaaggtttga tactcataca 540
   aatagtactg tagctttctg ttcataattg gaaaaataga caagacccaa tgtaatacag 600
   gctttccttc agccagttag cgttcagttt ttggatcacc attgcacaca tatacccagc 660
   atatgtctaa tatatatgta gaaatccgtg aagcaagagt tataatagct tgtgttttct 720
   attgtattgt attttcctct tatatcatct tcttcttcgt tcattaaaaa aaaaccgttc 780
   aagtaggtct aaattaatta ttggatcata agtagataaa atattttatt tcataacaca 840
   ttgacccgat gaatatgttt ctttgccaga catagtcctc atttccaagg taacaagcct 900
   gaaaaaatta tactggagca agtcaacagg taatgatggt agcttttcct tattgtcctg 960
   gggcaagaat aagacaaaag ataacagggt agaataaaga ttgtgtaaga aagaaggaca 1020
   gcaacaggac atgggaacct tttatagagt aacattttga taatggatga tgagaattaa 1080
   tgagttagac agggatgggt gggaatgatt gaaggtgtga gtactttagc acagattaag 1140
   accaaatcat taggatttaa agagttgtgt agagttagtg aaggaaaagc cttagaatta 1200
   aatttggctg cggataaaac attcttggat tagactgaag actcttttct gtgctaagta 1260
   agtatattta tgataatgat gatgactgta gtgctgaata tttaataaat aaaaacaaaa 1320
   ttaattgccg catacataat gtcctgaata ctattgtaaa tgttttatct tatttccttt 1380
   aaactgtcta cagcactata aggtaggtac cagtattgtc acagttacac agatatggaa 1440
   accgagacac agggaagtta agttacttga tcaatttcaa gcaatcggca agccatggag 1500
   catctatgtc agggctgcca ggacatgtga ctgtaaacag aagtttttca ctttttaact 1560
   caaagagggt atgtggctgg gttaatggaa agcttcagga ccctcagaaa acattactaa 1620
   caagcaaatg aaaggtgtat ctggaagatt aagttttaac agactcttca tttccatcga 1680
   tccaataatg cacttaggga gatgactggg catattgagg ataggaagag agaagtgaaa 1740
   acacagcttt ttatattgtt cttaacaggc ttgtgccaaa catcttctgg gtggatttag 1800
   gtgattgagg agaagaaaga cacaggagcg aaattctctg agcacaaggg aggagttcta 1860
   cactcagact gagccaacag acttttctgg cctgacaacc agggcggcgc aggatgctca 1920
   gtgcagagag gaagaagcag gtggtctttg cagctgaaag ctcagctgat ttgcatatgg 1980
   agtcattata caacatccca gaattcttta agggcagctg ccaggaagct aagaagcatc 2040
   ctctcttcta gctctcagag atggagacag acacactcct gctatgggtg ctgctgctct 2100
   gggttccagg tgagggtaca gataagtgtt atgagcaacc tctgtggcca ttatgatgct 2160
   ccatgcctct ctgttcttga tcactataat tagggcattt gtcactggtt ttaagtttcc 2220
   ccagtcccct gaattttcca ttttctcaga gtgatgtcca aaattattct taaaaattta 2280
   aatgaaaagg tcctctgctg tgaaggcttt taaagatata taaaaataat ctttgtgttt 2340
   atcattccag gtgccagatg tgttgtggtc ctcagccggt gctgcatcag ccgccggcca 2400
   tgtcctcggc ccttggaacc acaatccgcc tcacctgcac cctgaggaac gaccatgaca 2460
   tcggtgtgta cagcgtctac tggtaccagc agaggccggg ccaccctccc aggttcctgc 2520
   tgagatattt ctcacaatca gacaagagcc agggccccca ggtcccccct cgcttctctg 2580
   gatccaaaga tgtggccagg aacagggggt atttgagcat ctctgagctg cagcctgagg 2640
   acgaggctat gtattactgt gctatgcata actcagtgac gcatgtgttt ggcagcggga 2700
   cccagctcac cgttttaagt aagtaatttt tcactattgt cttctgaaat ttgggtctga 2760
   tggccagtat tgacttttag aggcttaaat aggagtttgg taaagattgg taaatgaggg 2820
   catttaagat ttgccatggg ttgcaaaagt taaactcagc ttcaaaaatg gatttggaga 2880
   aaaaaagatt aaattgctct aaactgaatg acacaaagta aaaaaaaaaa gtgtaactaa 2940
   aaaggaaccc ttgtatttct aaggagcaaa agtaaattta tttttgttca ctcttgccaa 3000
   atattgtatt ggttgttgct gattatgcat gatacagaaa agtggaaaaa tacatttttt 3060
   agtctttctc ccttttgttt gataaattat tttgtcagac aacaataaaa atcaatagca 3120
   cgccctaaga tctagatgca tgctcgagtg ccatttcatt acctctttct ccgcacccga 3180
   catagat                                                           3187
<210> 16
   <211> 17
   <212> DNA
   <213> Artificial Sequence
<220>
   <223> Synthetic
<400> 16
   tgtcctcggc ccttgga 17
<210> 17
   <211> 20
   <212> DNA
   <213> Artificial Sequence
<220>
   <223> Synthetic
<400> 17
   ccgatgtcat ggtcgttcct 20
<210> 18
   <211> 23
   <212> DNA
   <213> Artificial Sequence
<220>
   <223> Synthetic
<400> 18
   acaatccgcc tcacctgcac cct 23
<210> 19
   <211> 23
   <212> DNA
   <213> Artificial Sequence
<220>
   <223> synthetic
<400> 19
   agcagtctgc aacctgaaga ttt 23
<210> 20
   <211> 24
   <212> DNA
   <213> Artificial Sequence
<220>
   <223> synthetic
<400> 20
   gtttaatctc cagtcgtgtc cctt 24
<210> 21
   <211> 15
   <212> DNA
   <213> Artificial Sequence
<220>
   <223> synthetic
<400> 21
   cctccgatca ccttc 15
<210> 22
   <211> 20
   <212> DNA
   <213> Artificial Sequence
<220>
   <223> synthetic
<400> 22
   aaaccaggga aagcccctaa 20
<210> 23
   <211> 18
   <212> DNA
   <213> Artificial Sequence
<220>
   <223> synthetic
<400> 23
   atgggacccc actttgca 18
<210> 24
   <211> 19
   <212> DNA
   <213> Artificial Sequence
<220>
   <223> synthetic
<400> 24
   ctcctgatct atgctgcat 19
<210> 25
   <211> 21
   <212> DNA
   <213> Artificial Sequence
<220>
   <223> synthetic
<400> 25
   cagcagactg gagcctgaag a 21
<210> 26
   <211> 21
   <212> DNA
   <213> Artificial Sequence
<220>
   <223> synthetic
<400> 26
   tgatttccac cttggtccct t 21
<210> 27
   <211> 18
   <212> DNA
   <213> Artificial Sequence
<220>
   <223> synthetic
<400> 27
   tagctcacct tggacgtt 18
<210> 28
   <211> 22
   <212> DNA
   <213> Artificial Sequence
<220>
   <223> synthetic
<400> 28
   ctcctcatct atggtgcatc ca 22
<210> 29
   <211> 20
   <212> DNA
   <213> Artificial Sequence
<220>
   <223> synthetic
<400> 29
   gacccactgc cactgaacct 20
<210> 30
   <211> 13
   <212> DNA
   <213> Artificial Sequence
<220>
   <223> synthetic
<400> 30
   ccactggcat ccc 13
<210> 31
   <211> 19
   <212> DNA
   <213> Artificial Sequence
<220>
   <223> synthetic
<400> 31
   tgagcagcac cctcacgtt 19
<210> 32
   <211> 23
   <212> DNA
   <213> Artificial Sequence
<220>
   <223> synthetic
<400> 32
   gtggcctcac aggtatagct gtt 23
<210> 33
   <211> 18
   <212> DNA
   <213> Artificial Sequence
<220>
   <223> synthetic
<400> 33
   accaaggacg agtatgaa 18

## Description

### FIELD OF INVENTION

A genetically modified mouse is provided that expresses antibodies having a common human variable/mouse constant light chain associated with diverse human variable/mouse constant heavy chains. A method for making a human bispecific antibody from human variable region gene sequences of B cells of the mouse is provided.

### BACKGROUND

Antibodies typically comprise a homodimeric heavy chain component, wherein each heavy chain monomer is associated with an identical light chain. Antibodies having a heterodimeric heavy chain component (*e.g*., bispecific antibodies) are desirable as therapeutic antibodies. But making bispecific antibodies having a suitable light chain component that can satisfactorily associate with each of the heavy chains of a bispecific antibody has proved problematic.

In one approach, a light chain might be selected by surveying usage statistics for all light chain variable domains, identifying the most frequently employed light chain in human antibodies, and pairing that light chain *in vitro* with the two heavy chains of differing specificity.

In another approach, a light chain might be selected by observing light chain sequences in a phage display library (*e.g*., a phage display library comprising human light chain variable region sequences, *e.g*., a human scFv library) and selecting the most commonly used light chain variable region from the library. The light chain can then be tested on the two different heavy chains of interest.

In another approach, a light chain might be selected by assaying a phage display library of light chain variable sequences using the heavy chain variable sequences of both heavy chains of interest as probes. A light chain that associates with both heavy chain variable sequences might be selected as a light chain for the heavy chains.

In another approach, a candidate light chain might be aligned with the heavy chains' cognate light chains, and modifications are made in the light chain to more closely match sequence characteristics common to the cognate light chains of both heavy chains. If the chances of immunogenicity need to be minimized, the modifications preferably result in sequences that are present in known human light chain sequences, such that proteolytic processing is unlikely to generate a T cell epitope based on parameters and methods known in the art for assessing the likelihood of immunogenicity (*i.e*., *in silico* as well as wet assays).

All of the above approaches rely on in vitro methods that subsume a number of a priori restraints, *e*.*g*., sequence identity, ability to associate with specific pre-selected heavy chains, *etc.* There is a need in the art for compositions and methods that do not rely on manipulating in vitro conditions, but that instead employ more biologically sensible approaches to making human epitope-binding proteins that include a common light chain.

### SUMMARY

Genetically modified mice that express human immunoglobulin heavy and light chain variable domains, wherein the mice have a limited light chain variable repertoire, are provided. In particular, a genetically modified mouse is provided comprising a B cell that expresses a human light chain variable (V_{L}) domain derived from a rearranged human Vκ1-39/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4. Also provided is the use of such a mouse in making an antibody comprising a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4 associated with a human light chain variable region derived from a rearranged human Vκ1-39/Jκ sequence. Further provided is a method of producing an antibody to an antigen of interest comprising a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4 associated with a human light chain variable region derived from a rearranged human Vκ1-39/Jκ sequence, the method comprising: (a) immunizing the mouse provided with an antigen of interest; (b) obtaining from the mouse a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4; and (c) employing the immunoglobulin variable region sequence obtained in (b) associated with said human light chain variable region in an antibody that specifically binds the antigen of interest.

Also provided is a genetically modified mouse comprising a B cell that expresses a human light chain variable (V_{L}) domain derived from a rearranged human Vκ3-20/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4. Further provided is the use of such a mouse provided in making an antibody comprising a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4. Also provided is the use of such a mouse provided in making an antibody comprising a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4 associated with a human light chain variable region derived from a rearranged human Vκ3-20/Jκ sequence. Also provided is a method of producing an antibody to an antigen of interest comprising a human heavy chain variable domain derived from a rearranged human V_{H}/D_{H}/J_{H} region selected from 3-30/3-3/3, 3-33/1-7/4, 3-33/2-15/4 or 3-53/1-1/4 associated with a human light chain variable region derived from a rearranged human Vκ3-20/Jκ sequence, the method comprising: (a) immunizing the mouse provided with an antigen of interest; (b) obtaining from the mouse a human heavy chain variable domain derived from a rearranged human V_{H}/D_{H}/J_{H} region selected from 3-30/3-3/3, 3-33/1-7/4, 3-33/2-15/4 or 3-53/1-1/4; and (c) employing the immunoglobulin variable region sequence obtained in (b) associated with said human light chain variable region in an antibody that specifically binds the antigen of interest.

Also referred to is a biological system for generating a human light chain variable domain that associates and expresses with a diverse repertoire of affinity-matured human heavy chain variable domains. Also referred to are methods for making binding proteins comprising immunoglobulin variable domains, comprising immunizing mice that have a limited immunoglobulin light chain repertoire with an antigen of interest, and employing an immunoglobulin variable region gene sequence of the mouse in a binding protein that specifically binds the antigen of interest. Methods include methods for making human immunoglobulin heavy chain variable domains suitable for use in making multi-specific antigen-binding proteins.

The genetically engineered mice provided may select suitable affinity-matured human immunoglobulin heavy chain variable domains derived from a repertoire of unrearranged human heavy chain variable region gene segments, wherein the affinity-matured human heavy chain variable domains associate and express with a single human light chain variable domain derived from one human light chain variable region gene segment. Genetically engineered mice that present a choice of two human light chain variable region gene segments are also provided.

[Deleted]

Genetically engineered mice are provided that express a limited repertoire of human light chain variable domains, or a single human light chain variable domain, from a limited repertoire of human light chain variable region sequences. The mice are genetically engineered to include a single V/J human light chain sequence (or two V/J sequences) that express a variable region of a single light chain (or that express either or both of two variable regions). The rearranged sequences are a rearranged human Vκ1-39/Jκ sequence, a rearranged human Vκ3-20/Jκ, or both. A light chain comprising the variable sequence is capable of pairing with a plurality of affinity-matured human heavy chains clonally selected by the mice, wherein the heavy chain variable regions specifically bind different epitopes.

Also referred to is a genetically modified mouse that comprises a single human immunoglobulin light chain variable (V_{L}) region gene segment that is capable of rearranging with a human J gene segment (selected from one or a plurality of J_{L} segments) and encoding a human V_{L} domain of an immunoglobulin light chain. In another instance, the mouse comprises no more than two human V_{L} gene segments, each of which is capable of rearranging with a human J gene segment (selected from one or a plurality of J_{L} segments) and encoding a human V_{L} domain of an immunoglobulin light chain.

In one instance, the single human V_{L} gene segment is operably linked to a human J_{L} gene segment selected from Jκ1, Jκ2, Jκ3, Jκ4, and Jκ5, wherein the single human V_{L} gene segment is capable of rearranging to form a sequence encoding a light chain variable region gene with any of the one or more human J_{L} gene segments.

Also referred to is a genetically modified mouse that comprises an immunoglobulin light chain locus that does not comprise an endogenous mouse V_{L} gene segment that is capable of rearranging to form an immunoglobulin light chain gene, wherein the V_{L} locus comprises a single human V_{L} gene segment that is capable of rearranging to encode a V_{L} region of a light chain gene. In a specific instance, the human V_{L} gene segment is a human Vκ1-39Jκ5 gene segment or a human Vκ3-20Jκ1 gene segment. Also referred to is a genetically modified mouse comprises a V_{L} locus that does not comprise an endogenous mouse V_{L} gene segment that is capable of rearranging to form an immunoglobulin light chain gene, wherein the V_{L} locus comprises no more than two human V_{L} gene segments that are capable of rearranging to encode a V_{L} region of a light chain gene. In a specific instance, the no more than 2 human V_{L} gene segments are a human Vκ1-39Jκ gene segment and a human Vκ3-20Jκ gene segment.

In one aspect, the genetically modified mouse provided comprises a single rearranged (V/J) human immunoglobulin light chain variable (V_{L}) region (i.e., a V_{L}/J_{L} region) that encodes a human V_{L} domain of an immunoglobulin light chain. In another aspect, the mouse comprises no more than two rearranged human V_{L} regions that are capable of encoding a human V_{L} domain of an immunoglobulin light chain. The rearranged sequences are a rearranged human Vκ1-39/Jκ sequence, a rearranged human Vκ3-20/Jκ, or both.

The V_{L} region is a human Vκ1-39Jκ sequence or a human Vκ3-20Jκ sequence. In one embodiment, the human J_{L} segment of the rearranged V_{L}/J_{L} sequence is selected from Jκ1, Jκ2, Jκ3, Jκ4, and Jκ5 In a specific embodiment, the V_{L} region is a human Vκ1-39JK5 sequence or a human Vκ3-20Jκ1 sequence. In a specific embodiment the mouse has both a human Vκ1-39Jκ5 sequence and a human Vκ3-20Jκ1 sequence.

In one embodiment, the human V_{L} gene segment is operably linked to a human or mouse leader sequence. In one embodiment, the leader sequence is a mouse leader sequence. In a specific embodiment, the mouse leader sequence is a mouse Vκ3-7 leader sequence. In a specific embodiment, the leader sequence is operably linked to the rearranged human V_{L}/J_{L} sequence.

In one embodiment, the VL gene segment is operably linked to an immunoglobulin promoter sequence. In one embodiment, the promoter sequence is a human promoter sequence. In a specific embodiment, the human immunoglobulin promoter is a human Vκ3-15 promoter. In a specific embodiment, the promoter is operably linked to the rearranged human V_{L}/J_{L} sequence.

Also referred to is a light chain locus which comprises a leader sequence flanked 5' (with respect to transcriptional direction of a V_{L} gene segment) with a human immunoglobulin promoter and flanked 3' with a human V_{L} gene segment that rearranges with a human J segment and encodes a V_{L} domain of a reverse chimeric light chain comprising an endogenous mouse light chain constant region (C_{L}). In a specific embodiment, the V_{L} gene segment is at the mouse Vκ locus, and the mouse C_{L} is a mouse CK.

In one embodiment, the light chain locus comprises a leader sequence flanked 5' (with respect to transcriptional direction of a V_{L} gene segment) with a human immunoglobulin promoter and flanked 3' with a rearranged human V_{L} region (V_{L}/J_{L} sequence) and encodes a V_{L} domain of a reverse chimeric light chain comprising an endogenous mouse light chain constant region (C_{L}). In a specific embodiment, the rearranged human V_{L}/J_{L} sequence is at the mouse kappa (κ) locus, and the mouse C_{L} is a mouse Cκ.

In one embodiment, the V_{L} locus of the modified mouse is a κ light chain locus, and the κ light chain locus comprises a mouse κ intronic enhancer, a mouse κ 3' enhancer, or both an intronic enhancer and a 3' enhancer.

In one embodiment, the mouse comprises a nonfunctional immunoglobulin lambda (λ) light chain locus. In a specific embodiment, the λ light chain locus comprises a deletion of one or more sequences of the locus, wherein the one or more deletions renders the λ light chain locus incapable of rearranging to form a light chain gene. In another embodiment, all or substantially all of the V_{L} gene segments of the λ light chain locus are deleted.

In one embodiment, mouse makes a light chain that comprises a somatically mutated V_{L} domain derived from the human V_{L} gene segment. In one embodiment, the light chain comprises a somatically mutated V_{L} domain derived from the human V_{L} gene segment, and a mouse Cκ region. In one embodiment, the mouse does not express a λ light chain.

In one embodiment, the genetically modified mouse is capable of somatically hypermutating the human V_{L} region sequence.

In one embodiment, the mouse comprises a cell that expresses a light chain comprising a somatically mutated human V_{L} domain linked to a mouse Cκ, wherein the light chain associates with a heavy chain comprising a somatically mutated V_{H} domain derived from a human V_{H} gene segment and wherein the heavy chain comprises a mouse heavy chain constant region (C_{H}). In a specific embodiment, the heavy chain comprises a mouse C_{H}1, a mouse hinge, a mouse C_{H}2, and a mouse C_{H}3. In a specific embodiment, the heavy chain comprises a human C_{H}1, a hinge, a mouse C_{H}2, and a mouse C_{H}3.

In one embodiment, the mouse comprises a replacement of endogenous mouse V_{H} gene segments with one or more human V_{H} gene segments, wherein the human V_{H} gene segments are operably linked to a mouse C_{H} region gene, such that the mouse rearranges the human V_{H} gene segments and expresses a reverse chimeric immunoglobulin heavy chain that comprises a human V_{H} domain and a mouse C_{H}. In one embodiment, 90-100% of unrearranged mouse V_{H} gene segments are replaced with at least one unrearranged human V_{H} gene segment. In a specific embodiment, all or substantially all of the endogenous mouse V_{H} gene segments are replaced with at least one unrearranged human V_{H} gene segment. In one embodiment, the replacement is with at least 19, at least 39, or at least 80 or 81 unrearranged human V_{H} gene segments. In one embodiment, the replacement is with at least 12 functional unrearranged human V_{H} gene segments, at least 25 functional unrearranged human V_{H} gene segments, or at least 43 functional unrearranged human V_{H} gene segments. In one embodiment, the mouse comprises a replacement of all mouse D_{H} and J_{H} segments with at least one unrearranged human D_{H} segment and at least one unrearranged human J_{H} segment. In one embodiment, the at least one unrearranged human D_{H} segment is selected from 1-1, 1-7, 1-26, 2-8, 2-15, 3-3, 3-10, 3-16, 3-22, 5-5, 5-12, 6-6, 6-13, 7-27, and a combination thereof. In one embodiment, the at least one unrearranged human J_{H} segment is selected from 1, 2, 3, 4, 5, 6, and a combination thereof. In a specific embodiment, the one or more human V_{H} gene segment is selected from a 1-2, 1-8, 1-24, 1 - 69, 2-5, 3-7, 3-9, 3-11, 3-13, 3-15, 3-20, 3-23, 3-30, 3-33, 3-48, 3-53, 4-31 , 4-39, 4-59, 5-51, a 6-1 human V_{H} gene segment, and a combination thereof.

A genetically modified mouse is provided comprising a B cell that expresses a human light chain variable (V_{L}) domain derived from a rearranged human Vκ1-39/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4. Also provided is a genetically modified mouse comprising a B cell that expresses a human V_{L} domain derived from a rearranged human Vκ3-20/Jκ sequence, that is present in the germline of the mouse wherein the mouse lacks an unrearranged endogenous immunoglobulin Vκ gene segment and an unrearranged endogenous immunoglobulin Jκ gene segment; and wherein the human V_{L} domain(s) is associated with a human V_{H} domain derived from a rearranged human V_{H}/D_{H}/J_{H} region selected from 3-30/3-3/3, 3-33/1-7/4, 3-33/2-15/4 or 3-53/1-1/4.

Also referred to is a mouse that comprises a B cell that expresses a binding protein that specifically binds an antigen of interest, wherein the binding protein comprises a light chain derived from a human Vκ1-39/Jκ5 rearrangement or a human Vκ3-20/Jκ1 rearrangement, and wherein the cell comprises a rearranged immunoglobulin heavy chain gene derived from a rearrangement of human V_{H} gene segments selected from a 1-69, 2-5, 3-13, 3-23, 3-30, 3-33, 3-53, 4-39, 4-59, and 5-51 gene segment. In one instance, the one or more human V_{H} gene segments are rearranged with a human heavy chain J_{H} gene segment selected from 1, 2, 3, 4, 5, and 6. In one instance, the one or more human V_{H} and J_{H} gene segments are rearranged with a human D_{H} gene segment selected from 1-1, 1-7, 1-26, 2-8, 2-15, 3-3, 3-10, 3-16, 3-22, 5-5, 5-12, 6-6, 6-13, and 7-27. In a specific instance, the light chain gene has 1, 2, 3, 4, or 5 or more somatic hypermutations.

In one embodiment, the mouse comprises a B cell that comprises a rearranged immunoglobulin heavy chain variable region gene sequence comprising a V_{H}/D_{H}/J_{H} region selected from 2-5/6-6/1, 2-5/3-22/1, 3-13/6-6/5, 3-23/2-8/4, 3-23/3-3/4, 3-23/3-10/4, 3-23/6-6/4, 3-23/7-27/4, 3-30/1-1/4, 3-30/1-7/4, 3-30/3-3/3, 3-30/3-3/4, 3-30/3-22/5, 3-30/5-5/2, 3-30/5-12/4, 3-30/6-6/1, 3-30/6-6/3, 3-30/6-6/4, 3-30/6-6/5, 3-30/6-13/4, 3-30/7-27/4, 3-30/7-27/5, 3-30/7-27/6, 3-33/1-7/4, 3-33/2-15/4, 4-39/1-26/3, 4-59/3-16/3, 4-59/3-16/4, 4-59/3-22/3, 5-51/3-16/6, 5-51/5-5/3, 5-51/6-13/5, 3-53/1-1/4, 1-69/6-6/5, and 1-69/6-13/4. In a specific embodiment, the B cell expresses a binding protein comprising a human immunoglobulin heavy chain variable region fused with a mouse heavy chain constant region, and a human immunoglobulin light chain variable region fused with a mouse light chain constant region. In one embodiment, the rearranged human V_{L} region is a human Vκ1-39Jκ5 sequence, and the mouse expresses a reverse chimeric light chain comprising (i) a V_{L} domain derived from the human V_{L}/J_{L} sequence and (ii) a mouse C_{L}; wherein the light chain is associated with a reverse chimeric heavy chain comprising (i) a mouse C_{H} and (ii) a somatically mutated human V_{H} domain derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one embodiment, the mouse expresses a light chain that is somatically mutated. In one embodiment the C_{L} is a mouse Cκ. In a specific embodiment, the human V_{H} gene segment is selected from a 2-5, 3-13, 3-23, 3-30, 4-59, 5-51, and 1-69 gene segment. In a specific embodiment, the somatically mutated human V_{H} domain comprises a sequence derived from a D_{H} segment selected from 1-1, 1-7, 2-8, 3-3, 3-10, 3-16, 3-22, 5-5, 5-12, 6-6, 6-13, and 7-27. In a specific embodiment, the somatically mutated human V_{H} domain comprises a sequence derived from a J_{H} segment selected from 1, 2, 3, 4, 5, and 6. In a specific embodiment, the somatically mutated human V_{H} domain is encoded by a rearranged human V_{H}/D_{H}/J_{H} sequence selected from 2-5/6-6/1, 2-5/3-22/1, 3-13/6-6/5, 3-23/2-8/4, 3-23/3-3/4, 3-23/3-10/4, 3-23/6-6/4, 3-23/7-27/4, 3-30/1-1/4, 3-30/1-7/4, 3-30/3-3/4, 3-30/3-22/5, 3-30/5-5/2, 3-30/5-12/4, 3-30/6-6/1, 3-30/6-6/3, 3-30/6-6/4, 3-30/6-6/5, 3-30/6-13/4, 3-30/7-27/4, 3-30/7-27/5, 3-30/7-27/6, 4-59/3-16/3, 4-59/3-16/4, 4-59/3-22/3, 5-51/5-5/3, 1-69/6-6/5, and 1-69/6-13/4.

In one embodiment, the mouse comprises a B cell that expresses a binding protein that specifically binds an antigen of interest, wherein the binding protein comprises a light chain derived from a human Vκ1-39/Jκ5 rearrangement, and wherein the cell comprises a rearranged immunoglobulin heavy chain variable region gene sequence comprising a V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 and 1-69/6-13/4. In a specific embodiment, the B cell expresses a binding protein comprising a human immunoglobulin heavy chain variable region fused with a mouse heavy chain constant region, and a human immunoglobulin light chain variable region fused with a mouse light chain constant region.

In one embodiment, the rearranged human V_{L} region is a human Vκ3-20Jκ1 sequence, and the mouse expresses a reverse chimeric light chain comprising (i) a V_{L} domain derived from the rearranged human V_{L}/J_{L} sequence, and (ii) a mouse C_{L}; wherein the light chain is associated with a reverse chimeric heavy chain comprising (i) a mouse C_{H}, and (ii) a somatically mutated human V_{H} derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one embodiment, the mouse expresses a light chain that is somatically mutated. In one embodiment the C_{L} is a mouse Cκ. In a specific embodiment, the human V_{H} gene segment is selected from a 3-30, 3-33, 3-53, 4-39, and 5-51 gene segment. In a specific embodiment, the somatically mutated human V_{H} domain comprises a sequence derived from a D_{H} segment selected from 1-1, 1-7, 1-26, 2-15, 3-3, 3-16, and 6-13. In a specific embodiment, the somatically mutated human V_{H} domain comprises a sequence derived from a J_{H} segment selected from 3, 4, 5, and 6. In a specific embodiment, the somatically mutated human V_{H} domain is encoded by a rearranged human V_{H}/D_{H}/J_{H} sequence selected from 3-30/1-1/4, 3-30/3-3/3, 3-33/1-7/4, 3-33/2-15/4, 4-39/1-26/3, 5-51/3-16/6, 5-51/6-13/5, and 3-53/1-1/4.

In one embodiment, the mouse comprises a B cell that expresses a binding protein that specifically binds an antigen of interest, wherein the binding protein comprises a light chain derived from a human Vκ3-20/Jκ1 rearrangement, and wherein the cell comprises a rearranged immunoglobulin heavy chain variable region gene sequence comprising a V_{H}/D_{H}/J_{H} region selected from 3-30/3-3/3, 3-33/1-7/4, 3-33/2-15/4, and 3-53/1-1/4. In a specific embodiment, the B cell expresses a binding protein comprising a human immunoglobulin heavy chain variable region fused with a mouse heavy chain constant region, and a human immunoglobulin light chain variable region fused with a mouse light chain constant region.

In one embodiment, the mouse comprises both a rearranged human Vκ1-39Jκ5 sequence and a rearranged human Vκ3-20Jκ1 sequence, and the mouse expresses a reverse chimeric light chain comprising (i) a V_{L} domain derived from the human Vκ1-39Jκ5 sequence or the human Vκ3-20Jκ1 sequence, and (ii) a mouse C_{L}; wherein the light chain is associated with a reverse chimeric heavy chain comprising (i) a mouse C_{H}, and (ii) a somatically mutated human V_{H} derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5-51, a 6-1 human V_{H} gene segment, and a combination thereof. In one embodiment, the mouse expresses a light chain that is somatically mutated. In one embodiment the C_{L} is a mouse Cκ.

In various embodiments, the human immunoglobulin heavy chain variable region fused with a mouse heavy chain constant region and human immunoglobulin light chain variable region fused with a mouse light chain constant region expressed by the B cell are cognate in the mouse. In various embodiments, the chimeric light chain and chimeric heavy chain expressed by the mouse are cognate in the mouse.

In one embodiment, 90-100% of the endogenous unrearranged mouse V_{H} gene segments are replaced with at least one unrearranged human V_{H} gene segment. In a specific embodiment, all or substantially all of the endogenous unrearranged mouse V_{H} gene segments are replaced with at least one unrearranged human V_{H} gene segment. In one embodiment, the replacement is with at least 18, at least 39, at least 80, or 81 unrearranged human V_{H} gene segments. In one embodiment, the replacement is with at least 12 functional unrearranged human V_{H} gene segments, at least 25 functional unrearranged human V_{H} gene segments, or at least 43 unrearranged human VH gene segments.

In one embodiment, the genetically modified mouse is a C57BL strain, in a specific embodiment selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, C57BL/Ola. In a specific embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain.

In one embodiment, the mouse expresses a reverse chimeric antibody comprising a light chain that comprises a mouse C_{K} and a somatically mutated human V_{L} domain derived from a rearranged human Vκ1 -39Jκ5 sequence or a rearranged human Vκ3-20Jκ1 sequence, and a heavy chain that comprises a mouse C_{H} and a somatically mutated human V_{H} domain derived from a human V_{H} gene segment selected from a 1-2, 1-8, 1-24, 1-69, 2-5, 3-7, 3-9, 3-11, 3-13, 3-15, 3-20, 3-23, 3-30, 3-33, 3-48, 3-53, 4-31, 4-39, 4-59, 5- 51, and a 6-1 human V_{H} gene segment, wherein the mouse does not express a fully mouse antibody and does not express a fully human antibody. In one embodiment the mouse comprises a κ light chain locus that comprises a replacement of endogenous mouse κ light chain gene segments with the rearranged human Vκ1-39Jκ5 sequence or the rearranged human Vκ3-20Jκ1 sequence, and comprises a replacement of all or substantially all endogenous mouse V_{H} gene segments with a complete or substantially complete repertoire of human V_{H} gene segments.

Also referred to is a mouse that expresses an immunoglobulin light chain from a rearranged immunoglobulin light chain sequence in the germline of the mouse, wherein the immunoglobulin light chain comprises a human variable sequence.

In one embodiment, the germline of the mouse lacks a functional unrearranged immunoglobulin light chain V gene segment. In one embodiment, the germline of the mouse lacks a functional unrearranged immunoglobulin light chain J gene segment.

In one embodiment, the germline of the mouse comprises no more than one, no more than two, or no more than three rearranged (V/J) light chain sequences.

The κ light chain sequence is a human κ light chain sequence .The κ light chain sequence is selected from a human Vκ1-39/Jκ sequence, a human Vκ3-20/Jκ sequence, and a combination thereof. In a specific embodiment, the κ light chain sequence is a human Vκ1-39/Jκ5 sequence. In a specific embodiment, the κ light chain sequence is a human Vκ3-20/Jκ1 sequence.

In one embodiment, the mouse further comprises in its germline a sequence selected from a mouse κ intronic enhancer 5' with respect to the rearranged immunoglobulin light chain sequence, a mouse κ 3' enhancer, and a combination thereof.

In one embodiment, the mouse comprises an unrearranged human V_{H} gene segment, an unrearranged human D_{H} gene segment, and an unrearranged human J_{H} gene segment, wherein said V_{H}, D_{H}, and J_{H} gene segments are capable of rearranging to form an immunoglobulin heavy chain variable gene sequence operably linked to a heavy chain constant gene sequence. The mouse comprises a plurality of human V_{H}, D_{H} and J_{H} gene segments. In a specific embodiment, the human V_{H}, D_{H}, and J_{H} gene segments replace endogenous mouse V_{H}, D_{H}, and J_{H} gene segments at the endogenous mouse immunoglobulin heavy chain locus. In a specific embodiment, the mouse comprises a replacement of all or substantially all functional mouse V_{H}, D_{H}, and J_{H} gene segments with all or substantially all functional human V_{H}, D_{H}, and J_{H} gene segments.

In one embodiment, the mouse expresses an immunoglobulin light chain that comprises a mouse constant sequence. In one embodiment, the mouse expresses an immunoglobulin light chain that comprises a human constant sequence.

In one embodiment, the mouse expresses an immunoglobulin heavy chain that comprises a mouse sequence selected from a C_{H}1 sequence, a hinge sequence, a C_{H}2 sequence, a C_{H}3 sequence, and a combination thereof.

In one embodiment, the mouse expresses an immunoglobulin heavy chain that comprises a human sequence selected from a C_{H}1 sequence, a hinge sequence, a C_{H}2 sequence, a C_{H}3 sequence, and a combination thereof.

In one embodiment, the rearranged immunoglobulin light chain sequence in the germline of the mouse is at an endogenous mouse immunoglobulin light chain locus. In a specific embodiment, the rearranged immunoglobulin light chain sequence in the germline of the mouse replaces all or substantially all mouse light chain V and J sequences at the endogenous mouse immunoglobulin light chain locus.

Also referred to is a mouse cell that is isolated from a mouse as described herein. In one instance, the cell is an ES cell. In one instance, the cell is a lymphocyte. In one instance, the lymphocyte is a B cell. In one instance, the B cell expresses a chimeric heavy chain comprising a variable domain derived from a human gene segment; and a light chain derived from a rearranged human Vκ1-39/Jκ sequence, rearranged human Vκ3-20/Jκ sequence, or a combination thereof; wherein the heavy chain variable domain is fused to a mouse constant region and the light chain variable domain is fused to a mouse or a human constant region.

Also referred to is a mouse B cell that is isolated from a mouse as described herein, wherein the B cell expresses a chimeric heavy chain derived from a rearranged human V_{H}/D_{H}/J_{H} sequence selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 and 1-69/6-13/4; and a chimeric light chain derived from a rearranged human Vκ1-39/Jκ5 sequence; wherein the variable domain is fused to a mouse constant region and the light chain variable domain is fused to a mouse constant region.

Also referred to is a mouse B cell that is isolated from a mouse as described herein, wherein the B cell expresses a chimeric heavy chain derived from a rearranged human V_{H}/D_{H}/J_{H} sequence selected from 3-30/3-3/3, 3-33/1-7/4, 3-33/2-1 5/4, and 3-53/1-1/4; and a chimeric light chain derived from a rearranged human Vκ3-20/Jκ1 sequence; wherein the variable domain is fused to a mouse constant region and the light chain variable domain is fused to a mouse constant region.

In various instances, the chimeric heavy and light chains expressed by the B cell isolated from a mouse as described herein are cognate in the mouse.

Also referred to is a hybridoma, wherein the hybridoma is made with a B cell of a mouse as described herein. In a specific instance, the B cell is from a mouse as described herein that has been immunized with an immunogen comprising an epitope of interest, and the B cell expresses a binding protein that binds the epitope of interest, the binding protein has a somatically mutated human V_{H} domain and a mouse C_{H}, and has a human V_{L} domain derived from a rearranged human Vκ1-39Jκ5 or a rearranged human Vκ3-20Jκ1 and a mouse C_{L}.

Also referred to is a hybridoma that is made with a B cell of a mouse as described herein, wherein the hybridoma expresses a chimeric heavy chain derived from a rearranged human V_{H}/D_{H}/J_{H} sequence selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 and 1-69/6-13/4; and a chimeric light chain derived from a rearranged human Vκ1-39/Jκ5 sequence; wherein the variable domain is fused to a mouse constant region and the light chain variable domain is fused to a mouse constant region.

Also referred to is a hybridoma that is made with a B cell of a mouse as described herein, wherein the hybridoma expresses a chimeric heavy chain derived from a rearranged human V_{H}/D_{H}/J_{H} sequence selected from 3-30/3-3/3, 3-33/1-7/4, 3-33/2-15/4, and 3-53/1-1/4; and a chimeric light chain derived from a rearranged human Vκ3-20/Jκ1 sequence; wherein the variable domain is fused to a mouse constant region and the light chain variable domain is fused to a mouse constant region.

In various instances, the chimeric heavy and light chains expressed by the hybridoma that is made with a B cell of a mouse as described herein are cognate in the hybridoma. In various instances, the chimeric heavy and light chains expressed by the hybridoma that is made with a B cell of a mouse as described herein are cognate in the B cell of the mouse.

Also referred to is a mouse embryo, wherein the embryo comprises a donor ES cell that is derived from a mouse as described herein.

Also referred to is a targeting vector, comprising, from 5' to 3' in transcriptional direction with reference to the sequences of the 5' and 3' mouse homology arms of the vector, a 5' mouse homology arm, a human or mouse immunoglobulin promoter, a human or mouse leader sequence, and a human V_{L} region selected from a rearranged human Vκ1-39Jκ5 or a rearranged human Vκ3-20Jκ1, and a 3' mouse homology arm. In one instance, the 5' and 3' homology arms target the vector to a sequence 5' with respect to an enhancer sequence that is present 5' and proximal to the mouse Cκ gene. In one instance, the promoter is a human immunoglobulin variable region gene segment promoter. In a specific instance, the promoter is a human Vκ3-15 promoter. In one instance, the leader sequence is a mouse leader sequence. In a specific instance, the mouse leader sequence is a mouse VK3-7 leader sequence.

Also referred to is a targeting vector as described above, but in place of the 5' mouse homology arm the human or mouse promoter is flanked 5' with a site-specific recombinase recognition site (SRRS), and in place of the 3' mouse homology arm the human V_{L} region is flanked 3' with an SRRS.

Also referred to is a reverse chimeric antibody made by a mouse as described herein, wherein the reverse chimeric antibody comprises a light chain comprising a human V_{L} and a mouse C_{L}, and a heavy chain comprising a human V_{H} and a mouse C_{H}.

Also provided is a method of producing an antibody to an antigen of interest comprising a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4 associated with a human light chain variable region derived from a rearranged human Vκ1-39/Jκ sequence, the method comprising:
(a) immunizing the mouse provided with an antigen of interest;
(b) obtaining from the mouse a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4; and
(c) employing the immunoglobulin variable region sequence obtained in (b) associated with said human light chain variable region in an antibody that specifically binds the antigen of interest.

Additionally provided is a method of producing an antibody to an antigen of interest comprising a human heavy chain variable domain derived from a rearranged human V_{H}/D_{H}/J_{H} region selected from 3-30/3-3/3, 3-33/1-7/4, 3-33/2-15/4 or 3-53/1-1/4 associated with a human light chain variable region derived from a rearranged human Vκ3-20/Jκ sequence, the method comprising:
(a) immunizing the mouse provided with an antigen of interest;
(b) obtaining from the mouse a human heavy chain variable domain derived from a rearranged human V_{H}/D_{H}/J_{H} region selected from 3-30/3-3/3, 3-33/1-7/4, 3-33/2-15/4 or 3-53/1-1/4; and
(c) employing the immunoglobulin variable region sequence obtained in (b) associated with said human light chain variable region in an antibody that specifically binds the antigen of interest.

Also referred to is a method for making an antibody, comprising expressing in a single cell (a) a first V_{H} gene sequence of an immunized mouse as described herein fused with a human C_{H} gene sequence; (b) a V_{L} gene sequence of an immunized mouse as described herein fused with a human C_{L} gene sequence; and, (c) maintaining the cell under conditions sufficient to express a fully human antibody, and isolating the antibody. In one instance, the cell comprises a second V_{H} gene sequence of a second immunized mouse as described herein fused with a human C_{H} gene sequence, the first V_{H} gene sequence encodes a V_{H} domain that recognizes a first epitope, and the second V_{H} gene sequence encodes a V_{H} domain that recognizes a second epitope, wherein the first epitope and the second epitope are not identical.

Also referred to is a method for making an epitope-binding protein, comprising exposing a mouse as described herein with an immunogen that comprises an epitope of interest, maintaining the mouse under conditions sufficient for the mouse to generate an immunoglobulin molecule that specifically binds the epitope of interest, and isolating the immunoglobulin molecule that specifically binds the epitope of interest;
wherein the epitope-binding protein comprises a heavy chain that comprises a somatically mutated human V_{H} and a mouse C_{H}, associated with a light chain comprising a mouse C_{L} and a human V_{L} derived from a rearranged human Vκ1-39Jκ5 or a rearranged human Vκ3-20Jκ1.

Also referred to is a cell that expresses an epitope-binding protein wherein the cell comprises: (a) a human nucleotide sequence encoding a human V_{L} domain that is derived from a rearranged human Vκ1-39Jκ5 or a rearranged human Vκ3-20Jκ1, wherein the human nucleotide sequence is fused (directly or through a linker) to a human immunoglobulin light chain constant domain cDNA sequence (e.g., a human κ constant domain DNA sequence); and, (b) a first human V_{H} nucleotide sequence encoding a human V_{H} domain derived from a first human V_{H} nucleotide sequence, wherein the first human V_{H} nucleotide sequence is fused (directly or through a linker) to a human immunoglobulin heavy chain constant domain cDNA sequence; wherein the epitope-binding protein recognizes a first epitope. In one instance, the epitope-binding protein binds the first epitope with a dissociation constant of lower than 10⁻⁶ M, lower than 10⁻⁸ M, lower than 10⁻⁹ M, lower than 10⁻¹⁰ M, lower than 10⁻¹¹ M, or lower than 10⁻¹² M.

In one instance, the cell comprises a second human nucleotide sequence encoding a second human V_{H} domain, wherein the second human sequence is fused (directly or through a linker) to a human immunoglobulin heavy chain constant domain cDNA sequence, and wherein the second human V_{H} domain does not specifically recognize the first epitope (e.g., displays a dissociation constant of, e.g., 10⁻⁶ M, 10⁻⁵ M, 10⁻⁴ M, or higher), and wherein the epitope-binding protein recognizes the first epitope and the second epitope, and wherein the first and the second immunoglobulin heavy chains each associate with an identical light chain of (a).

In one instance, the second V_{H} domain binds the second epitope with a dissociation constant that is lower than 10⁻⁶ M, lower than 10⁻⁷ M, lower than 10⁻⁸ M, lower than 10⁻⁹ M, lower than 10⁻¹⁰ M, lower than 10⁻¹¹ M, or lower than 10⁻¹² M.

In one instance, the epitope-binding protein comprises a first immunoglobulin heavy chain and a second immunoglobulin heavy chain, each associated with an identical light chain derived from a rearranged human V_{L} region selected from a human Vκ1-39Jκ5 or a human Vκ3-20Jκ1, wherein the first immunoglobulin heavy chain binds a first epitope with a dissociation constant in the nanomolar to picomolar range, the second immunoglobulin heavy chain binds a second epitope with a dissociation constant in the nanomolar to picomolar range, the first epitope and the second epitope are not identical, the first immunoglobulin heavy chain does not bind the second epitope or binds the second epitope with a dissociation constant weaker than the micromolar range (e.g., the millimolar range), the second immunoglobulin heavy chain does not bind the first epitope or binds the first epitope with a dissociation constant weaker than the micromolar range (e.g., the millimolar range), and one or more of the V_{L}, the V_{H} of the first immunoglobulin heavy chain, and the V_{H} of the second immunoglobulin heavy chain, are somatically mutated.

In one instance, the first immunoglobulin heavy chain comprises a protein A-binding residue, and the second immunoglobulin heavy chain lacks the protein A-binding residue.

In one instance, the cell is selected from CHO, COS, 293, HeLa, and a retinal cell expressing a viral nucleic acid sequence (e.g., a PERC.6™ cell).

A reverse chimeric antibody is also referred to comprising a human V_{H} and a mouse heavy chain constant domain, a human V_{L} and a mouse light chain constant domain, wherein the antibody is made by a process that comprises immunizing a mouse as described herein with an immunogen comprising an epitope, and the antibody specifically binds the epitope of the immunogen with which the mouse was immunized. In one instance, the V_{L} domain is somatically mutated. In one instance the V_{H} domain is somatically mutated. In one instance, both the V_{L} domain and the V_{H} domain are somatically mutated. In one instance, the V_{L} is linked to a mouse Cκ domain.

In one aspect, in the mouse provided, the mouse comprises human VH gene segments replacing all or substantially all mouse V_{H} gene segments at the endogenous mouse heavy chain locus; no more than one or two rearranged human light chain V_{L}/J_{L} sequences selected from a rearranged Vκ1-39/Jκ5 and a rearranged Vκ3-20/Jκ1 or a combination thereof, replacing all mouse light chain gene segments; wherein the human heavy chain variable gene segments are linked to a mouse constant gene, and the rearranged human light chain sequences are linked to a human or mouse constant gene.

Also referred to is a mouse ES cell comprising a replacement of all or substantially all mouse heavy chain variable gene segments with human heavy chain variable gene segments, and no more than one or two rearranged human light chain V_{L}/J_{L} sequences, wherein the human heavy chain variable gene segments are linked to a mouse immunoglobulin heavy chain constant gene, and the rearranged human light chain V_{L}/J_{L} sequences are linked to a mouse or human immunoglobulin light chain constant gene. In a specific instance, the light chain constant gene is a mouse constant gene.

Also referred to is an antigen-binding protein made by a mouse as described herein. In a specific instance, the antigen-binding protein comprises a human immunoglobulin heavy chain variable region fused with a mouse constant region, and a human immunoglobulin light chain variable region derived from a Vκ1-39 gene segment or a Vκ3-20 gene segment, wherein the light chain constant region is a mouse constant region.

Also referred to is a fully human antigen-binding protein made from an immunoglobulin variable region gene sequence from a mouse as described herein, wherein the antigen-binding protein comprises a fully human heavy chain comprising a human variable region derived from a sequence of a mouse as described herein, and a fully human light chain comprising a Vκ1-39 or a Vκ3-20. In one instance, the light chain variable region comprises one to five somatic mutations. In one instance, the light chain variable region is a cognate light chain variable region that is paired in a B cell of the mouse with the heavy chain variable region.

In one instance, the fully human antigen-binding protein comprises a first heavy chain and a second heavy chain, wherein the first heavy chain and the second heavy chain comprise non-identical variable regions independently derived from a mouse as described herein, and wherein each of the first and second heavy chains express from a host cell associated with a human light chain derived from a Vκ1-39 gene segment or a Vκ3-20 gene segment. In one instance, the first heavy chain comprises a first heavy chain variable region that specifically binds a first epitope of a first antigen, and the second heavy chain comprises a second heavy chain variable region that specifically binds a second epitope of a second antigen. In a specific instance, the first antigen and the second antigen are different. In a specific instance, the first antigen and the second antigen are the same, and the first epitope and the second epitope are not identical; in a specific instance, binding of the first epitope by a first molecule of the binding protein does not block binding of the second epitope by a second molecule of the binding protein.

In one instance, a fully human binding protein derived from a human immunoglobulin sequence of a mouse as described herein comprises a first immunoglobulin heavy chain and a second immunoglobulin heavy chain, wherein the first immunoglobulin heavy chain comprises a first variable region that is not identical to a variable region of the second immunoglobulin heavy chain, and wherein the first immunoglobulin heavy chain comprises a wild type protein A binding determinant, and the second heavy chain lacks a wild type protein A binding determinant. In one instance, the first immunoglobulin heavy chain binds protein A under isolation conditions, and the second immunoglobulin heavy chain does not bind protein A or binds protein A at least 10-fold, a hundred-fold, or a thousand fold weaker than the first immunoglobulin heavy chain binds protein A under isolation conditions. In a specific instance, the first and the second heavy chains are lgG1 isotypes, wherein the second heavy chain comprises a modification selected from 95R (EU 435R), 96F (EU 436F), and a combination thereof, and wherein the first heavy chain lacks such modification.

Also referred to is a method for making a bispecific antigen-binding protein, comprising exposing a first mouse as described herein to a first antigen of interest that comprises a first epitope, exposing a second mouse as described herein to a second antigen of interest that comprises a second epitope, allowing the first and the second mouse to each mount immune responses to the antigens of interest, identifying in the first mouse a first human heavy chain variable region that binds the first epitope of the first antigen of interest, identifying in the second mouse a second human heavy chain variable region that binds the second epitope of the second antigen of interest, making a first fully human heavy chain gene that encodes a first heavy chain that binds the first epitope of the first antigen of interest, making a second fully human heavy chain gene that encodes a second heavy chain that binds the second epitope of the second antigen of interest, expressing the first heavy chain and the second heavy chain in a cell that expresses a single fully human light chain derived from a human Vκ1-39 or a human Vκ3-20 gene segment to form a bispecific antigen-binding protein, and isolating the bispecific antigen- binding protein.

In one instance, the first antigen and the second antigen are not identical.

In one instance, the first antigen and the second antigen are identical, and the first epitope and the second epitope are not identical. In one instance, binding of the first heavy chain variable region to the first epitope does not block binding of the second heavy chain variable region to the second epitope.

In one instance, the first antigen is selected from a soluble antigen and a cell surface antigen (e.g., a tumor antigen), and the second antigen comprises a cell surface receptor. In a specific instance, the cell surface receptor is an immunoglobulin receptor. In a specific instance, the immunoglobulin receptor is an Fc receptor. In one instance, the first antigen and the second antigen are the same cell surface receptor, and binding of the first heavy chain to the first epitope does not block binding of the second heavy chain to the second epitope.

In one instance, the light chain variable domain of the light chain comprises 2 to 5 somatic mutations. In one instance, the light chain variable domain is a somatically mutated cognate light chain expressed in a B cell of the first or the second immunized mouse with either the first or the second heavy chain variable domain.

In one instance, the first fully human heavy chain bears an amino acid modification that reduces its affinity to protein A, and the second fully human heavy chain does not comprise a modification that reduces its affinity to protein A.

Also referred to is an antibody or a bispecific antibody comprising a human heavy chain variable domain made in accordance with the invention. Also referred to is use of a mouse as described herein to make a fully human antibody or a fully human bispecific antibody.

In one instance, a genetically modified mouse, embryo, or cell described herein comprises a κ light chain locus that retains endogenous regulatory or control elements, e.g., a mouse κ intronic enhancer, a mouse κ 3' enhancer, or both an intronic enhancer and a 3' enhancer, wherein the regulatory or control elements facilitate somatic mutation and affinity maturation of an expressed sequence of the κ light chain locus.

Also referred to is a mouse that comprises a B cell population characterized by having immunoglobulin light chains derived from no more than one, or no more than two, rearranged or unrearranged immunoglobulin light chain V and J gene segments, wherein the mouse exhibits a κ:λ light chain ratio that is about the same as a mouse that comprises a wild type complement of immunoglobulin light chain V and J gene segments.

In one instance, the immunoglobulin light chains are derived from no more than one, or no more than two, rearranged immunoglobulin light chain V and J gene segments. In a specific embodiment, the light chains are derived from no more than one rearranged immunoglobulin light chain V and J gene segments.

In one instance, the mouse exhibits a κ:λ light chain ratio that is about from 55:1 to 75:1, 60:1 to 70:1, 63:1 to 68: 1, or about from 65:1 to 67:1 as compared to a mouse that comprises a wild type complement of immunoglobulin light chain V and J gene segments. In a specific instance, the mouse exhibits a κ:λ light chain ratio that is 66:1 as compared to a mouse that comprises a wild type complement of immunoglobulin light chain V and J gene segments. In one instance, the immunoglobulin light chains derived from no more than one, or no more than two, rearranged or unrearranged immunoglobulin light chain V and J gene segments include human Vκ and J gene segments selected from human Vκ1-39, human Vκ3-20, human Jκ1 and human Jκ5. In a specific instance, the immunoglobulin light chains are derived from a single human light chain sequence comprising a human VK1-39 sequence.

In one instance, the mouse exhibits a κ:λ light chain ratio that is about from 18:1 to 23:1 or about from 19:1 to 22:1 as compared to a mouse that comprises a wild type complement of immunoglobulin light chain V and J gene segments. In one instance, the mouse exhibits a κ:λ light chain ratio that is 21:1 as compared to a mouse that comprises a wild type complement of immunoglobulin light chain V and J gene segments. In one instance, the mouse exhibits a κ:λ light chain ratio that is about the same or 20:1 as compared to a mouse that comprises a wild type complement of immunoglobulin light chain V and J gene segments. In one instance, the immunoglobulin light chains derived from no more than one, or no more than two, rearranged or unrearranged immunoglobulin light chain V and J gene segments include human Vκ and Jκ gene segments selected from human Vκ1-39, human Vκ3-20, human Jκ1 and human Jκ5. In a specific instance, the immunoglobulin light chains are derived from a single human light chain sequence comprising a human Vκ3-20 sequence.

Also referred to is a mouse that expresses an immunoglobulin light chain derived from no more than one, or no more than two, human Vκ/Jκ sequences, wherein the mouse comprises a replacement of all or substantially all endogenous mouse heavy chain variable region gene segments with one or more human heavy chain variable region gene segments, and the mouse exhibits a ratio of (a) CD19⁺ B cells that express an immunoglobulin having a λ light chain, to (b) CD19⁺ B cells that express an immunoglobulin having a κ light chain, of about 1 to about 20.

In one instance, the mouse expresses a single κ light chain derived from a human Vκ1-39Jκ5 sequence, and the ratio of CD19⁺ B cells that express an immunoglobulin having a λ light chain to CD19⁺ B cells that express an immunoglobulin having a κ light chain is about 1 to about 20; in one instance, the ratio is about 1 to at least about 66; in a specific embodiment, the ratio is about 1 to 66.

In one instance, the mouse expresses a single light chain derived from a human Vκ3-20Jκ5 sequence, and the ratio of CD19⁺ B cells that express an immunoglobulin having a λ light chain to CD19⁺ B cells that express an immunoglobulin having a κ light chain is about 1 to about 20; in one instance, the ratio is about 1 to about 21. In specific embodiments, the ratio is 1 to 20, or 1 to 21.

Also referred to is a genetically modified mouse is provided that expresses a single rearranged κ light chain, wherein the mouse comprises a functional λ light chain locus, and wherein the mouse expresses a B cell population that comprises Igκ+ cells that express a κ light chain derived from the same single rearranged κ light chain. In one instance, the percent of Igκ⁺Igλ⁺ B cells in the mouse is about the same as in a wild type mouse. In a specific instance, the percent of Igκ⁺Igλ⁺ B cells in the mouse is about 2 to about 6 percent. In a specific instance, the percent of Igκ⁺Igλ⁺ B cells in a mouse wherein the single rearranged κ light chain is derived from a Vκ1-39Jκ5 sequence is about 2 to about 3; in a specific instance, about 2.6. In a specific embodiment, the percent of Igκ⁺Igλ⁺ B cells in a mouse wherein the single rearranged κ light chain is derived from a Vκ3-20Jκ1 sequence is about 4 to about 8; in a specific embodiment, about 6.

Also referred to is a genetically modified mouse, wherein the mouse expresses a single rearranged κ light chain derived from a human Vκ and Jκ gene segment, wherein the mouse expresses a B cell population that comprises a single κ light chain derived from the single rearranged κ light chain sequence, wherein the genetically modified mouse has not been rendered resistant to somatic hypermutations. In one embodiment, at least 90% of the κ light chains expressed on a B cell of the mouse exhibit from at least one to about five somatic hypermutations.

Also referred to is a genetically modified mouse that is modified to express a single κ light chain derived from no more than one, or no more than two, rearranged κ light chain sequences, wherein the mouse exhibits a κ light chain usage that is about twofold or more, at least about three-fold or more, or at least about four-fold or more greater than the κ light chain usage exhibited by a wild type mouse, or greater than the κ light chain usage exhibited by a mouse of the same strain that comprises a wild type repertoire of κ light chain gene segments. In a specific instance, the mouse expresses the single κ light chain from no more than one rearranged κ light chain sequence. In a more specific instance, the rearranged κ light chain sequence is selected from a Vκ1-39Jκ5 and Vκ3-20Jκ1 sequence. In one instance, the rearranged κ light chain sequence is a Vκ1-39Jκ5 sequence. In one instance, the rearranged κ light chain sequence is a Vκ3-20Jκ1 sequence.

Also referred to is a genetically modified mouse that expresses a single κ light chain derived from no more than one, or no more than two, rearranged κ light chain sequences, wherein the mouse exhibits a κ light chain usage that is about 100-fold or more, at least about 200-fold or more, at least about 300-fold or more, at least about 400- fold or more, at least about 500-fold or more, at least about 600-fold or more, at least about 700-fold or more, at least about 800-fold or more, at least about 900-fold or more, at least about 1000-fold or more greater than the same κ light chain usage exhibited by a mouse bearing a complete or substantially complete human κ light chain locus. In a specific instance, the mouse bearing a complete or substantially complete human κ light chain locus lacks a functional unrearranged mouse κ light chain sequence. In a specific instance, the mouse expresses the single κ light chain from no more than one rearranged κ light chain sequence. In one instance, the mouse comprises one copy of a rearranged κ light chain sequence (e.g., a heterozygote). In one embodiment, the mouse comprises two copies of a rearranged κ light chain sequence (e.g., a homozygote). In a more specific instance, the rearranged κ light chain sequence is selected from a Vκ1-39Jκ5 and Vκ3-20Jκ1 sequence. In one instance, the rearranged κ light chain sequence is a Vκ1-39Jκ5 sequence. In one instance, the rearranged κ light chain sequence is a Vκ3-20Jκ1 sequence.

Also referred to is a genetically modified mouse that expresses a single light chain derived from no more than one, or no more than two, rearranged light chain sequences, wherein the light chain in the genetically modified mouse exhibits a level of expression that is at least 10-fold to about 1,000-fold, 100-fold to about 1,000-fold, 200-fold to about 1,000-fold, 300-fold to about 1,000-fold, 400-fold to about 1,000-fold, 500-fold to about 1,000-fold, 600-fold to about 1,000-fold, 700-fold to about 1,000-fold, 800-fold to about 1,000-fold, or 900-fold to about 1,000-fold higher than expression of the same rearranged light chain exhibited by a mouse bearing a complete or substantially complete light chain locus. In one instance, the light chain comprises a human sequence. In a specific instance, the human sequence is a κ sequence. In one instance, the human sequence is a λ sequence. In one instance, the light chain is a fully human light chain.

In one instance, the level of expression is characterized by quantitating mRNA of transcribed light chain sequence, and comparing it to transcribed light chain sequence of a mouse bearing a complete or substantially complete light chain locus.

Also referred to is a genetically modified mouse that expresses a single κ light chain derived from no more than one, or no more than two, rearranged κ light chain sequences, wherein the mouse, upon immunization with antigen, exhibits a serum titer that is comparable to a wild type mouse immunized with the same antigen. In a specific instance, the mouse expresses a single κ light chain from no more than one rearranged κ light chain sequence. In one instance, the serum titer is characterized as total immunoglobulin. In a specific instance, the serum titer is characterized as IgM specific titer. In a specific instance, the serum titer is characterized as IgG specific titer. In a more specific instance, the rearranged κ light chain sequence is selected from a Vκ1-39Jκ5 and Vκ3-20Jκ1 sequence. In one instance, the rearranged κ light chain sequence is a Vκ1-39Jκ5 sequence. In one instance, the rearranged κ light chain sequence is a Vκ3-20Jκ1 sequence.

Also referred to is a genetically modified mouse that expresses a plurality of immunoglobulin heavy chains associated with a single light chain. In one instance, the heavy chain comprises a human sequence. In various instances, the human sequence is selected from a variable sequence, a CH₁, a hinge, a CH₂, a CH₃, and a combination thereof. In one instance, the single light chain comprises a human sequence. In various instances, the human sequence is selected from a variable sequence, a constant sequence, and a combination thereof. In one instance, the mouse comprises a disabled endogenous immunoglobulin locus and expresses the heavy chain and/or the light chain from a transgene or extrachromosomal episome. In one instance, the mouse comprises a replacement at an endogenous mouse locus of some or all endogenous mouse heavy chain gene segments (i.e., V, D, J), and/or some or all endogenous mouse heavy chain constant sequences (e.g., CH₁, hinge, CH₂, CH₃, or a combination thereof), and/or some or all endogenous mouse light chain sequences (e.g., V, J, constant, or a combination thereof), with one or more human immunoglobulin sequences.

Also referred to is a mouse suitable for making antibodies that have the same light chain, wherein all or substantially all antibodies made in the mouse are expressed with the same light chain. In one instance, the light chain is expressed from an endogenous light chain locus.

Also referred to is a method for making a light chain for a human antibody, comprising obtaining from a mouse as described herein a light chain sequence and a heavy chain sequence, and employing the light chain sequence and the heavy chain sequence in making a human antibody. In one instance, the human antibody is a bispecific antibody.

[Deleted].

### BRIEF DESCRIPTION OF THE FIGURES

**FIG. 1** illustrates a targeting strategy for replacing endogenous mouse immunoglobulin light chain variable region gene segments with a human Vκ1-39Jκ5 gene region.
**FIG. 2** illustrates a targeting strategy for replacing endogenous mouse immunoglobulin light chain variable region gene segments with a human Vκ3-20Jκ1 gene region.
**FIG. 3** illustrates a targeting strategy for replacing endogenous mouse immunoglobulin light chain variable region gene segments with a human VpreB/Jλ5 gene region.
**FIG. 4** shows the percent of CD19⁺ B cells (y-axis) from peripheral blood for wild type mice (WT), mice homozygous for an engineered human rearranged Vκ4-39Jκ5 light chain region (Vκ1-39Jκ5 HO) and mice homozygous for an engineered human rearranged Vκ3-20Jκ1 light chain region (Vκ3-20Jκ1 HO).

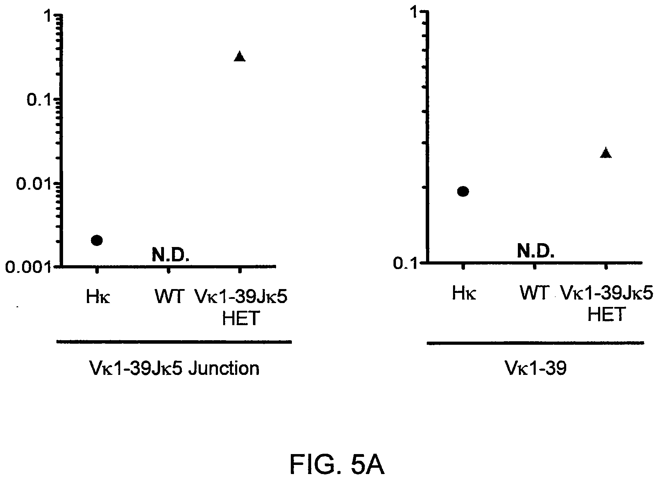

**FIG. 5A** shows the relative mRNA expression (y-axis) of a Vκ1-39-derived light chain in a quantitative PCR assay using probes specific for the junction of an engineered human rearranged Vκ4-39Jκ5 light chain region (Vκ1-39Jκ5 Junction Probe) and the human Vκ1-39 gene segment (Vκ1-39 Probe) in a mouse homozygous for a replacement of the endogenous Vκ and Jκ gene segments with human Vκ and Jκ gene segments (Hκ), a wild type mouse (WT), and a mouse heterozygous for an engineered human rearranged Vκ4-39Jκ5 light chain region (Vκ1-39Jκ5 HET). Signals are normalized to expression of mouse Cκ. N.D.: not detected.
**FIG. 5B** shows the relative mRNA expression (y-axis) of a Vκ1-39-derived light chain in a quantitative PCR assay using probes specific for the junction of an engineered human rearranged Vκ4-39Jκ5 light chain region (Vκ1-39Jκ5 Junction Probe) and the human Vκ1-39 gene segment (Vκ1-39 Probe) in a mouse homozygous for a replacement of the endogenous Vκ and Jκ gene segments with human Vκ and Jκ gene segments (Hκ), a wild type mouse (WT), and a mouse homozygous for an engineered human rearranged Vκ4-39Jκ5 light chain region (Vκ1-39Jκ5 HO). Signals are normalized to expression of mouse Cκ.

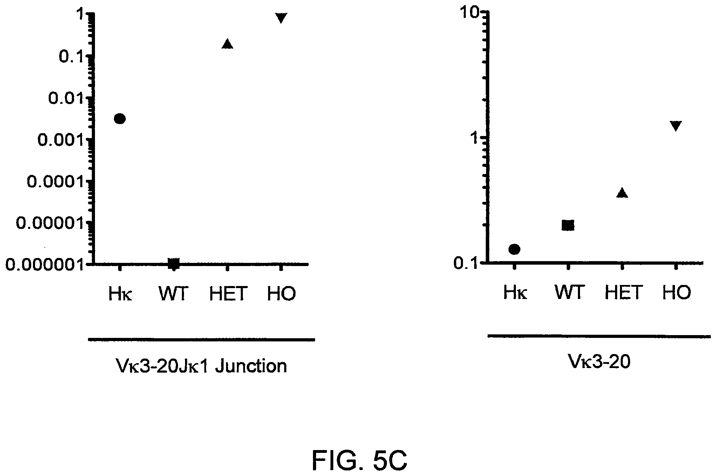

**FIG. 5C** shows the relative mRNA expression (y-axis) of a Vκ3-20-derived light chain in a quantitative PCR assay using probes specific for the junction of an engineered human rearranged Vκ3-20Jκ1 light chain region (Vκ3-20Jκ1 Junction Probe) and the human Vκ3-20 gene segment (Vκ3-20 Probe) in a mouse homozygous for a replacement of the endogenous Vκ and Jκ gene segments with human Vκ and Jκ gene segments (Hκ), a wild type mouse (WT), and a mouse heterozygous (HET) and homozygous (HO) for an engineered human rearranged Vκ3-20Jκ1 light chain region. Signals are normalized to expression of mouse Cκ.
**FIG. 6A** shows IgM (left) and IgG (right) titer in wild type (WT; N=2) and mice homozygous for an engineered human rearranged Vκ4-39Jκ5 light chain region (Vκ1-39Jκ5 HO; N=2) immunized with β-galactosidase.
**FIG. 6B** shows total immunoglobulin (IgM, IgG, IgA) titer in wild type (WT; N=5) and mice homozygous for an engineered human rearranged Vκ3-20Jκ1 light chain region (Vκ3-20Jκ1 HO; N=5) immunized with β-galactosidase.

### DETAILED DESCRIPTION

This invention is not limited to particular methods, and experimental conditions described, as such methods and conditions may vary. It is also to be understood that the terminology used herein is for the purpose of describing particular embodiments only, and is not intended to be limiting, since the scope of the present invention is defined by the claims.

[Deleted].

The term "antibody", as used herein, includes immunoglobulin molecules comprising four polypeptide chains, two heavy (H) chains and two light (L) chains inter-connected by disulfide bonds. Each heavy chain comprises a heavy chain variable (V_{H}) region and a heavy chain constant region (C_{H}). The heavy chain constant region comprises three domains, C_{H}1, C_{H}2 and C_{H}3. Each light chain comprises a light chain variable (V_{L}) region and a light chain constant region (C_{L}). The V_{H} and V_{L} regions can be further subdivided into regions of hypervariability, termed complementarity determining regions (CDR), interspersed with regions that are more conserved, termed framework regions (FR). Each V_{H} and V_{L} comprises three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. The term "high affinity" antibody refers to an antibody that has a K_{D} with respect to its target epitope about of 10⁻⁹ M or lower (*e.g*., about 1 x 10⁻⁹ M, 1 x 10⁻¹⁰ M, 1 x 10⁻¹¹ M, or about 1 x 10⁻¹² M). In one embodiment, K_{D} is measured by surface plasmon resonance, *e.g*., BIACORE™; in another embodiment, K_{D} is measured by ELISA.
The phrase "bispecific antibody" includes an antibody capable of selectively binding two or more epitopes. Bispecific antibodies generally comprise two nonidentical heavy chains, with each heavy chain specifically binding a different epitope-either on two different molecules (*e.g*., different epitopes on two different immunogens) or on the same molecule (*e.g*., different epitopes on the same immunogen). If a bispecific antibody is capable of selectively binding two different epitopes (a first epitope and a second epitope), the affinity of the first heavy chain for the first epitope will generally be at least one to two or three or four or more orders of magnitude lower than the affinity of the first heavy chain for the second epitope, and vice versa. Epitopes specifically bound by the bispecific antibody can be on the same or a different target (*e.g*., on the same or a different protein). Bispecific antibodies can be made, for example, by combining heavy chains that recognize different epitopes of the same immunogen. For example, nucleic acid sequences encoding heavy chain variable sequences that recognize different epitopes of the same immunogen can be fused to nucleic acid sequences encoding the same or different heavy chain constant regions, and such sequences can be expressed in a cell that expresses an immunoglobulin light chain. A typical bispecific antibody has two heavy chains each having three heavy chain CDRs, followed by (N-terminal to C-terminal) a C_{H}1 domain, a hinge, a C_{H}2 domain, and a C_{H}3 domain, and an immunoglobulin light chain that either does not confer epitope-binding specificity but that can associate with each heavy chain, or that can associate with each heavy chain and that can bind one or more of the epitopes bound by the heavy chain epitope-binding regions, or that can associate with each heavy chain and enable binding or one or both of the heavy chains to one or both epitopes.

The term "cell" includes any cell that is suitable for expressing a recombinant nucleic acid sequence. Cells include those of prokaryotes and eukaryotes (single-cell or multiple-cell), bacterial cells (e.g., strains of *E. coli, Bacillus spp., Streptomyces spp., etc*.), mycobacteria cells, fungal cells, yeast cells (e.g., *S. cerevisiae, S. pombe, P. pastoris, P. methanolica, etc*.)*,* plant cells, insect cells (e.g., SF-9, SF-21 , baculovirus-infected insect cells, *Trichoplusia ni, etc*.), non-human animal cells, human cells, or cell fusions such as, for example, hybridomas or quadromas. In some instances, the cell is a human, monkey, ape, hamster, rat, or mouse cell. In some instance, the cell is eukaryotic and is selected from the following cells: CHO (e.g., CHO K1, DXB-1 1 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, e.g., a retinal cell that expresses a viral gene (e.g., a PER.C6™ cell).

The phrase "complementarity determining region," or the term "CDR," includes an amino acid sequence encoded by a nucleic acid sequence of an organism's immunoglobulin genes that normally (i.e., in a wild type animal) appears between two framework regions in a variable region of a light or a heavy chain of an immunoglobulin molecule (e.g., an antibody or a T cell receptor). A CDR can be encoded by, for example, a germline sequence or a rearranged or unrearranged sequence, and, for example, by a naive or a mature B cell or a T cell. A CDR can be somatically mutated (e.g., vary from a sequence encoded in an animal's germline), humanized, and/or modified with amino acid substitutions, additions, or deletions. In some circumstances (e.g., for a CDR3), CDRs can be encoded by two or more sequences (e.g., germline sequences) that are not contiguous (e.g., in an unrearranged nucleic acid sequence) but are contiguous in a B cell nucleic acid sequence, e.g., as the result of splicing or connecting the sequences (e.g., V-D-J recombination to form a heavy chain CDR3).

The term "conservative," when used to describe a conservative amino acid substitution, includes substitution of an amino acid residue by another amino acid residue having a side chain R group with similar chemical properties (e.g., charge or hydrophobicity). In general, a conservative amino acid substitution will not substantially change the functional properties of interest of a protein, for example, the ability of a variable region to specifically bind a target epitope with a desired affinity. Examples of groups of amino acids that have side chains with similar chemical properties include aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and, sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be substitution of any native residue in a protein with alanine, as used in, for example, alanine scanning mutagenesis. In some embodiments, a conservative substitution is made that has a positive value in the PAM250 log-likelihood matrix disclosed in Gonnet et al. (1992) Exhaustive Matching of the Entire Protein Sequence Database, Science 256:1443-45. In some embodiments, the substitution is a moderately conservative substitution wherein the substitution has a nonnegative value in the PAM250 log-likelihood matrix.

In some instances, residue positions in an immunoglobulin light chain or heavy chain differ by one or more conservative amino acid substitutions. In some embodiments, residue positions in an immunoglobulin light chain or functional fragment thereof (e.g., a fragment that allows expression and secretion from, e.g., a B cell) are not identical to a light chain whose amino acid sequence is listed herein, but differs by one or more conservative amino acid substitutions.

The phrase "epitope-binding protein" includes a protein having at least one CDR and that is capable of selectively recognizing an epitope, e.g., is capable of binding an epitope with a K_{D} that is at about one micromolar or lower (e.g., a K_{D} that is about 1 x 10⁻⁶ M, 1 x 10⁻⁷ M, 1 x 10⁻⁹ M , 1 x 10⁻⁹ M, 1 x 10⁻¹⁰ M, 1 x 10⁻¹¹ M, or about 1 x 10⁻¹² M). Therapeutic epitope-binding proteins (*e.g*., therapeutic antibodies) frequently require a K_{D} that is in the nanomolar or the picomolar range.

The phrase "functional fragment" includes fragments of epitope-binding proteins that can be expressed, secreted, and specifically bind to an epitope with a K_{D} in the micromolar, nanomolar, or picomolar range. Specific recognition includes having a K_{D} that is at least in the micromolar range, the nanomolar range, or the picomolar range.

The term "germline" includes reference to an immunoglobulin nucleic acid sequence in a non-somatically mutated cell, *e.g.,* a non-somatically mutated B cell or pre-B cell or hematopoietic cell.

The phrase "heavy chain," or "immunoglobulin heavy chain" includes an immunoglobulin heavy chain constant region sequence from any organism. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a C_{H}1 domain, a hinge, a C_{H}2 domain, and a C_{H}3 domain. A functional fragment of a heavy chain includes a fragment that is capable of specifically recognizing an epitope (*e.g*., recognizing the epitope with a K_{D} in the micromolar, nanomolar, or picomolar range), that is capable of expressing and secreting from a cell, and that comprises at least one CDR.

The term "identity" when used in connection with sequence includes identity as determined by a number of different algorithms known in the art that can be used to measure nucleotide and/or amino acid sequence identity. In some embodiments described herein, identities are determined using a ClustalW v. 1.83 (slow) alignment employing an open gap penalty of 10.0, an extend gap penalty of 0.1, and using a Gonnet similarity matrix (MACVECTOR™ 10.0.2, MacVector Inc., 2008). The length of the sequences compared with respect to identity of sequences will depend upon the particular sequences, but in the case of a light chain constant domain, the length should contain sequence of sufficient length to fold into a light chain constant domain that is capable of self-association to form a canonical light chain constant domain, *e.g.,* capable of forming two beta sheets comprising beta strands and capable of interacting with at least one C_{H}1 domain of a human or a mouse. In the case of a C_{H}1 domain, the length of sequence should contain sequence of sufficient length to fold into a C_{H}1 domain that is capable of forming two beta sheets comprising beta strands and capable of interacting with at least one light chain constant domain of a mouse or a human.

The phrase "immunoglobulin molecule" includes two immunoglobulin heavy chains and two immunoglobulin light chains. The heavy chains may be identical or different, and the light chains may be identical or different.

The phrase "light chain" includes an immunoglobulin light chain sequence from any organism, and unless otherwise specified includes human κ and λ light chains and a VpreB, as well as surrogate light chains. Light chain variable (V_{L}) domains typically include three light chain CDRs and four framework (FR) regions, unless otherwise specified. Generally, a full-length light chain includes, from amino terminus to carboxyl terminus, a V_{L} domain that includes FR1-CDR1-FR2-CDR2-FR3-CDR3-FR4, and a light chain constant domain. Light chains include those, *e.g.,* that do not selectively bind either a first or a second epitope selectively bound by the epitope-binding protein in which they appear. Light chains also include those that bind and recognize, or assist the heavy chain with binding and recognizing, one or more epitopes selectively bound by the epitope-binding protein in which they appear. Common light chains are those derived from a rearranged human Vκ1-39Jκ5 sequence or a rearranged human Vκ3-20Jκ1 sequence, and include somatically mutated (*e.g*., affinity matured) versions.

The phrase "micromolar range" is intended to mean 1-999 micromolar; the phrase "nanomolar range" is intended to mean 1-999 nanomolar; the phrase "picomolar range" is intended to mean 1-999 picomolar.

The phrase "somatically mutated" includes reference to a nucleic acid sequence from a B cell that has undergone class-switching, wherein the nucleic acid sequence of an immunoglobulin variable region (*e.g.,* a heavy chain variable domain or including a heavy chain CDR or FR sequence) in the class-switched B cell is not identical to the nucleic acid sequence in the B cell prior to class-switching, such as, for example, a difference in a CDR or framework nucleic acid sequence between a B cell that has not undergone class-switching and a B cell that has undergone class-switching. "Somatically mutated" includes reference to nucleic acid sequences from affinity-matured B cells that are not identical to corresponding immunoglobulin variable region sequences in B cells that are not affinity-matured (*i.e.,* sequences in the genome of germline cells). The phrase "somatically mutated" also includes reference to an immunoglobulin variable region nucleic acid sequence from a B cell after exposure of the B cell to an epitope of interest, wherein the nucleic acid sequence differs from the corresponding nucleic acid sequence prior to exposure of the B cell to the epitope of interest. The phrase "somatically mutated" refers to sequences from antibodies that have been generated in an animal, *e.g.,* a mouse having human immunoglobulin variable region nucleic acid sequences, in response to an immunogen challenge, and that result from the selection processes inherently operative in such an animal.

The term "unrearranged," with reference to a nucleic acid sequence, includes nucleic acid sequences that exist in the germline of an animal cell.

The phrase "variable domain" includes an amino acid sequence of an immunoglobulin light or heavy chain (modified as desired) that comprises the following amino acid regions, in sequence from N-terminal to C-terminal (unless otherwise indicated): FR1, CDR1, FR2, CDR2, FR3, CDR3, FR4.

### Common Light Chain

Prior efforts to make useful multispecific epitope-binding proteins, *e.g.,* bispecific antibodies, have been hindered by variety of problems that frequently share a common paradigm: *in vitro* selection or manipulation of sequences to rationally engineer, or to engineer through trial-and-error, a suitable format for pairing a heterodimeric bispecific human immunoglobulin. Unfortunately, most if not all of the *in vitro* engineering approaches provide largely *ad hoc* fixes that are suitable, if at all, for individual molecules. On the other hand, *in vivo* methods for employing complex organisms to select appropriate pairings that are capable of leading to human therapeutics have not been realized.

Generally, native mouse sequences are frequently not a good source for human therapeutic sequences. For at least that reason, generating mouse heavy chain immunoglobulin variable regions that pair with a common human light chain is of limited practical utility. More *in vitro* engineering efforts would be expended in a trial-and-error process to try to humanize the mouse heavy chain variable sequences while hoping to retain epitope specificity and affinity while maintaining the ability to couple with the common human light chain, with uncertain outcome. At the end of such a process, the final product may maintain some of the specificity and affinity, and associate with the common light chain, but ultimately immunogenicity in a human would likely remain a profound risk.

Therefore, a suitable mouse for making human therapeutics would include a suitably large repertoire of human heavy chain variable region gene segments in place of endogenous mouse heavy chain variable region gene segments. The human heavy chain variable region gene segments should be able to rearrange and recombine with an endogenous mouse heavy chain constant domain to form a reverse chimeric heavy chain (*i.e.,* a heavy chain comprising a human variable domain and a mouse constant region). The heavy chain should be capable of class switching and somatic hypermutation so that a suitably large repertoire of heavy chain variable domains are available for the mouse to select one that can associate with the limited repertoire of human light chain variable regions.

A mouse that selects a common light chain for a plurality of heavy chains has a practical utility. In various embodiments, antibodies that express in a mouse that can only express a common light chain will have heavy chains that can associate and express with an identical or substantially identical light chain. This is particularly useful in making bispecific antibodies. For example, such a mouse can be immunized with a first immunogen to generate a B cell that expresses an antibody that specifically binds a first epitope. The mouse (or a mouse genetically the same) can be immunized with a second immunogen to generate a B cell that expresses an antibody that specifically binds the second epitope. Variable heavy regions can be cloned from the B cells and expresses with the same heavy chain constant region, and the same light chain, and expressed in a cell to make a bispecific antibody, wherein the light chain component of the bispecific antibody has been selected by a mouse to associate and express with the light chain component.

The inventors have engineered a mouse for generating immunoglobulin light chains that will suitably pair with a rather diverse family of heavy chains, including heavy chains whose variable regions depart from germline sequences, *e.g.,* affinity matured or somatically mutated variable regions. In various embodiments, the mouse is devised to pair human light chain variable domains with human heavy chain variable domains that comprise somatic mutations, thus enabling a route to high affinity binding proteins suitable for use as human therapeutics.

The genetically engineered mouse, through the long and complex process of antibody selection within an organism, makes biologically appropriate choices in pairing a diverse collection of human heavy chain variable domains with a limited number of human light chain options. In order to achieve this, the mouse is engineered to present a limited number of human light chain variable domain options in conjunction with a wide diversity of human heavy chain variable domain options. Upon challenge with an immunogen, the mouse maximizes the number of solutions in its repertoire to develop an antibody to the immunogen, limited largely or solely by the number or light chain options in its repertoire. In various embodiments, this includes allowing the mouse to achieve suitable and compatible somatic mutations of the light chain variable domain that will nonetheless be compatible with a relatively large variety of human heavy chain variable domains, including in particular somatically mutated human heavy chain variable domains.
To achieve a limited repertoire of light chain options, the mouse is engineered to render nonfunctional or substantially nonfunctional its ability to make, or rearrange, a native mouse light chain variable domain. This can be achieved, *e.g.,* by deleting the mouse's light chain variable region gene segments. The endogenous mouse locus can then be modified by an exogenous suitable human light chain variable region gene segment of choice, operably linked to the endogenous mouse light chain constant domain, in a manner such that the exogenous human variable region gene segments can combine with the endogenous mouse light chain constant region gene and form a rearranged reverse chimeric light chain gene (human variable, mouse constant). In various embodiments, the light chain variable region is capable of being somatically mutated. In various embodiments, to maximize ability of the light chain variable region to acquire somatic mutations, the appropriate enhancer(s) is retained in the mouse. For example, in modifying a mouse κ light chain locus to replace endogenous mouse κ light chain gene segments with human κ light chain gene segments, the mouse κ intronic enhancer and mouse 3' enhancer are functionally maintained, or undisrupted.

A genetically engineered mouse is provided that expresses a limited repertoire of reverse chimeric (human variable, mouse constant) light chains associated with a diversity of reverse chimeric (human variable, mouse constant) heavy chains. In various embodiments, the endogenous mouse κ light chain gene segments are deleted and replaced with a single (or two) rearranged human light chain region, operably linked to the endogenous mouse Cκ gene. In embodiments for maximizing somatic hypermutation of the rearranged human light chain region, the mouse κ intronic enhancer and the mouse κ 3' enhancer are maintained. In various embodiments, the mouse also comprises a nonfunctional λ light chain locus, or a deletion thereof or a deletion that renders the locus unable to make a λ light chain.

Hence a genetically modified mouse is provided comprising a B cell that expresses a human light chain variable (V_{L}) domain derived from a rearranged human Vκ1-39/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4. Also provided is a genetically modified comprising a B cell that expresses a human light chain variable (V_{L}) domain derived from a rearranged human Vκ3-20/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4.

The genetically engineered mouse provided in various embodiments, comprises a light chain variable region locus lacking endogenous mouse light chain V_{L} and J_{L} gene segments and comprising a rearranged human light chain variable region, which is a rearranged human Vκ1-39/Jκ sequence or a human Vκ3-20/Jκ sequence, operably linked to a mouse constant region, wherein the locus is capable of undergoing somatic hypermutation, and wherein the locus expresses a light chain comprising the human V_{L}/J_{L} sequence linked to a mouse constant region. Thus, in various embodiments, the locus comprises a mouse κ 3' enhancer, which is correlated with a normal, or wild type, level of somatic hypermutation.

The genetically engineered mouse in various embodiments when immunized with an antigen of interest generates B cells that exhibit a diversity of rearrangements of human immunoglobulin heavy chain variable regions that express and function with one or with two rearranged light chains, including embodiments where the one or two light chains comprise human light chain variable regions that comprise, e.g., 1 to 5 somatic mutations. In various embodiments, the human light chains so expressed are capable of associating and expressing with any human immunoglobulin heavy chain variable region expressed in the mouse. In one instance, the genetically modified mouse comprises a B cell that expresses a human V_{L} domain derived from a rearranged human Vκ1-39/Jκ sequence that is present in the germline of the mouse, wherein the mouse lacks an unrearranged endogenous immunoglobulin Vκ gene segment and an unrearranged endogenous immunoglobulin Jκ gene segment; and wherein the human light chain variable (V_{L}) domain(s) is associated with a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4. In another instance, the genetically modified mouse comprises a B cell that expresses a human light chain variable (V_{L}) domain derived from a rearranged human Vκ3-20/Jκ sequence that is present in the germline of the mouse, wherein the mouse lacks an unrearranged endogenous immunoglobulin Vκ gene segment and an unrearranged endogenous immunoglobulin JK gene segment; and wherein the human V_{L} domain(s) is associated with a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4.

### Epitope-binding Proteins Binding More Than One Epitope

The compositions and methods of described herein can be used to make binding proteins that bind more than one epitope with high affinity, e.g., bispecific antibodies. Advantages of the invention include the ability to select suitably high binding (e.g., affinity matured) heavy chain immunoglobulin chains each of which will associate with a single light chain.

Synthesis and expression of bispecific binding proteins has been problematic, in part due to issues associated with identifying a suitable light chain that can associate and express with two different heavy chains, and in part due to isolation issues. The methods and compositions described herein allow for a genetically modified mouse to select, through otherwise natural processes, a suitable light chain that can associate and express with more than one heavy chain, including heavy chains that are somatically mutated (e.g., affinity matured). Human V_{L} and V_{H} sequences from suitable B cells of immunized mice as described herein that express affinity matured antibodies having reverse chimeric heavy chains (i.e., human variable and mouse constant) can be identified and cloned in frame in an expression vector with a suitable human constant region gene sequence (e.g., a human lgG1). Two such constructs can be prepared, wherein each construct encodes a human heavy chain variable domain that binds a different epitope. One of the human V_{L}s (e.g., human Vκ1-39Jκ5 or human Vκ3-20Jκ1), in germline sequence or from a B cell wherein the sequence has been somatically mutated, can be fused in frame to a suitable human constant region gene (e.g., a human κ constant gene). These three fully human heavy and light constructs can be placed in a suitable cell for expression. The cell will express two major species: a homodimeric heavy chain with the identical light chain, and a heterodimeric heavy chain with the identical light chain. To allow for a facile separation of these major species, one of the heavy chains is modified to omit a Protein A-binding determinant, resulting in a differential affinity of a homodimeric binding protein from a heterodimeric binding protein. Compositions and methods that address this issue are described in USSN 12/832,838, filed 25 June 2010, entitled "Readily Isolated Bispecific Antibodies with Native Immunoglobulin Format," published as US 2010/0331527A1.

In one instance, an epitope-binding protein as described herein is referred to, wherein human V_{L} and V_{H} sequences are derived from mice described herein that have been immunized with an antigen comprising an epitope of interest.

Also referred to is an epitope-binding protein that comprises a first and a second polypeptide, the first polypeptide comprising, from N-terminal to C- terminal, a first epitope-binding region that selectively binds a first epitope, followed by a constant region that comprises a first C_{H}3 region of a human IgG selected from IgG1, IgG2, IgG4, and a combination thereof; and, a second polypeptide comprising, from N-terminal to C-terminal, a second epitope-binding region that selectively binds a second epitope, followed by a constant region that comprises a second C_{H}3 region of a human IgG selected from IgG1 , IgG2, IgG4, and a combination thereof, wherein the second C_{H}3 region comprises a modification that reduces or eliminates binding of the second C_{H}3 domain to protein A.

In one instance, the second C_{H}3 region comprises an H95R modification (by IMGT exon numbering; H435R by EU numbering). In another embodiment, the second CH3 region further comprises a Y96F modification (IMGT; Y436F by EU).

In one instance, the second C_{H}3 region is from a modified human lgG1, and further comprises a modification selected from the group consisting of D16E, L18M, N44S, K52N, V57M, and V82I (IMGT; D356E, L358M, N384S, K392N, V397M, and V422I by EU).

In one instance, the second C_{H}3 region is from a modified human lgG2, and further comprises a modification selected from the group consisting of N44S, K52N, and V82I (IMGT; N384S, K392N, and V422I by EU).

In one instance, the second C_{H}3 region is from a modified human IgG4, and further comprises a modification selected from the group consisting of Q15R, N44S, K52N, V57M, R69K, E79Q, and V82I (IMGT; Q355R, N384S, K392N, V397M, R409K, E419Q, and V4221 by EU).

One method referred to herein for making an epitope-binding protein that binds more than one epitope is to immunize a first mouse in accordance with the invention with an antigen that comprises a first epitope of interest, wherein the mouse comprises an endogenous immunoglobulin light chain variable region locus that does not contain an endogenous mouse V_{L} that is capable of rearranging and forming a light chain, wherein at the endogenous mouse immunoglobulin light chain variable region locus is a single rearranged human V_{L} region operably linked to the mouse endogenous light chain constant region gene, and the rearranged human V_{L} region is selected from a human Vκ1-39Jκ5 and a human Vκ3-20Jκ1, and the endogenous mouse V_{H} gene segments have been replaced in whole or in part with human V_{H} gene segments, such that immunoglobulin heavy chains made by the mouse are solely or substantially heavy chains that comprise human variable domains and mouse constant domains. When immunized, such a mouse will make a reverse chimeric antibody, comprising only one of two human light chain variable domains (e.g., one of human Vκ1-39Jκ5 or human Vκ3-20Jκ1). Once a B cell is identified that encodes a V_{H} that binds the epitope of interest, the nucleotide sequence of the V_{H} (and, optionally, the V_{L}) can be retrieved (e.g., by PCR) and cloned into an expression construct in frame with a suitable human immunoglobulin constant domain. This process can be repeated to identify a second V_{H} domain that binds a second epitope, and a second V_{H} gene sequence can be retrieved and cloned into an expression vector in frame to a second suitable immunoglobulin constant domain. The first and the second immunoglobulin constant domains can the same or different isotype, and one of the immunoglobulin constant domains (but not the other) can be modified as described herein or in US 2010/0331527A1, and epitope-binding protein can be expressed in a suitable cell and isolated based on its differential affinity for Protein A as compared to a homodimeric epitope-binding protein, e.g., as described in US 2010/0331527A1.

Also referred to is a method for making a bispecific epitope-binding protein, comprising identifying a first affinity-matured (e.g., comprising one or more somatic hypermutations) human V_{H} nucleotide sequence (V_{H}1) from a mouse as described herein, identifying a second affinity-matured (e.g., comprising one or more somatic hypermutations) human V_{H} nucleotide sequence (V_{H}2) from a mouse as described herein, cloning V_{H}1 in frame with a human heavy chain lacking a Protein A-determinant modification as described in US 2010/0331527A1 for form heavy chain 1 (HC1), cloning V_{H}2 in frame with a human heavy chain comprising a Protein A-determinant as described in US 2010/0331527A1 to form heavy chain 2 (HC2), introducing an expression vector comprising HC1 and the same or a different expression vector comprising HC2 into a cell, wherein the cell also expresses a human immunoglobulin light chain that comprises a human Vκ1-39/human Jκ5 or a human Vκ3-20/human Jκ1 fused to a human light chain constant domain, allowing the cell to express a bispecific epitope-binding protein comprising a V_{H} domain encoded by V_{H}1 and a V_{H} domain encoded by V_{H}2, and isolating the bispecific epitope-binding protein based on its differential ability to bind Protein A as compared with a monospecific homodimeric epitope-binding protein. In a specific instance, HC1 is an IgG1, and HC2 is an IgG1 that comprises the modification H95R (IMGT; H435R by EU) and further comprises the modification Y96F (IMGT; Y436F by EU). In one instance, the V_{H} domain encoded by V_{H}1, the V_{H} domain encoded by V_{H}2, or both, are somatically mutated.

### Human V_{H} Genes That Express with a Common Human V_{L}

A variety of human variable regions from affinity-matured antibodies raised against four different antigens were expressed with either their cognate light chain, or at least one of a human light chain selected from human Vκ1-39Jκ5, human Vκ3-20Jκ1, or human VpreBJλ5 (see Example 1). For antibodies to each of the antigens, somatically mutated high affinity heavy chains from different gene families paired successfully with rearranged human germline Vκ1-39Jκ5 and Vκ3-20Jκ1 regions and were secreted from cells expressing the heavy and light chains. For Vκ1-39Jκ5 and Vκ3-20Jκ1, V_{H} domains derived from the following human V_{H} gene families expressed favorably: 1-2, 1-8, 1-24, 2-5, 3-7, 3-9, 3-11, 3-13, 3-15, 3-20, 3-23, 3-30, 3-33, 3-48, 4-31, 4-39, 4-59, 5-51, and 6-1. Thus, a mouse that is engineered to express a limited repertoire of human V_{L} domains from one or both of Vκ1-39Jκ5 and VκK3-20Jκ1 will generate a diverse population of somatically mutated human V_{H} domains from a V_{H} locus modified to replace mouse V_{H} gene segments with human V_{H} gene segments.

Mice genetically engineered to express reverse chimeric (human variable, mouse constant) immunoglobulin heavy chains associated with a single rearranged light chain (*e.g.,* a Vκ1-39/J or a Vκ3-20/J), when immunized with an antigen of interest, generated B cells that comprised a diversity of human V_{H} rearrangements and expressed a diversity of high-affinity antigen-specific antibodies with diverse properties with respect to their ability to block binding of the antigen to its ligand, and with respect to their ability to bind variants of the antigen (see Examples 5 through 10).

Thus, the mice and methods described herein are useful in making and selecting human immunoglobulin heavy chain variable domains, including somatically mutated human heavy chain variable domains, that result from a diversity of rearrangements, that exhibit a wide variety of affinities (including exhibiting a K_{D} of about a nanomolar or less), a wide variety of specificities (including binding to different epitopes of the same antigen), and that associate and express with the same or substantially the same human immunoglobulin light chain variable region.

The following examples are provided so as to describe to those of ordinary skill in the art how to make and use methods and compositions of the invention, and are not intended to limit the scope of what the inventors regard as their invention. Efforts have been made to ensure accuracy with respect to numbers used (*e.g*., amounts, temperature, *etc*.) but some experimental errors and deviations should be accounted for. Unless indicated otherwise, parts are parts by weight, molecular weight is average molecular weight, temperature is indicated in Celsius, and pressure is at or near atmospheric.

### EXAMPLES

The following examples are provided so as to describe how to make and use methods and compositions of the invention, and are not intended to limit the scope of what the inventors regard as their invention. Unless indicated otherwise, temperature is indicated in Celsius, and pressure is at or near atmospheric.

### Example 1

### Identification of Human V_{H} Regions That Associate with Selected Human V_{L} Regions

An *in vitro* expression system was constructed to determine if a single rearranged human germline light chain could be co-expressed with human heavy chains from antigen specific human antibodies.

Methods for generating human antibodies in genetically modified mice are known (see *e.g.,* US 6,596,541, Regeneron Pharmaceuticals, VELOCIMMUNE®). The VELOCIMMUNE® technology involves generation of a genetically modified mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibodies produced from a VELOCIMMUNE® mouse are fully human. Initially, high affinity chimeric antibodies are isolated having a human variable region and a mouse constant region. As described below, the antibodies are characterized and selected for desirable characteristics, including affinity, selectivity, epitope, *etc.* The mouse constant regions are replaced with a desired human constant region to generate a fully human antibody containing a non-IgM isotype, for example, wild type or modified IgG1, IgG2, IgG3 or IgG4. While the constant region selected may vary according to specific use, high affinity antigen-binding and target specificity characteristics reside in the variable region.

A VELOCIMMUNE® mouse was immunized with a growth factor that promotes angiogenesis (Antigen C) and antigen-specific human antibodies were isolated and sequenced for V gene usage using standard techniques recognized in the art. Selected antibodies were cloned onto human heavy and light chain constant regions and 69 heavy chains were selected for pairing with one of three human light chains: (1) the cognate κ light chain linked to a human κ constant region, (2) a rearranged human germline Vκ1-39Jκ5 linked to a human κ constant region, or (3) a rearranged human germline Vκ3-20Jκ1 linked to a human κ constant region. Each heavy chain and light chain pair were co-transfected in CHO-K1 cells using standard techniques. Presence of antibody in the supernatant was detected by anti-human IgG in an ELISA assay. Antibody titer (ng/ml) was determined for each heavy chain/light chain pair and titers with the different rearranged germline light chains were compared to the titers obtained with the parental antibody molecule (*i.e.,* heavy chain paired with cognate light chain) and percent of native titer was calculated (Table 1). V_{H}: Heavy chain variable gene. ND: no expression detected under current experimental conditions.

**Table 1**

| V_{H} | Antibody Titer (ng/mL) | | | | Percent of Native Titer | |
|---|---|---|---|---|---|---|
| | Cognate LC | Vκ1-39Jκ5 | Vκ3-20Jκ1 | | Vκ1-39Jκ5 | Vκ3-20Jκ1 |
| 3-15 | 63 | 23 | 11 | | 36.2 | 17.5 |
| 1-2 | 103 | 53 | ND | | 51.1 | - |
| 3-23 | 83 | 60 | 23 | | 72.0 | 27.5 |
| 3-33 | 15 | 77 | ND | | 499.4 | - |
| 4-31 | 22 | 69 | 17 | | 309.4 | 76.7 |
| 3-7 | 53 | 35 | 28 | | 65.2 | 53.1 |
| - | 22 | 32 | 19 | | 148.8 | 89.3 |
| 1-24 | 3 | 13 | ND | | 455.2 | - |
| 3-33 | 1 | 47 | ND | | 5266.7 | - |
| 3-33 | 58 | 37 | ND | | 63.1 | - |
| - | 110 | 67 | 18 | | 60.6 | 16.5 |
| 3-23 | 127 | 123 | 21 | | 96.5 | 16.3 |
| 3-33 | 28 | 16 | 2 | | 57.7 | 7.1 |
| 3-23 | 32 | 50 | 38 | | 157.1 | 119.4 |
| - | 18 | 45 | 18 | | 254.3 | 101.7 |
| 3-9 | 1 | 30 | 23 | | 2508.3 | 1900.0 |
| 3-11 | 12 | 26 | 6 | | 225.9 | 48.3 |
| 1-8 | 16 | ND | 13 | | - | 81.8 |
| 3-33 | 54 | 81 | 10 | | 150.7 | 19.1 |
| - | 34 | 9 | ND | | 25.9 | - |
| 3-20 | 7 | 14 | 54 | | 203.0 | 809.0 |
| 3-33 | 19 | 38 | ND | | 200.5 | - |
| 3-11 | 48 | ND | 203 | | - | 423.6 |
| - | 11 | 23 | 8 | | 212.7 | 74.5 |
| 3-33 | 168 | 138 | 182 | | 82.0 | 108.2 |
| 3-20 | 117 | 67 | 100 | | 57.5 | 86.1 |
| 3-23 | 86 | 61 | 132 | | 70.7 | 154.1 |
| 3-33 | 20 | 12 | 33 | | 60.9 | 165.3 |
| 4-31 | 69 | 92 | 52 | | 133.8 | 75.0 |
| 3-23 | 87 | 78 | 62 | | 89.5 | 71.2 |
| 1-2 | 31 | 82 | 51 | | 263.0 | 164.6 |
| 3-23 | 53 | 93 | 151 | | 175.4 | 285.4 |
| - | 11 | 8 | 17 | | 75.7 | 151.4 |
| 3-33 | 114 | 36 | 27 | | 31.6 | 23.4 |
| 3-15 | 73 | 39 | 44 | | 53.7 | 59.6 |
| 3-33 | 1 | 34 | 16 | | 5600.0 | 2683.3 |
| 3-9 | 58 | 112 | 57 | | 192.9 | 97.6 |
| 3-33 | 67 | 20 | 105 | | 30.1 | 157.0 |
| 3-33 | 34 | 21 | 24 | | 62.7 | 70.4 |
| 3-20 | 10 | 49 | 91 | | 478.4 | 888.2 |
| 3-33 | 66 | 32 | 25 | | 48.6 | 38.2 |
| 3-23 | 17 | 59 | 56 | | 342.7 | 329.8 |
| - | 58 | 108 | 19 | | 184.4 | 32.9 |
| - | 68 | 54 | 20 | | 79.4 | 29.9 |
| 3-33 | 42 | 35 | 32 | | 83.3 | 75.4 |
| - | 29 | 19 | 13 | | 67.1 | 43.9 |
| 3-9 | 24 | 34 | 29 | | 137.3 | 118.4 |
| 3-30/33 | 17 | 33 | 7 | | 195.2 | 43.1 |
| 3-7 | 25 | 70 | 74 | | 284.6 | 301.6 |
| 3-33 | 87 | 127 | ND | | 145.1 | - |
| 6-1 | 28 | 56 | ND | | 201.8 | - |
| 3-33 | 56 | 39 | 20 | | 69.9 | 36.1 |
| 3-33 | 10 | 53 | 1 | | 520.6 | 6.9 |
| 3-33 | 20 | 67 | 10 | | 337.2 | 52.3 |
| 3-33 | 11 | 36 | 18 | | 316.8 | 158.4 |
| 3-23 | 12 | 42 | 32 | | 356.8 | 272.9 |
| 3-33 | 66 | 95 | 15 | | 143.6 | 22.5 |
| 3-15 | 55 | 68 | ND | | 123.1 | - |
| - | 32 | 68 | 3 | | 210.9 | 10.6 |
| 1-8 | 28 | 48 | ND | | 170.9 | - |
| 3-33 | 124 | 192 | 21 | | 154.3 | 17.0 |
| 3-33 | 0 | 113 | ND | | 56550.0 | - |
| 3-33 | 10 | 157 | 1 | | 1505.8 | 12.5 |
| 3-33 | 6 | 86 | 15 | | 1385.5 | 243.5 |
| 3-23 | 70 | 115 | 22 | | 163.5 | 31.0 |
| 3-7 | 71 | 117 | 21 | | 164.6 | 29.6 |
| 3-33 | 82 | 100 | 47 | | 122.7 | 57.1 |
| 3-7 | 124 | 161 | 41 | | 130.0 | 33.5 |

In a similar experiment, VELOCIMMUNE® mice were immunized with several different antigens and selected heavy chains of antigen specific human antibodies were tested for their ability to pair with different rearranged human germline light chains (as described above). The antigens used in this experiment included an enzyme involved in cholesterol homeostasis (Antigen A), a serum hormone involved in regulating glucose homeostasis (Antigen B), a growth factor that promotes angiogenesis (Antigen C) and a cell-surface receptor (Antigen D). Antigen specific antibodies were isolated from mice of each immunization group and the heavy chain and light chain variable regions were cloned and sequenced. From the sequence of the heavy and light chains, V gene usage was determined and selected heavy chains were paired with either their cognate light chain or a rearranged human germline Vκ1-39Jκ5 region. Each heavy/light chain pair was co-transfected in CHO-K1 cells and the presence of antibody in the supernatant was detected by anti-human IgG in an ELISA assay. Antibody titer (µg/ml) was determined for each heavy chain/light chain pairing and titers with the different rearranged human germline light chains were compared to the titers obtained with the parental antibody molecule (*i.e.,* heavy chain paired with cognate light chain) and percent of native titer was calculated (Table 2). V_{H}: Heavy chain variable gene. Vκ: κ light chain variable gene. ND: no expression detected under current experimental conditions.

**Table 2**

| Antigen | Antibody | V_{H} | Vκ | Titer (µg/ml) | | | Percent of Native Titer |
|---|---|---|---|---|---|---|---|
| | | | | V_{H} Alone | V_{H} + Vκ | V_{H} + Vκ1-39Jκ5 | |
| A | 320 | 1-18 | 2-30 | 0.3 | 3.1 | 2.0 | 66 |
| | 321 | 2-5 | 2-28 | 0.4 | 0.4 | 1.9 | 448 |
| | 334 | 2-5 | 2-28 | 0.4 | 2.7 | 2.0 | 73 |
| | 313 | 3-13 | 3-15 | 0.5 | 0.7 | 4.5 | 670 |
| | 316 | 3-23 | 4-1 | 0.3 | 0.2 | 4.1 | 2174 |
| | 315 | 3-30 | 4-1 | 0.3 | 0.2 | 3.2 | 1327 |
| | 318 | 4-59 | 1-17 | 0.3 | 4.6 | 4.0 | 86 |
| | 257 | 3-13 | 1-5 | 0.4 | 3.1 | 3.2 | 104 |
| | 283 | 3-13 | 1-5 | 0.4 | 5.4 | 3.7 | 69 |
| | 637 | 3-13 | 1-5 | 0.4 | 4.3 | 3.0 | 70 |
| | 638 | 3-13 | 1-5 | 0.4 | 4.1 | 3.3 | 82 |
| B | 624 | 3-23 | 1-17 | 0.3 | 5.0 | 3.9 | 79 |
| | 284 | 3-30 | 1-17 | 0.3 | 4.6 | 3.4 | 75 |
| | 653 | 3-33 | 1-17 | 0.3 | 4.3 | 0.3 | 7 |
| | 268 | 4-34 | 1-27 | 0.3 | 5.5 | 3.8 | 69 |
| | 633 | 4-34 | 1-27 | 0.6 | 6.9 | 3.0 | 44 |
| | 730 | 3-7 | 1-5 | 0.3 | 1.1 | 2.8 | 249 |
| | 728 | 3-7 | 1-5 | 0.3 | 2.0 | 3.2 | 157 |
| | 691 | 3-9 | 3-20 | 0.3 | 2.8 | 3.1 | 109 |
| | 749 | 3-33 | 3-15 | 0.3 | 3.8 | 2.3 | 62 |
| | 750 | 3-33 | 1-16 | 0.3 | 3.0 | 2.8 | 92 |
| | 724 | 3-33 | 1-17 | 0.3 | 2.3 | 3.4 | 151 |
| | 706 | 3-33 | 1-16 | 0.3 | 3.6 | 3.0 | 84 |
| C | 744 | 1-18 | 1-12 | 0.4 | 5.1 | 3.0 | 59 |
| | 696 | 3-11 | 1-16 | 0.4 | 3.0 | 2.9 | 97 |
| | 685 | 3-13 | 3-20 | 0.3 | 0.5 | 3.4 | 734 |
| | 732 | 3-15 | 1-17 | 0.3 | 4.5 | 3.2 | 72 |
| | 694 | 3-15 | 1-5 | 0.4 | 5.2 | 2.9 | 55 |
| | 743 | 3-23 | 1-12 | 0.3 | 3.2 | 0.3 | 10 |
| | 742 | 3-23 | 2-28 | 0.4 | 4.2 | 3.1 | 74 |
| | 693 | 3-23 | 1-12 | 0.5 | 4.2 | 4.0 | 94 |
| | 136 | 3-23 | 2-28 | 0.4 | 5.0 | 2.7 | 55 |
| | 155 | 3-30 | 1-16 | 0.4 | 1.0 | 2.2 | 221 |
| | 163 | 3-30 | 1-16 | 0.3 | 0.6 | 3.0 | 506 |
| | 171 | 3-30 | 1-16 | 0.3 | 1.0 | 2.8 | 295 |
| | 145 | 3-43 | 1-5 | 0.4 | 4.4 | 2.9 | 65 |
| D | 49 | 3-48 | 3-11 | 0.3 | 1.7 | 2.6 | 155 |
| | 51 | 3-48 | 1-39 | 0.1 | 1.9 | 0.1 | 4 |
| | 159 | 3-7 | 6-21 | 0.4 | 3.9 | 3.6 | 92 |
| | 169 | 3-7 | 6-21 | 0.3 | 1.3 | 3.1 | 235 |
| | 134 | 3-9 | 1-5 | 0.4 | 5.0 | 2.9 | 58 |
| | 141 | 4-31 | 1-33 | 2.4 | 4.2 | 2.6 | 63 |
| | 142 | 4-31 | 1-33 | 0.4 | 4.2 | 2.8 | 67 |

The results obtained from these experiments demonstrate that somatically mutated, high affinity heavy chains from different gene families are able to pair with rearranged human germline Vκ1-39Jκ5 and Vκ3-20Jκ1 regions and be secreted from the cell as a normal antibody molecule. As shown in Table 1, antibody titer was increased for about 61% (42 of 69) heavy chains when paired with the rearranged human Vκ1-39Jκ5 light chain and about 29% (20 of 69) heavy chains when paired with the rearranged human Vκ3-20Jκ1 light chain as compared to the cognate light chain of the parental antibody. For about 20% (14 of 69) of the heavy chains, both rearranged human germline light chains conferred an increase in expression as compared to the cognate light chain of the parental antibody. As shown in Table 2, the rearranged human germline Vκ1-39Jκ5 region conferred an increase in expression of several heavy chains specific for a range of different classes of antigens as compared to the cognate light chain for the parental antibodies. Antibody titer was increased by more than two-fold for about 35% (15/43) of the heavy chains as compared to the cognate light chain of the parental antibodies. For two heavy chains (315 and 316), the increase was greater than ten-fold as compared to the parental antibody. Within all the heavy chains that showed increase expression relative to the cognate light chain of the parental antibody, family three (V_{H}3) heavy chains are over represented in comparison to other heavy chain variable region gene families. This demonstrates a favorable relationship of human V_{H}3 heavy chains to pair with rearranged human germline Vκ1-39Jκ5 and Vκ3-20Jκ1 light chains.

### Example 2

### Generation of a Rearranged Human Germline Light Chain Locus

Various rearranged human germline light chain targeting vectors were made using VELOCIGENE® technology (see, *e.g.,* US Pat. No. 6,586,251 and Valenzuela et al. (2003) High-throughput engineering of the mouse genome coupled with high-resolution expression analysis, Nature Biotech. 21(6):652-659) to modify mouse genomic Bacterial Artificial Chromosome (BAC) clones 302g12 and 254m04 (Invitrogen). Using these two BAC clones, genomic constructs were engineered to contain a single rearranged human germline light chain region and inserted into an endogenous κ light chain locus that was previously modified to delete the endogenous κ variable and joining gene segments.

**Construction of Rearranged Human Germline Light Chain Targeting Vectors.** Three different rearranged human germline light chain regions were made using standard molecular biology techniques recognized in the art. The human variable gene segments used for constructing these three regions included rearranged human Vκ1-39Jκ5 sequence, a rearranged human Vκ3-20Jκ1 sequence and a rearranged human VpreBJ 5 sequence.

A DNA segment containing exon 1 (encoding the leader peptide) and intron 1 of the mouse Vκ3-7 gene was made by de novo DNA synthesis (Integrated DNA Technologies). Part of the 5' untranslated region up to a naturally occurring BlpI restriction enzyme site was included. Exons of human Vκ1-39 and Vκ3-20 genes were PCR amplified from human genomic BAC libraries. The forward primers had a 5' extension containing the splice acceptor site of intron 1 of the mouse Vκ3-7 gene. The reverse primer used for PCR of the human Vκ1-39 sequence included an extension encoding human Jκ5, whereas the reverse primer used for PCR of the human Vκ3-20 sequence included an extension encoding human Jκ1. The human VpreBJλ5 sequence was made by de novo DNA synthesis (Integrated DNA Technologies). A portion of the human Jκ-Cκ intron including the splice donor site was PCR amplified from plasmid pBS-296-HA18-PISceI. The forward PCR primer included an extension encoding part of either a human Jκ5, Jκ1, or Jλ5 sequence. The reverse primer included a PI-SceI site, which was previously engineered into the intron.

The mouse Vκ3-7 exon1/intron 1, human variable light chain exons, and human Jκ-Cκ intron fragments were sewn together by overlap extension PCR, digested with BlpI and PI-SceI, and ligated into plasmid pBS-296-HA18-PISceI, which contained the promoter from the human Vκ3-15 variable gene segment. A loxed hygromycin cassette within plasmid pBS-296-HA18-PISceI was replaced with a FRTed hygromycin cassette flanked by NotI and AscI sites. The NotI/PI-SceI fragment of this plasmid was ligated into modified mouse BAC 254m04, which contained part of the mouse Jκ-Cκ intron, the mouse Cκ exon, and about 75 kb of genomic sequence downstream of the mouse κ locus, which provided a 3' homology arm for homologous recombination in mouse ES cells. The NotI/AscI fragment of this BAC was then ligated into modified mouse BAC 302g12, which contained a FRTed neomycin cassette and about 23 kb of genomic sequence upstream of the endogenous κ locus for homologous recombination in mouse ES cells.

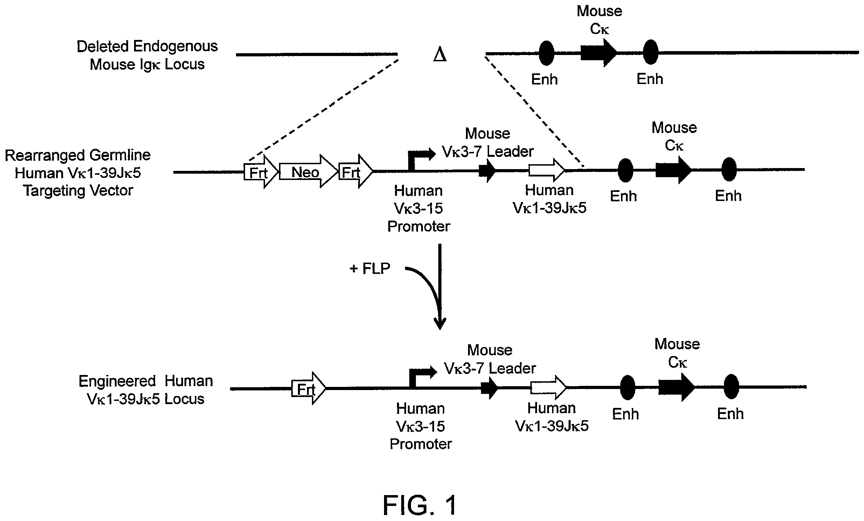

**Rearranged Human Germline Vκ1-39Jκ5 Targeting Vector (****FIG. 1****).** Restriction enzyme sites were introduced at the 5' and 3' ends of an engineered light chain insert for cloning into a targeting vector: an AscI site at the 5' end and a PI-SceI site at the 3' end. Within the 5' AscI site and the 3' PI-SceI site the targeting construct from 5' to 3' included a 5' homology arm containing sequence 5' to the endogenous mouse κ light chain locus obtained from mouse BAC clone 302g12, a FRTed neomycin resistance gene, an genomic sequence including the human Vκ3-15 promoter, a leader sequence of the mouse Vκ3-7 variable gene segment, a intron sequence of the mouse Vκ3-7 variable gene segment, an open reading frame of a rearranged human germline Vκ1-39Jκ5 region, a genomic sequence containing a portion of the human Jκ-Cκ intron, and a 3' homology arm containing sequence 3' of the endogenous mouse Jκ5 gene segment obtained from mouse BAC clone 254m04 (Figure 1, middle). Genes and/or sequences upstream of the endogenous mouse κ light chain locus and downstream of the most 3' Jκ gene segment (*e.g.,* the endogenous 3' enhancer) were unmodified by the targeting construct (see Figure 1). The sequence of the engineered human Vκ1-39Jκ5 locus is shown in SEQ ID NO:1.

Targeted insertion of the rearranged human germline Vκ1-39Jκ5 region into BAC DNA was confirmed by polymerase chain reaction (PCR) using primers located at sequences within the rearranged human germline light chain region. Briefly, the intron sequence 3' to the mouse Vκ3-7 leader sequence was confirmed with primers ULC-m1F (AGGTGAGGGT ACAGATAAGT GTTATGAG; SEQ ID NO:2) and ULC-m1R (TGACAAATGC CCTAATTATA GTGATCA; SEQ ID NO:3). The open reading frame of the rearranged human germline Vκ1-39Jκ5 region was confirmed with primers 1633-h2F (GGGCAAGTCA GAGCATTAGC A; SEQ ID NO:4) and 1633-h2R (TGCAAACTGG ATGCAGCATA G; SEQ ID NO:5). The neomycin cassette was confirmed with primers neoF (GGTGGAGAGG CTATTCGGC; SEQ ID NO:6) and neoR (GAACACGGCG GCATCAG; SEQ ID NO:7). Targeted BAC DNA was then used to electroporate mouse ES cells to created modified ES cells for generating chimeric mice that express a rearranged human germline Vκ1-39Jκ5 region.

Positive ES cell clones were confirmed by TAQMAN™ screening and karyotyping using probes specific for the engineered Vκ1-39Jκ5 light chain region inserted into the endogenous locus. Briefly, probe neoP (TGGGCACAAC AGACAATCGG CTG; SEQ ID NO:8) which binds within the neomycin marker gene, probe ULC-m1P (CCATTATGAT GCTCCATGCC TCTCTGTTC; SEQ ID NO:9) which binds within the intron sequence 3' to the mouse Vκ3-7 leader sequence, and probe 1633h2P (ATCAGCAGAA ACCAGGGAAA GCCCCT; SEQ ID NO:10) which binds within the rearranged human germline Vκ1-39Jκ5 open reading frame. Positive ES cell clones were then used to implant female mice to give rise to a litter of pups expressing the germline Vκ1-39Jκ5 light chain region.

Alternatively, ES cells bearing the rearranged human germline Vκ1-39Jκ5 light chain region are transfected with a construct that expresses FLP in order to remove the FRTed neomycin cassette introduced by the targeting construct. Optionally, the neomycin cassette is removed by breeding to mice that express FLP recombinase (*e.g*., US 6,774,279). Optionally, the neomycin cassette is retained in the mice.

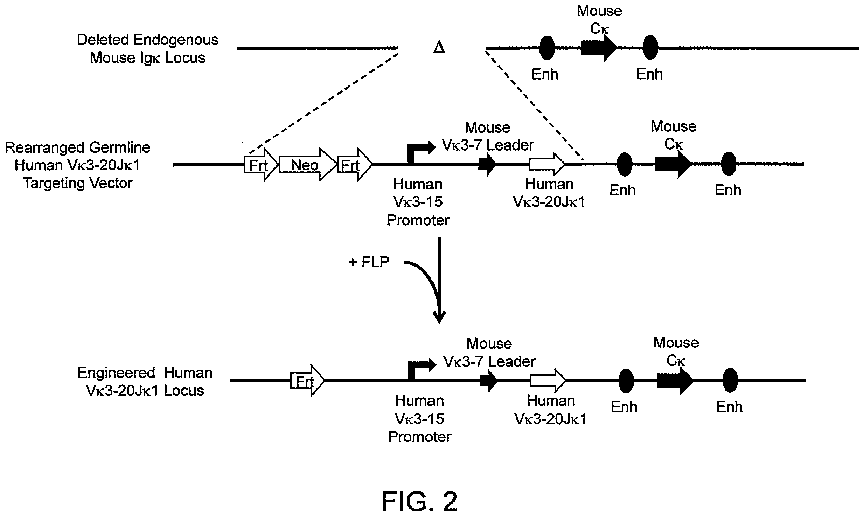

**Rearranged Human Germline Vκ3-20Jκ1 Targeting Vector (****FIG. 2****).** In a similar fashion, an engineered light chain locus expressing a rearranged human germline Vκ3-20Jκ1 region was made using a targeting construct including, from 5' to 3', a 5' homology arm containing sequence 5' to the endogenous mouse κ light chain locus obtained from mouse BAC clone 302g12, a FRTed neomycin resistance gene, a genomic sequence including the human Vκ3-15 promoter, a leader sequence of the mouse Vκ3-7 variable gene segment, an intron sequence of the mouse Vκ3-7 variable gene segment, an open reading frame of a rearranged human germline Vκ3-20Jκ1 region, a genomic sequence containing a portion of the human Jκ-Cκ intron, and a 3' homology arm containing sequence 3' of the endogenous mouse Jκ5 gene segment obtained from mouse BAC clone 254m04 (Figure 2, middle). The sequence of the engineered human Vκ3-20JK1 locus is shown in SEQ ID NO:11.

Targeted insertion of the rearranged human germline Vκ3-20Jκ1 region into BAC DNA was confirmed by polymerase chain reaction (PCR) using primers located at sequences within the rearranged human germline Vκ3-20Jκ1 light chain region. Briefly, the intron sequence 3' to the mouse Vκ3-7 leader sequence was confirmed with primers ULC-m1F (SEQ ID NO:2) and ULC-m1R (SEQ ID NO:3). The open reading frame of the rearranged human germline Vκ3-20Jκ1 region was confirmed with primers 1635-h2F (TCCAGGCACC CTGTCTTTG; SEQ ID NO:12) and 1635-h2R (AAGTAGCTGC TGCTAACACT CTGACT; SEQ ID NO:13). The neomycin cassette was confirmed with primers neoF (SEQ ID NO:6) and neoR (SEQ ID NO:7). Targeted BAC DNA was then used to electroporate mouse ES cells to created modified ES cells for generating chimeric mice that express the rearranged human germline Vκ3-20Jκ1 light chain.

Positive ES cell clones were confirmed by TAQMAN™ screening and karyotyping using probes specific for the engineered Vκ3-20Jκ1 light chain region inserted into the endogenous κ light chain locus. Briefly, probe neoP (SEQ ID NO:8) which binds within the neomycin marker gene, probe ULC-m1P (SEQ ID NO:9) which binds within the mouse Vκ3-7 leader sequence, and probe 1635h2P (AAAGAGCCAC CCTCTCCTGC AGGG; SEQ ID NO:14) which binds within the human Vκ3-20Jκ1 open reading frame. Positive ES cell clones were then used to implant female mice. A litter of pups expressing the human germline Vκ3-20Jκ1 light chain region.

Alternatively, ES cells bearing human germline Vκ3-20Jκ1 light chain region can be transfected with a construct that expresses FLP in order to remove the FRTed neomycin cassette introduced by the targeting construct. Optionally, the neomycin cassette may be removed by breeding to mice that express FLP recombinase (e.g., US 6,774,279). Optionally, the neomycin cassette is retained in the mice.

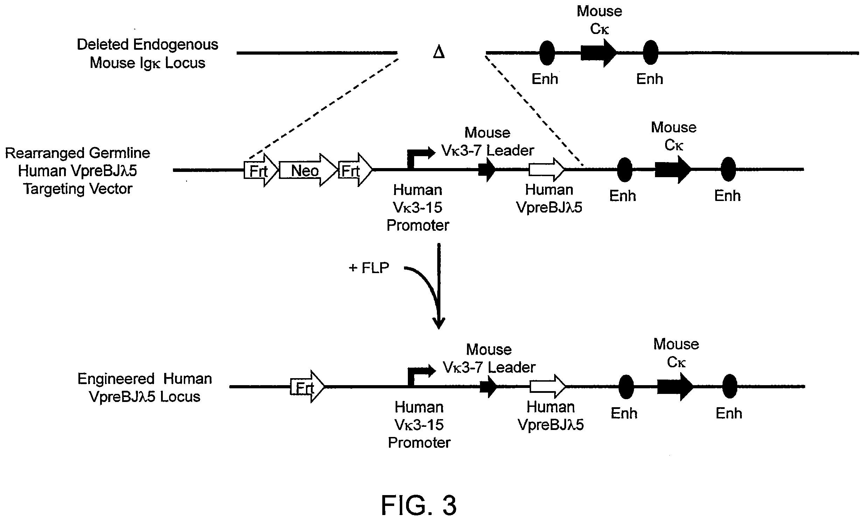

**Rearranged Human Germline VpreBJλ5 Targeting Vector (****FIG. 3****).** In a similar fashion, an engineered light chain locus expressing a rearranged human germline VpreBJ 5 region was made using a targeting construct including, from 5' to 3', a 5' homology arm containing sequence 5' to the endogenous mouse κ light chain locus obtained from mouse BAC clone 302g12, a FRTed neomycin resistance gene, an genomic sequence including the human Vκ3-15 promoter, a leader sequence of the mouse Vκ3-7 variable gene segment, an intron sequence of the mouse Vκ3-7 variable gene segment, an open reading frame of a rearranged human germline VpreBJλ5 region, a genomic sequence containing a portion of the human Jκ-Cκ intron, and a 3' homology arm containing sequence 3' of the endogenous mouse Jκ5 gene segment obtained from mouse BAC clone 254m04 (Figure 3, middle). The sequence of the engineered human VpreBJ 5 locus is shown in SEQ ID NO:15.

Targeted insertion of the rearranged human germline VpreBJ 5 region into BAC DNA was confirmed by polymerase chain reaction (PCR) using primers located at sequences within the rearranged human germline VpreBJλ5 region light chain region. Briefly, the intron sequence 3' to the mouse Vκ3-7 leader sequence was confirmed with primers ULC-m1F (SEQ ID NO:2) and ULC-m1R (SEQ ID NO:3). The open reading frame of the rearranged human germline VpreBJλ5 region was confirmed with primers 1616-h1F (TGTCCTCGGC CCTTGGA; SEQ ID NO:16) and 1616-h1R (CCGATGTCAT GGTCGTTCCT; SEQ ID NO:17). The neomycin cassette was confirmed with primers neoF (SEQ ID NO:6) and neoR (SEQ ID NO:7). Targeted BAC DNA was then used to electroporate mouse ES cells to created modified ES cells for generating chimeric mice that express the rearranged human germline VpreBJλ5 light chain.

Positive ES cell clones are confirmed by TAQMAN™ screening and karyotyping using probes specific for the engineered VpreBJλ5 light chain region inserted into the endogenous κ light chain locus. Briefly, probe neoP (SEQ ID NO:8) which binds within the neomycin marker gene, probe ULC-m1P (SEQ ID NO:9) which binds within the mouse IgVκ3-7 leader sequence, and probe 1616h1P (ACAATCCGCC TCACCTGCAC CCT; SEQ ID NO:18) which binds within the human VpreBJλ5 open reading frame. Positive ES cell clones are then used to implant female mice to give rise to a litter of pups expressing a germline light chain region.

Alternatively, ES cells bearing the rearranged human germline VpreBJ 5 light chain region are transfected with a construct that expresses FLP in order to remove the FRTed neomycin cassette introduced by the targeting construct. Optionally, the neomycin cassette is removed by breeding to mice that express FLP recombinase (*e.g*., US 6,774,279). Optionally, the neomycin cassette is retained in the mice.

### Example 3

### Generation of Mice expressing a single rearranged human light chain

Targeted ES cells described above were used as donor ES cells and introduced into an 8-cell stage mouse embryo by the VELOCIMOUSE® method (see, *e.g.,* US Pat. No. 7,294,754 and Poueymirou et al. (2007) F0 generation mice that are essentially fully derived from the donor gene-targeted ES cells allowing immediate phenotypic analyses Nature Biotech. 25(1):91-99. VELOCIMICE® independently bearing an engineered human germline Vκ1-39Jκ5 light chain region, a Vκ3-20Jκ1 light chain region or a VpreBJ 5 light chain region are identified by genotyping using a modification of allele assay (Valenzuela *et al.*, *supra*) that detects the presence of the unique rearranged human germline light chain region.

Pups are genotyped and a pup heterozygous or homozygous for the unique rearranged human germline light chain region are selected for characterizing expression of the rearranged human germline light chain region.

**Flow Cytometry.** Expression of the rearranged human light chain region in the normal antibody repertoire of common light chain mice was validated by analysis of immunoglobulin κ and λ expression in splenocytes and peripheral blood of common light chain mice. Cell suspensions from harvested spleens and peripheral blood of wild type (n=5), Vκ1-39Jκ5 common light chain heterozygote (n=3), Vκ1-39Jκ5 common light chain homozygote (n=3), Vκ3-20Jκ1 common light chain heterozygote (n=2), and Vκ3-20Jκ1 common light chain homozygote (n=2) mice were made using standard methods and stained with CD19⁺, Igλ⁺ and Igκ⁺ using fluorescently labeled antibodies (BD Pharmigen).

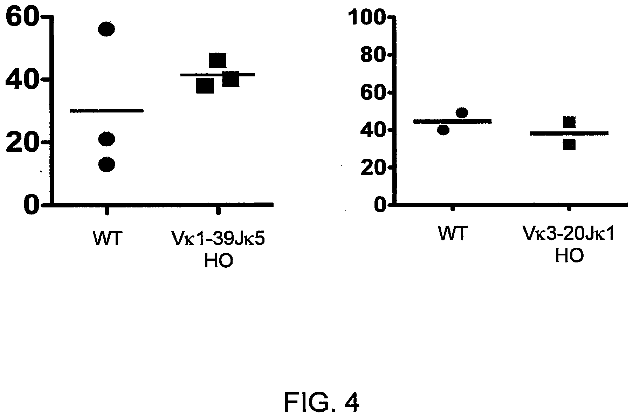

Briefly, 1x10⁶ cells were incubated with anti-mouse CD16/CD32 (clone 2.4G2, BD Pharmigen) on ice for 10 minutes, followed by labeling with the following antibody cocktail for 30 minutes on ice: APC conjugated anti-mouse CD19 (clone 1D3, BD Pharmigen), PerCP-Cy5.5 conjugated anti-mouse CD3 (clone 17A2, BioLegend), FITC conjugated anti-mouse IgK (clone 187.1, BD Pharmigen), PE conjugated anti-mouse Igλ (clone RML-42, BioLegend). Following staining, cells were washed and fixed in 2% formaldehyde. Data acquisition was performed on an LSRII flow cytometer and analyzed with FlowJo. Gating: total B cells (CD19⁺CD3⁻), Igκ⁺ B cells (Igκ⁺Igλ⁻CD19⁺CD3⁻), Igλ⁺ B cells (Igκ⁻Igλ⁺CD19⁺CD3⁻). Data gathered from blood and splenocyte samples demonstrated similar results. Table 3 sets forth the percent positive CD19⁺ B cells from peripheral blood of one representative mouse from each group that are Igλ⁺, Igκ⁺, or Igλ⁺Igκ⁺. Percent of CD19⁺ B cells in peripheral blood from wild type (WT) and mice homozygous for either the Vκ1-39Jκ5 or Vκ3-20Jκ1 common light chain are shown in FIG. 4.

**Table 3**

| Mouse | CD19⁺ B cells | | |
|---|---|---|---|
| | Igλ⁺ | Igκ⁺ | Igλ⁺Igκ⁺ |
| Wild type | 4.8 | 93 | 0.53 |
| Vκ1-39Jκ5 | 1.4 | 93 | 2.6 |
| Vκ3-20Jκ1 | 4.2 | 88 | 6 |

**Common Light Chain Expression.** Expression of each common light chain (Vκ1-39Jκ5 and Vκ3-20Jκ1) was analyzed in heterozygous and homozygous mice using a quantitative PCR assay (*e.g*. TAQMAN™).

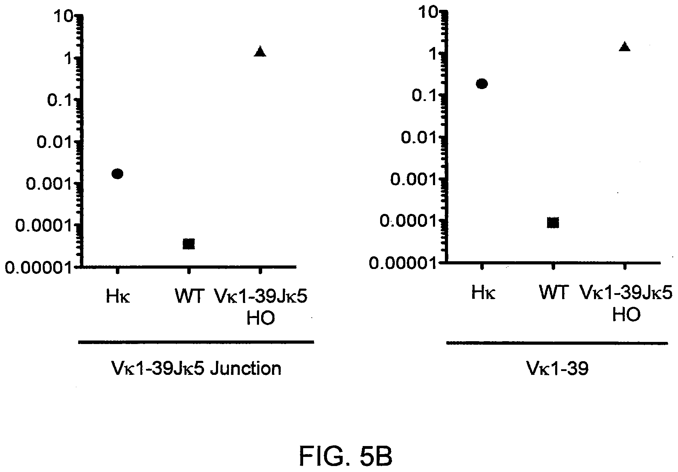

Briefly, CD19⁺ B cells were purified from the spleens of wild type, mice homozygous for a replacement of the mouse heavy chain and κ light chain variable region loci with corresponding human heavy chain and κ light chain variable region loci (Hκ), as well as mice homozygous and heterozygous for each rearranged human light chain region (Vκ1-39Jκ5 or Vκ3-20Jκ1) using mouse CD19 Microbeads (Miltenyi Biotec) according to manufacturer's specifications. Total RNA was purified from CD19⁺ B cells using RNeasy Mini kit (Qiagen) according to manufacturer's specifications and genomic RNA was removed using a RNase-free DNase on-column treatment (Qiagen). 200 ng mRNA was reverse-transcribed into cDNA using the First Stand cDNA Synthesis kit (Invitrogen) and the resulting cDNA was amplified with the Taqman Universal PCR Master Mix (Applied Biosystems). All reactions were performed using the ABI 7900 Sequence Detection System (Applied Biosystems) using primers and Taqman MGB probes spanning (1) the Vκ-Jκ junction for both common light chains, (2) the Vκ gene alone (*i.e.* Vκ1-39 and Vκ3-20), and (3) the mouse Cκ region. Table 4 sets forth the sequences of the primers and probes employed for this assay. Relative expression was normalized to expression of the mouse Cκ region. Results are shown in FIG. 5A, 5B and 5C.

**Table 4**

| Region | Primer/Probe Description (5'-3') | SEQ ID NOs: |
|---|---|---|
| Vκ1-39Jκ5 Junction | (sense) AGCAGTCTGC AACCTGAAGA TTT | 19 |
| | (anti-sense) GTTTAATCTC CAGTCGTGTC CCTT | 20 |
| | (probe) CCTCCGATCA CCTTC | 21 |
| Vκ1-39 | (sense) AAACCAGGGA AAGCCCCTAA | 22 |
| | (anti-sense) ATGGGACCCC ACTTTGCA | 23 |
| | (probe) CTCCTGATCT ATGCTGCAT | 24 |
| Vκ3-20Jκ1 Junction | (sense) CAGCAGACTG GAGCCTGAAG A | 25 |
| | (anti-sense) TGATTTCCAC CTTGGTCCCT T | 26 |
| | (probe) TAGCTCACCT TGGACGTT | 27 |
| Vκ3-20 | (sense) CTCCTCATCT ATGGTGCATC CA | 28 |
| | (anti-sense) GACCCACTGC CACTGAACCT | 29 |
| | (probe) CCACTGGCAT CCC | 30 |
| Mouse CK | (sense) TGAGCAGCAC CCTCACGTT | 31 |
| | (anti-sense) GTGGCCTCAC AGGTATAGCT GTT | 32 |
| | (probe) ACCAAGGACG AGTATGAA | 33 |

**Antigen Specific Common Light Chain Antibodies.** Common light chain mice bearing either a Vκ1-39Jκ5 or Vκ3-20Jκ1 common light chain at the endogenous mouse κ light chain locus were immunized with β-galactosidase and antibody titer was measured.

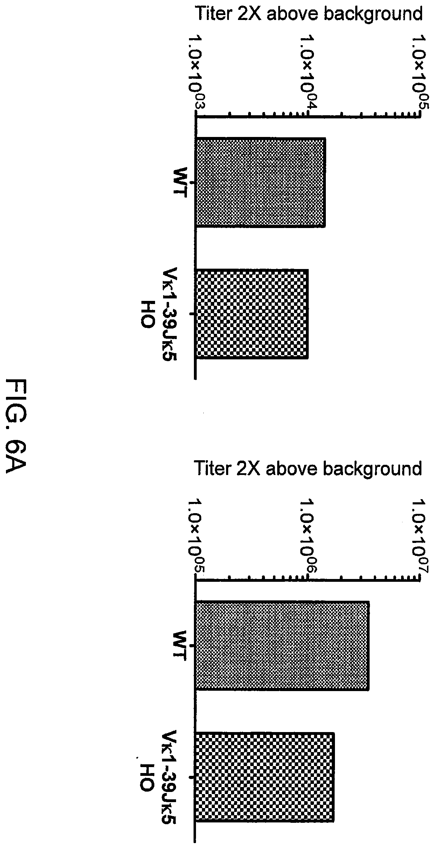

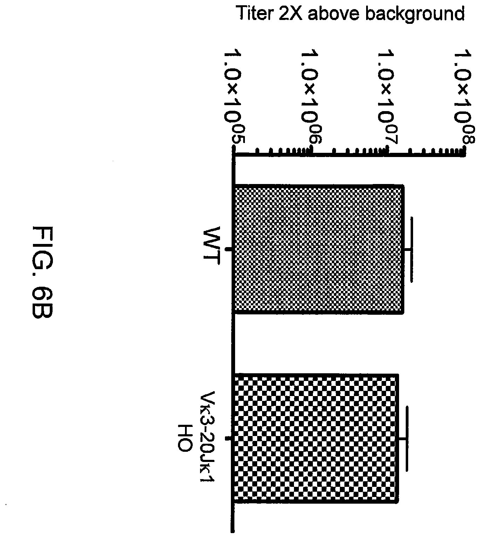

Briefly, β-galactosidase (Sigma) was emulsified in titermax adjuvant (Sigma), as per manufacturers directions. Wild type (n=7), Vκ1-39Jκ5 common light chain homozygotes (n=2) and Vκ3-20Jκ1 common light chain homozygotes (n=5) were immunized by subcutaneous injection with 100 µg β-galactosidase/Titermax. Mice were boosted by subcutaneous injection two times, 3 weeks apart, with 50 µg β-galactosidase/Titermax. After the second boost, blood was collected from anaesthetized mice using a retro-orbital bleed into serum separator tubes (BD Biosciences) as per manufacturer's directions. To measure anti-β-galactosidase IgM or IgG antibodies, ELISA plates (Nunc) were coated with 1 µg/mL β-galactosidase overnight at 4°C. Excess antigen was washed off before blocking with PBS with 1% BSA for one hour at room temperature. Serial dilutions of serum were added to the plates and incubated for one hour at room temperature before washing. Plates were then incubated with HRP conjugated anti-IgM (Southern Biotech) or anti-IgG (Southern Biotech) for one hour at room temperature. Following another wash, plates were developed with TMB substrate (BD Biosciences). Reactions were stopped with IN sulfuric acid and OD₄₅₀ was read using a Victor X5 Plate Reader (Perkin Elmer). Data was analyzed with GraphPad Prism and signal was calculated as the dilution of serum that is two times above background. Results are shown in FIG. 6A and 6B.

As shown in this Example, the ratio of κ/λ B cells in both the splenic and peripheral compartments of Vκ1-39Jκ5 and Vκ3-20Jκ1 common light chain mice demonstrated a near wild type pattern (Table 3 and FIG. 4). VpreBJλ5 common light chain mice, however, demonstrated fewer peripheral B cells, of which about 1-2% express the engineered human light chain region (data not shown). The expression levels of the Vκ1-39Jκ5 and Vκ3-20Jκ1 rearranged human light chain regions from the endogenous κ light chain locus were elevated in comparison to an endogenous κ light chain locus containing a complete replacement of mouse Vκ and Jκ gene segments with human Vκ and Jκ gene segments (FIG. 5A, 5B and 5C). The expression levels of the VpreBJ 5 rearranged human light chain region demonstrated similar high expression from the endogenous κ light chain locus in both heterozygous and homozygous mice (data not shown). This demonstrates that in direct competition with the mouse λ, κ, or both endogenous light chain loci, a single rearranged human V_{L}/J_{L} sequence can yield better than wild type level expression from the endogenous κ light chain locus and give rise to normal splenic and blood B cell frequency. Further, the presence of an engineered κ light chain locus having either a human Vκ1-39Jκ5 or human VK3-20Jκ1 sequence was well tolerated by the mice and appear to function in wild type fashion by representing a substantial portion of the light chain repertoire in the humoral component of the immune response (FIG 6A and 6B).

### Example 4

### Breeding of Mice Expressing a Single Rearranged Human Germline Light Chain

This Example describes several other genetically modified mouse strains that can be bred to any one of the common light chain mice described herein to create multiple genetically modified mouse strains harboring multiple genetically modified immunoglobulin loci.

**Endogenous Igλ Knockout (KO).** To optimize the usage of the engineered light chain locus, mice bearing one of the rearranged human germline light chain regions are bred to another mouse containing a deletion in the endogenous λ light chain locus. In this manner, the progeny obtained will express, as their only light chain, the rearranged human germline light chain region as described in Example 2. Breeding is performed by standard techniques recognized in the art and, alternatively, by a commercial breeder (*e.g*., The Jackson Laboratory). Mouse strains bearing an engineered light chain locus and a deletion of the endogenous λ light chain locus are screened for presence of the unique light chain region and absence of endogenous mouse λ light chains.

**Humanized Endogenous Heavy Chain Locus.** Mice bearing an engineered human germline light chain locus are bred with mice that contain a replacement of the endogenous mouse heavy chain variable gene locus with the human heavy chain variable gene locus (see US 6,596,541; the VELOCIMMUNE® mouse, Regeneron Pharmaceuticals, Inc.). The VELOCIMMUNE® mouse comprises a genome comprising human heavy chain variable regions operably linked to endogenous mouse constant region loci such that the mouse produces antibodies comprising a human heavy chain variable region and a mouse heavy chain constant region in response to antigenic stimulation. The DNA encoding the variable regions of the heavy chains of the antibodies is isolated and operably linked to DNA encoding the human heavy chain constant regions. The DNA is then expressed in a cell capable of expressing the fully human heavy chain of the antibody.

Mice bearing a replacement of the endogenous mouse V_{H} locus with the human VH locus and a single rearranged human germline V_{L} region at the endogenous κ light chain locus are obtained. Reverse chimeric antibodies containing somatically mutated heavy chains (human V_{H} and mouse C_{H}) with a single human light chain (human V_{L} and mouse C_{L}) are obtained upon immunization with an antigen of interest. V_{H} and V_{L} nucleotide sequences of B cells expressing the antibodies are identified and fully human antibodies are made by fusion the V_{H} and V_{L} nucleotide sequences to human C_{H} and C_{L} nucleotide sequences in a suitable expression system.

### Example 5

### Generation of Antibodies from Mice Expressing Human Heavy Chains and a Rearranged Human Germline Light Chain Region

After breeding mice that contain the engineered human light chain region to various desired strains containing modifications and deletions of other endogenous Ig loci (as described in Example 4), selected mice can be immunized with an antigen of interest.

Generally, a VELOCIMMUNE® mouse containing one of the single rearranged human germline light chain regions is challenged with an antigen, and lymphatic cells (such as B-cells) are recovered from serum of the animals. The lymphatic cells are fused with a myeloma cell line to prepare immortal hybridoma cell lines, and such hybridoma cell lines are screened and selected to identify hybridoma cell lines that produce antibodies containing human heavy chain variables and a rearranged human germline light chains which are specific to the antigen used for immunization. DNA encoding the variable regions of the heavy chains and the light chain are isolated and linked to desirable isotypic constant regions of the heavy chain and light chain. Due to the presence of the endogenous mouse sequences and any additional cis-acting elements present in the endogenous locus, the single light chain of each antibody may be somatically mutated. This adds additional diversity to the antigen-specific repertoire comprising a single light chain and diverse heavy chain sequences. The resulting cloned antibody sequences are subsequently expressed in a cell, such as a CHO cell. Alternatively, DNA encoding the antigen-specific chimeric antibodies or the variable domains of the light and heavy chains are identified directly from antigen-specific lymphocytes.

Initially, high affinity chimeric antibodies are isolated having a human variable region and a mouse constant region. As described above, the antibodies are characterized and selected for desirable characteristics, including affinity, selectivity, epitope, *etc.* The mouse constant regions are replaced with a desired human constant region to generate the fully human antibody containing a somatically mutated human heavy chain and a single light chain derived from a rearranged human germline light chain region of the invention. Suitable human constant regions include, for example wild type or modified IgG1 or IgG4.

Separate cohorts of VELOCIMMUNE® mice containing a replacement of the endogenous mouse heavy chain locus with human V_{H}, D_{H}, and J_{H} gene segments and a replacement of the endogenous mouse κ light chain locus with either the engineered germline Vκ1-39Jκ5 human light chain region or the engineered germline Vκ3-20Jκ1 human light chain region (described above) were immunized with a human cell-surface receptor protein (Antigen E). Antigen E is administered directly onto the hind footpad of mice with six consecutive injections every 3-4 days. Two to three micrograms of Antigen E are mixed with 10 µg of CpG oligonucleotide (Cat # tlrl-modn - ODN1826 oligonucleotide; InVivogen, San Diego, CA) and 25 µg of Adju-Phos (Aluminum phosphate gel adjuvant, Cat# H-71639-250; Brenntag Biosector, Frederikssund, Denmark) prior to injection. A total of six injections are given prior to the final antigen recall, which is given 3-5 days prior to sacrifice. Bleeds after the 4th and 6th injection are collected and the antibody immune response is monitored by a standard antigen-specific immunoassay.

When a desired immune response is achieved splenocytes are harvested and fused with mouse myeloma cells to preserve their viability and form hybridoma cell lines. The hybridoma cell lines are screened and selected to identify cell lines that produce Antigen E-specific common light chain antibodies. Using this technique several anti-Antigen E-specific common light chain antibodies (*i.e*., antibodies possessing human heavy chain variable domains, the same human light chain variable domain, and mouse constant domains) are obtained.

Alternatively, anti-Antigen E common light chain antibodies are isolated directly from antigen-positive B cells without fusion to myeloma cells, as described in U.S. 2007/0280945A1, herein specifically incorporated by reference in its entirety. Using this method, several fully human anti-Antigen E common light chain antibodies (*i.e*., antibodies possessing human heavy chain variable domains, either an engineered human Vκ1-39Jκ5 light chain or an engineered human Vκ3-20Jκ1 light chain region, and human constant domains) were obtained.

The biological properties of the exemplary anti-Antigen E common light chain antibodies generated in accordance with the methods of this Example are described in detail in the sections set forth below.

### Example 6

### Heavy Chain Gene Segment Usage in Antigen-Specific Common Light Chain Antibodies

To analyze the structure of the human anti-Antigen E common light chain antibodies produced, nucleic acids encoding heavy chain antibody variable regions were cloned and sequenced. From the nucleic acid sequences and predicted amino acid sequences of the antibodies, gene usage was identified for the heavy chain variable region (HCVR) of selected common light chain antibodies obtained from immunized VELOCIMMUNE® mice containing either the engineered human Vκ1-39Jκ5 light chain or engineered human Vκ3-20Jκ1 light chain region. Results are shown in Tables 5 and 6, which demonstrate that mice according to the invention generate antigen-specific common light chain antibodies from a variety of human heavy chain gene segments, due to a variety of rearrangements, when employing either a mouse that expresses a light chain from only a human Vκ1-39- or a human Vκ3-20-derived light chain. Human V_{H} gene segments of the 2, 3, 4, and 5 families rearranged with a variety of human D_{H} segments and human J_{H} segments to yield antigen-specific antibodies.

**Table 5**

| Vκ1-39Jκ5 | | | | | | | | |
|---|---|---|---|---|---|---|---|---|
| Common Light Chain Antibodies | | | | | | | | |
| Antibody | HCVR | | | | Antibody | HCVR | | |
| | V_{H} | D_{H} | J_{H} | | | V_{H} | D_{H} | J_{H} |
| 2952 | 2-5 | 6-6 | 1 | | 6030 | 3-30 | 6-6 | 5 |
| 5978 | 2-5 | 6-6 | 1 | | 6032 | 3-30 | 6-6 | 5 |
| 5981 | 2-5 | 3-22 | 1 | | 2985 | 3-30 | 6-13 | 4 |
| 6027 | 3-13 | 6-6 | 5 | | 2997 | 3-30 | 6-13 | 4 |
| 3022 | 3-23 | 3-10 | 4 | | 3011 | 3-30 | 6-13 | 4 |
| 3028 | 3-23 | 3-3 | 4 | | 3047 | 3-30 | 6-13 | 4 |
| 5999 | 3-23 | 6-6 | 4 | | 5982 | 3-30 | 6-13 | 4 |
| 6009 | 3-23 | 2-8 | 4 | | 6002 | 3-30 | 6-13 | 4 |
| 6011 | 3-23 | 7-27 | 4 | | 6003 | 3-30 | 6-13 | 4 |
| 5980 | 3-30 | 1-1 | 4 | | 6012 | 3-30 | 6-13 | 4 |
| 3014 | 3-30 | 1-7 | 4 | | 6013 | 3-30 | 6-13 | 4 |
| 3015 | 3-30 | 1-7 | 4 | | 6014 | 3-30 | 6-13 | 4 |
| 3023 | 3-30 | 1-7 | 4 | | 6015 | 3-30 | 6-13 | 4 |
| 3024 | 3-30 | 1-7 | 4 | | 6016 | 3-30 | 6-13 | 4 |
| 3032 | 3-30 | 1-7 | 4 | | 6017 | 3-30 | 6-13 | 4 |
| 6024 | 3-30 | 1-7 | 4 | | 6020 | 3-30 | 6-13 | 4 |
| 6025 | 3-30 | 1-7 | 4 | | 6034 | 3-30 | 6-13 | 4 |
| 6031 | 3-30 | 1-7 | 4 | | 2948 | 3-30 | 7-27 | 4 |
| 6007 | 3-30 | 3-3 | 4 | | 2987 | 3-30 | 7-27 | 4 |
| 2982 | 3-30 | 3-22 | 5 | | 2996 | 3-30 | 7-27 | 4 |
| 6001 | 3-30 | 3-22 | 5 | | 3005 | 3-30 | 7-27 | 4 |
| 6005 | 3-30 | 3-22 | 5 | | 3012 | 3-30 | 7-27 | 4 |
| 6035 | 3-30 | 5-5 | 2 | | 3020 | 3-30 | 7-27 | 4 |
| 3013 | 3-30 | 5-12 | 4 | | 3021 | 3-30 | 7-27 | 4 |
| 3042 | 3-30 | 5-12 | 4 | | 3025 | 3-30 | 7-27 | 4 |
| 2955 | 3-30 | 6-6 | 1 | | 3030 | 3-30 | 7-27 | 4 |
| 3043 | 3-30 | 6-6 | 3 | | 3036 | 3-30 | 7-27 | 4 |
| 3018 | 3-30 | 6-6 | 4 | | 5997 | 3-30 | 7-27 | 4 |
| 2949 | 3-30 | 6-6 | 5 | | 6033 | 3-30 | 7-27 | 4 |
| 2950 | 3-30 | 6-6 | 5 | | 3004 | 3-30 | 7-27 | 5 |
| 2954 | 3-30 | 6-6 | 5 | | 6028 | 3-30 | 7-27 | 6 |
| 2978 | 3-30 | 6-6 | 5 | | 3010 | 4-59 | 3-16 | 3 |
| 3016 | 3-30 | 6-6 | 5 | | 3019 | 4-59 | 3-16 | 3 |
| 3017 | 3-30 | 6-6 | 5 | | 6018 | 4-59 | 3-16 | 3 |
| 3033 | 3-30 | 6-6 | 5 | | 6026 | 4-59 | 3-16 | 3 |
| 3041 | 3-30 | 6-6 | 5 | | 6029 | 4-59 | 3-16 | 3 |
| 5979 | 3-30 | 6-6 | 5 | | 6036 | 4-59 | 3-16 | 3 |
| 5998 | 3-30 | 6-6 | 5 | | 6037 | 4-59 | 3-16 | 3 |
| 6004 | 3-30 | 6-6 | 5 | | 2964 | 4-59 | 3-22 | 3 |
| 6010 | 3-30 | 6-6 | 5 | | 3027 | 4-59 | 3-16 | 4 |
| 6019 | 3-30 | 6-6 | 5 | | 3046 | 5-51 | 5-5 | 3 |
| 6021 | 3-30 | 6-6 | 5 | | 6000 | 1-69 | 6-13 | 4 |
| 6022 | 3-30 | 6-6 | 5 | | 6006 | 1-69 | 6-6 | 5 |
| 6023 | 3-30 | 6-6 | 5 | | 6008 | 1-69 | 6-13 | 4 |

**Table 6**

| Vκ3-20Jκ1 | | | | | | | | |
|---|---|---|---|---|---|---|---|---|
| Common Light Chain Antibodies | | | | | | | | |
| Antibody | HCVR | | | | Antibody | HCVR | | |
| | V_{H} | D_{H} | J_{H} | | | V_{H} | D_{H} | J_{H} |
| 5989 | 3-30 | 3-3 | 3 | | 5992 | 4-39 | 1-26 | 3 |
| 5994 | 3-33 | 1-7 | 4 | | 2975 | 5-51 | 6-13 | 5 |
| 5985 | 3-33 | 2-15 | 4 | | 2972 | 5-51 | 3-16 | 6 |
| 5987 | 3-33 | 2-15 | 4 | | 5986 | 5-51 | 3-16 | 6 |
| 5995 | 3-33 | 2-15 | 4 | | 5993 | 5-51 | 3-16 | 6 |
| 2968 | 4-39 | 1-26 | 3 | | 5996 | 5-51 | 3-16 | 6 |
| 5988 | 4-39 | 1-26 | 3 | | 5984 | 3-53 | 1-1 | 4 |
| 5990 | 4-39 | 1-26 | 3 | | | | | |

### Example 7

### Determination of Blocking Ability of Antigen-Specific Common Light Chain Antibodies by LUMINEX™ Assay

Ninety-eight human common light chain antibodies raised against Antigen E were tested for their ability to block binding of Antigen E's natural ligand (Ligand Y) to Antigen E in a bead-based assay.

The extracellular domain (ECD) of Antigen E was conjugated to two myc epitope tags and a 6X histidine tag (Antigen E-mmH) and amine-coupled to carboxylated microspheres at a concentration of 20 µg/mL in MES buffer. The mixture was incubated for two hours at room temperature followed by bead deactivation with 1M Tris pH 8.0 followed by washing in PBS with 0.05% (v/v) Tween-20. The beads were then blocked with PBS (Irvine Scientific, Santa Ana, CA) containing 2% (w/v) BSA (Sigma-Aldrich Corp., St. Louis, MO). In a 96-well filter plate, supernatants containing Antigen E-specific common light chain antibodies were diluted 1:15 in buffer. A negative control containing a mock supernatant with the same media components as for the antibody supernatant was prepared. Antigen E-labeled beads were added to the supernatants and incubated overnight at 4°C. Biotinylated-Ligand Y protein was added to a final concentration of 0.06 nM and incubated for two hours at room temperature. Detection of biotinylated-Ligand Y bound to Antigen E-myc-myc-6His labeled beads was determined with R-Phycoerythrin conjugated to Streptavidin (Moss Inc, Pasadena, MD) followed by measurement in a LUMINEX™ flow cytometry-based analyzer. Background Mean Fluorescence Intensity (MFI) of a sample without Ligand Y was subtracted from all samples. Percent blocking was calculated by division of the background-subtracted MFI of each sample by the adjusted negative control value, multiplying by 100 and subtracting the resulting value from 100.

In a similar experiment, the same 98 human common light chain antibodies raised against Antigen E were tested for their ability to block binding of Antigen E to Ligand Y-labeled beads.

Briefly, Ligand Y was amine-coupled to carboxylated microspheres at a concentration of 20 µg/mL diluted in MES buffer. The mixture and incubated two hours at room temperature followed by deactivation of beads with 1M Tris pH 8 then washing in PBS with 0.05% (v/v) Tween-20. The beads were then blocked with PBS (Irvine Scientific, Santa Ana, CA) containing 2% (w/v) BSA (Sigma-Aldrich Corp., St. Louis, MO). In a 96-well filter plate, supernatants containing Antigen E-specific common light chain antibodies were diluted 1:15 in buffer. A negative control containing a mock supernatant with the same media components as for the antibody supernatant was prepared. A biotinylated-Antigen E-mmH was added to a final concentration of 0.42 nM and incubated overnight at 4°C. Ligand Y-labeled beads were then added to the antibody/Antigen E mixture and incubated for two hours at room temperature. Detection of biotinylated-Antigen E-mmH bound to Ligand Y-beads was determined with R-Phycoerythrin conjugated to Streptavidin (Moss Inc, Pasadena, MD) followed by measurement in a LUMINEX™ flow cytometry-based analyzer. Background Mean Fluorescence Intensity (MFI) of a sample without Antigen E was subtracted from all samples. Percent blocking was calculated by division of the background-subtracted MFI of each sample by the adjusted negative control value, multiplying by 100 and subtracting the resulting value from 100.

Tables 7 and 8 show the percent blocking for all 98 anti-Antigen E common light chain antibodies tested in both LUMINEX™ assays. ND: not determined under current experimental conditions.

**Table 7**

| Vκ1-39Jκ5 | | |
|---|---|---|
| Common Light Chain Antibodies | | |
| Antibody | % Blocking of Antigen E-Labeled Beads | % Blocking of Antigen E In Solution |
| 2948 | 81.1 | 47.8 |
| 2948G | 38.6 | ND |
| 2949 | 97.6 | 78.8 |
| 2949G | 97.1 | 73.7 |
| 2950 | 96.2 | 81.9 |
| 2950G | 89.8 | 31.4 |
| 2952 | 96.1 | 74.3 |
| 2952G | 93.5 | 39.9 |
| 2954 | 93.7 | 70.1 |
| 2954G | 91.7 | 30.1 |
| 2955 | 75.8 | 30.0 |
| 2955G | 71.8 | ND |
| 2964 | 92.1 | 31.4 |
| 2964G | 94.6 | 43.0 |
| 2978 | 98.0 | 95.1 |
| 2978G | 13.9 | 94.1 |
| 2982 | 92.8 | 78.5 |
| 2982G | 41.9 | 52.4 |
| 2985 | 39.5 | 31.2 |
| 2985G | 2.0 | 5.0 |
| 2987 | 81.7 | 67.8 |
| 2987G | 26.6 | 29.3 |
| 2996 | 87.3 | 55.3 |
| 2996G | 95.9 | 38.4 |
| 2997 | 93.4 | 70.6 |
| 2997G | 9.7 | 7.5 |
| 3004 | 79.0 | 48.4 |
| 3004G | 60.3 | 40.7 |
| 3005 | 97.4 | 93.5 |
| 3005G | 77.5 | 75.6 |
| 3010 | 98.0 | 82.6 |
| 3010G | 97.9 | 81.0 |
| 3011 | 87.4 | 42.8 |
| 3011G | 83.5 | 41.7 |
| 3012 | 91.0 | 60.8 |
| 3012G | 52.4 | 16.8 |
| 3013 | 80.3 | 65.8 |
| 3013G | 17.5 | 15.4 |
| 3014 | 63.4 | 20.7 |
| 3014G | 74.4 | 28.5 |
| 3015 | 89.1 | 55.7 |
| 3015G | 58.8 | 17.3 |
| 3016 | 97.1 | 81.6 |
| 3016G | 93.1 | 66.4 |
| 3017 | 94.8 | 70.2 |
| 3017G | 87.9 | 40.8 |
| 3018 | 85.4 | 54.0 |
| 3018G | 26.1 | 12.7 |
| 3019 | 99.3 | 92.4 |
| 3019G | 99.3 | 88.1 |
| 3020 | 96.7 | 90.3 |
| 3020G | 85.2 | 41.5 |
| 3021 | 74.5 | 26.1 |
| 3021G | 81.1 | 27.4 |
| 3022 | 65.2 | 17.6 |
| 3022G | 67.2 | 9.1 |
| 3023 | 71.4 | 28.5 |
| 3023G | 73.8 | 29.7 |
| 3024 | 73.9 | 32.6 |
| 3024G | 89.0 | 10.0 |
| 3025 | 70.7 | 15.6 |
| 3025G | 76.7 | 24.3 |
| 3027 | 96.2 | 61.6 |
| 3027G | 98.6 | 75.3 |
| 3028 | 92.4 | 29.0 |
| 3028G | 87.3 | 28.8 |
| 3030 | 6.0 | 10.6 |
| 3030G | 41.3 | 14.2 |
| 3032 | 76.5 | 31.4 |
| 3032G | 17.7 | 11.0 |
| 3033 | 98.2 | 86.1 |
| 3033G | 93.6 | 64.0 |
| 3036 | 74.7 | 32.7 |
| 3036G | 90.1 | 51.2 |
| 3041 | 95.3 | 75.9 |
| 3041G | 92.4 | 51.6 |
| 3042 | 88.1 | 73.3 |
| 3042G | 60.9 | 25.2 |
| 3043 | 90.8 | 65.8 |
| 3043G | 92.8 | 60.3 |

**Table 8**

| Vκ3-20Jκ1 | | |
|---|---|---|
| Common Light Chain Antibodies | | |
| Antibody | % Blocking of Antigen E-Labeled Beads | % Blocking of Antigen E In Solution |
| 2968 | 97.1 | 73.3 |
| 2968G | 67.1 | 14.6 |
| 2969 | 51.7 | 20.3 |
| 2969G | 37.2 | 16.5 |
| 2970 | 92.2 | 34.2 |
| 2970G | 92.7 | 27.2 |
| 2971 | 23.4 | 11.6 |
| 2971G | 18.8 | 18.9 |
| 2972 | 67.1 | 38.8 |
| 2972G | 64.5 | 39.2 |
| 2973 | 77.7 | 27.0 |
| 2973G | 51.1 | 20.7 |
| 2974 | 57.8 | 12.4 |
| 2974G | 69.9 | 17.6 |
| 2975 | 49.4 | 18.2 |
| 2975G | 32.0 | 19.5 |
| 2976 | 1.0 | 1.0 |
| 2976G | 50.4 | 20.4 |

In the first LUMINEX™ experiment described above, 80 common light chain antibodies containing the Vκ1-39Jκ5 engineered light chain were tested for their ability to block Ligand Y binding to Antigen E-labeled beads. Of these 80 common light chain antibodies, 68 demonstrated >50% blocking, while 12 demonstrated <50% blocking (6 at 25-50% blocking and 6 at <25% blocking). For the 18 common light chain antibodies containing the Vκ3-20Jκ1 engineered light chain, 12 demonstrated >50% blocking, while 6 demonstrated <50% blocking (3 at 25-50% blocking and 3 at <25% blocking) of Ligand Y binding to Antigen E-labeled beads.

In the second LUMINEX™ experiment described above, the same 80 common light chain antibodies containing the Vκ1-39Jκ5 engineered light chain were tested for their ability to block binding of Antigen E to Ligand Y-labeled beads. Of these 80 common light chain antibodies, 36 demonstrated >50% blocking, while 44 demonstrated <50% blocking (27 at 25-50% blocking and 17 at <25% blocking). For the 18 common light chain antibodies containing the Vκ3-20Jκ1 engineered light chain, 1 demonstrated >50% blocking, while 17 demonstrated <50% blocking (5 at 25-50% blocking and 12 at <25% blocking) of Antigen E binding to Ligand Y-labeled beads.

The data of Tables 7 and 8 establish that the rearrangements described in Tables 5 and 6 generated anti-Antigen E-specific common light chain antibodies that blocked binding of Ligand Y to its cognate receptor Antigen E with varying degrees of efficacy, which is consistent with the anti-Antigen E common light chain antibodies of Tables 5 and 6 comprising antibodies with overlapping and non-overlapping epitope specificity with respect to Antigen E.

### Example 8

### Determination of Blocking Ability of Antigen-Specific Common Light Chain Antibodies by ELISA

Human common light chain antibodies raised against Antigen E were tested for their ability to block Antigen E binding to a Ligand Y-coated surface in an ELISA assay.

Ligand Y was coated onto 96-well plates at a concentration of 2 µg/mL diluted in PBS and incubated overnight followed by washing four times in PBS with 0.05% Tween-20. The plate was then blocked with PBS (Irvine Scientific, Santa Ana, CA) containing 0.5% (w/v) BSA (Sigma-Aldrich Corp., St. Louis, MO) for one hour at room temperature. In a separate plate, supernatants containing anti-Antigen E common light chain antibodies were diluted 1:10 in buffer. A mock supernatant with the same components of the antibodies was used as a negative control. Antigen E-mmH (described above) was added to a final concentration of 0.150 nM and incubated for one hour at room temperature. The antibody/Antigen E-mmH mixture was then added to the plate containing Ligand Y and incubated for one hour at room temperature. Detection of Antigen E-mmH bound to Ligand Y was determined with Horse-Radish Peroxidase (HRP) conjugated to anti-Penta-His antibody (Qiagen, Valencia, CA) and developed by standard colorimetric response using tetramethylbenzidine (TMB) substrate (BD Biosciences, San Jose, CA) neutralized by sulfuric acid. Absorbance was read at OD450 for 0.1 sec. Background absorbance of a sample without Antigen E was subtracted from all samples. Percent blocking was calculated by division of the background-subtracted MFI of each sample by the adjusted negative control value, multiplying by 100 and subtracting the resulting value from 100.

Tables 9 and 10 show the percent blocking for all 98 anti-Antigen E common light chain antibodies tested in the ELISA assay. ND: not determined under current experimental conditions.

**Table 9**

| Vκ1-39Jκ5 | | | | |
|---|---|---|---|---|
| Common Light Chain Antibodies | | | | |
| Antibody | % Blocking of Antigen E In Solution | | Antibody | % Blocking of Antigen E In Solution |
| 2948 | 21.8 | | 3015 | 23.7 |
| 2948G | 22.9 | | 3015G | 10.2 |
| 2949 | 79.5 | | 3016 | 78.1 |
| 2949G | 71.5 | | 3016G | 37.4 |
| 2950 | 80.4 | | 3017 | 61.6 |
| 2950G | 30.9 | | 3017G | 25.2 |
| 2952 | 66.9 | | 3018 | 40.6 |
| 2952G | 47.3 | | 3018G | 14.5 |
| 2954 | 55.9 | | 3019 | 94.6 |
| 2954G | 44.7 | | 3019G | 92.3 |
| 2955 | 12.1 | | 3020 | 80.8 |
| 2955G | 25.6 | | 3020G | ND |
| 2964 | 34.8 | | 3021 | 7.6 |
| 2964G | 47.7 | | 3021G | 20.7 |
| 2978 | 90.0 | | 3022 | 2.4 |
| 2978G | 90.2 | | 3022G | 15.0 |
| 2982 | 59.0 | | 3023 | 9.1 |
| 2982G | 20.4 | | 3023G | 19.2 |
| 2985 | 10.5 | | 3024 | 7.5 |
| 2985G | ND | | 3024G | 15.2 |
| 2987 | 31.4 | | 3025 | ND |
| 2987G | ND | | 3025G | 13.9 |
| 2996 | 29.3 | | 3027 | 61.4 |
| 2996G | ND | | 3027G | 82.7 |
| 2997 | 48.7 | | 3028 | 40.3 |
| 2997G | ND | | 3028G | 12.3 |
| 3004 | 16.7 | | 3030 | ND |
| 3004G | 3.5 | | 3030G | 9.5 |
| 3005 | 87.2 | | 3032 | ND |
| 3005G | 54.3 | | 3032G | 13.1 |
| 3010 | 74.5 | | 3033 | 77.1 |
| 3010G | 84.6 | | 3033G | 32.9 |
| 3011 | 19.4 | | 3036 | 17.6 |
| 3011G | ND | | 3036G | 24.6 |
| 3012 | 45.0 | | 3041 | 59.3 |
| 3012G | 12.6 | | 3041G | 30.7 |
| 3013 | 39.0 | | 3042 | 39.9 |
| 3013G | 9.6 | | 3042G | 16.1 |
| 3014 | 5.2 | | 3043 | 57.4 |
| 3014G | 17.1 | | 3043G | 46.1 |

**Table 10**

| Vκ3-20Jκ1 | | | | |
|---|---|---|---|---|
| Common Light Chain Antibodies | | | | |
| Antibody | % Blocking of Antigen E In Solution | | Antibody | % Blocking of Antigen E In Solution |
| 2968 | 68.9 | | 2972G | 35.7 |
| 2968G | 15.2 | | 2973 | 20.7 |
| 2969 | 10.1 | | 2973G | 23.1 |
| 2969G | 23.6 | | 2974 | ND |
| 2970 | 34.3 | | 2974G | 22.0 |
| 2970G | 41.3 | | 2975 | 8.7 |
| 2971 | 6.3 | | 2975G | 19.2 |
| 2971G | 27.1 | | 2976 | 4.6 |
| 2972 | 9.6 | | 2976G | 26.7 |

As described in this Example, of the 80 common light chain antibodies containing the Vκ1-39Jκ5 engineered light chain tested for their ability to block Antigen E binding to a Ligand Y-coated surface, 22 demonstrated >50% blocking, while 58 demonstrated <50% blocking (20 at 25-50% blocking and 38 at <25% blocking). For the 18 common light chain antibodies containing the Vκ3-20Jκ1 engineered light chain, one demonstrated >50% blocking, while 17 demonstrated <50% blocking (5 at 25-50% blocking and 12 at <25% blocking) of Antigen E binding to a Ligand Y-coated surface.

These results are also consistent with the Antigen E-specific common light chain antibody pool comprising antibodies with overlapping and non-overlapping epitope specificity with respect to Antigen E.

### Example 9

### BIACORE™ Affinity Determination for Antigen-Specific Common Light Chain Antibodies

Equilibrium dissociation constants (K_{D}) for selected antibody supernatants were determined by SPR (Surface Plasmon Resonance) using a BIACORE™ T100 instrument (GE Healthcare). All data was obtained using HBS-EP (10mM Hepes, 150mM NaCl, 0.3mM EDTA, 0.05% Surfactant P20, pH 7.4) as both the running and sample buffers, at 25°C. Antibodies were captured from crude supernatant samples on a CM5 sensor chip surface previously derivatized with a high density of anti-human Fc antibodies using standard amine coupling chemistry. During the capture step, supernatants were injected across the anti-human Fc surface at a flow rate of 3 µL/min, for a total of 3 minutes. The capture step was followed by an injection of either running buffer or analyte at a concentration of 100 nM for 2 minutes at a flow rate of 35 µL/min. Dissociation of antigen from the captured antibody was monitored for 6 minutes. The captured antibody was removed by a brief injection of 10 mM glycine, pH 1.5. All sensorgrams were double referenced by subtracting sensorgrams from buffer injections from the analyte sensorgrams, thereby removing artifacts caused by dissociation of the antibody from the capture surface. Binding data for each antibody was fit to a 1:1 binding model with mass transport using BIAcore T100 Evaluation software v2.1. Results are shown in Tables 11 and 12.

**Table 11**

| Vκ1-39Jκ5 | | | | | | |
|---|---|---|---|---|---|---|
| Common Light Chain Antibodies | | | | | | |
| Antibody | 100 nM Antigen E | | | Antibody | 100 nM Antigen E | |
| | K_{D} (nM) | T_{1/2} (min) | | | K_{D} (nM) | T_{1/2} (min) |
| 2948 | 8.83 | 28 | | 3015 | 29.1 | 11 |
| 2948G | 95.0 | 1 | | 3015G | 65.9 | 0 |
| 2949 | 3.57 | 18 | | 3016 | 4.99 | 17 |
| 2949G | 6.37 | 9 | | 3016G | 18.9 | 4 |
| 2950 | 4.91 | 17 | | 3017 | 9.83 | 8 |
| 2950G | 13.6 | 5 | | 3017G | 55.4 | 2 |
| 2952 | 6.25 | 7 | | 3018 | 11.3 | 36 |
| 2952G | 7.16 | 4 | | 3018G | 32.5 | 3 |
| 2954 | 2.37 | 24 | | 3019 | 1.54 | 59 |
| 2954G | 5.30 | 9 | | 3019G | 2.29 | 42 |
| 2955 | 14.4 | 6 | | 3020 | 5.41 | 39 |
| 2955G | 12.0 | 4 | | 3020G | 41.9 | 6 |
| 2964 | 14.8 | 6 | | 3021 | 50.1 | 6 |
| 2964G | 13.0 | 9 | | 3021G | 26.8 | 4 |
| 2978 | 1.91 | 49 | | 3022 | 25.7 | 17 |
| 2978G | 1.80 | 58 | | 3022G | 20.8 | 12 |
| 2982 | 6.41 | 19 | | 3023 | 263 | 9 |
| 2982G | 16.3 | 9 | | 3023G | 103 | 5 |
| 2985 | 64.4 | 9 | | 3024 | 58.8 | 7 |
| 2985G | 2.44 | 8 | | 3024G | 7.09 | 10 |
| 2987 | 21.0 | 11 | | 3025 | 35.2 | 6 |
| 2987G | 37.6 | 4 | | 3025G | 42.5 | 8 |
| 2996 | 10.8 | 9 | | 3027 | 7.15 | 6 |
| 2996G | 24.0 | 2 | | 3027G | 4.24 | 18 |
| 2997 | 7.75 | 19 | | 3028 | 6.89 | 37 |
| 2997G | 151 | 1 | | 3028G | 7.23 | 22 |
| 3004 | 46.5 | 14 | | 3030 | 46.2 | 7 |
| 3004G | 1.93 | 91 | | 3030G | 128 | 3 |
| 3005 | 2.35 | 108 | | 3032 | 53.2 | 9 |
| 3005G | 6.96 | 27 | | 3032G | 13.0 | 1 |
| 3010 | 4.13 | 26 | | 3033 | 4.61 | 17 |
| 3010G | 2.10 | 49 | | 3033G | 12.0 | 5 |
| 3011 | 59.1 | 5 | | 3036 | 284 | 12 |
| 3011G | 41.7 | 5 | | 3036G | 18.2 | 10 |
| 3012 | 9.71 | 20 | | 3041 | 6.90 | 12 |
| 3012G | 89.9 | 2 | | 3041G | 22.9 | 2 |
| 3013 | 20.2 | 20 | | 3042 | 9.46 | 34 |
| 3013G | 13.2 | 4 | | 3042G | 85.5 | 3 |
| 3014 | 213 | 4 | | 3043 | 9.26 | 29 |
| 3014G | 36.8 | 3 | | 3043G | 13.1 | 22 |

**Table 12**

| Vκ3-20Jκ1 | | | | | | |
|---|---|---|---|---|---|---|
| Common Light Chain Antibodies | | | | | | |
| Antibody | 100 nM Antigen E | | | Antibody | 100 nM Antigen E | |
| | K_{D} (nM) | T_{1/2} (min) | | | K_{D} (nM) | T_{1/2} (min) |
| 2968 | 5.50 | 8 | | 2973 | 5.35 | 39 |
| 2968G | 305 | 0 | | 2973G | 11.0 | 44 |
| 2969 | 34.9 | 2 | | 2974 | 256 | 0 |
| 2969G | 181 | 1 | | 2974G | 138 | 0 |
| 2970G | 12.3 | 3 | | 2975 | 38.0 | 2 |
| 2971G | 32.8 | 22 | | 2975G | 134 | 1 |
| 2972 | 6.02 | 13 | | 2976 | 6.73 | 10 |
| 2972G | 74.6 | 26 | | 2976G | 656 | 8 |

The binding affinities of common light chain antibodies comprising the rearrangements shown in Tables 5 and 6 vary, with nearly all exhibiting a K_{D} in the nanomolar range. The affinity data is consistent with the common light chain antibodies resulting from the combinatorial association of rearranged variable domains described in Tables 5 and 6 being high-affinity, clonally selected, and somatically mutated. Coupled with data previously shown, the common light chain antibodies described in Tables 5 and 6 comprise a collection of diverse, high-affinity antibodies that exhibit specificity for one or more epitopes on Antigen E.

### Example 10

### Determination of Binding Specificities of Antigen-Specific Common Light Chain Antibodies by LUMINEX™ Assay

Selected anti-Antigen E common light chain antibodies were tested for their ability to bind to the ECD of Antigen E and Antigen E ECD variants, including the cynomolgous monkey ortholog (*Mf* Antigen E), which differs from the human protein in approximately 10% of its amino acid residues; a deletion mutant of Antigen E lacking the last 10 amino acids from the C-terminal end of the ECD (Antigen E-ΔCT); and two mutants containing an alanine substitution at suspected locations of interaction with Ligand Y (Antigen E-Ala1 and AntigenE-Ala2). The Antigen E proteins were produced in CHO cells and each contained a myc-myc-His C-terminal tag.

For the binding studies, Antigen E ECD protein or variant protein (described above) from 1 mL of culture medium was captured by incubation for 2 hr at room temperature with 1 x 10⁶ microsphere (LUMINEX™) beads covalently coated with an anti-myc monoclonal antibody (MAb 9E10, hybridoma cell line CRL-1729™; ATCC, Manassas, VA). The beads were then washed with PBS before use. Supernatants containing anti-Antigen E common light chain antibodies were diluted 1:4 in buffer and added to 96-well filter plates. A mock supernatant with no antibody was used as negative control. The beads containing the captured Antigen E proteins were then added to the antibody samples (3000 beads per well) and incubated overnight at 4°C. The following day, the sample beads were washed and the bound common light chain antibody was detected with a R-phycoerythrin-conjugated anti-human IgG antibody. The fluorescence intensity of the beads (approximately 100 beads counted for each antibody sample binding to each Antigen E protein) was measured with a LUMINEX™ flow cytometry-based analyzer, and the median fluorescence intensity (MFI) for at least 100 counted beads per bead/antibody interaction was recorded. Results are shown in Tables 13 and 14.

**Table 13**

| Vκ1-39Jκ5 Common Light Chain Antibodies | | | | | |
|---|---|---|---|---|---|
| Antibody | Mean Fluorescence Intensity (MFI) | | | | |
| | Antigen E-ECD | Antigen E-ΔCT | Antigen E-Ala1 | Antigen E-Ala2 | *Mf* Antigen E |
| 2948 | 1503 | 2746 | 4953 | 3579 | 1648 |
| 2948G | 537 | 662 | 2581 | 2150 | 863 |
| 2949 | 3706 | 4345 | 8169 | 5678 | 5142 |
| 2949G | 3403 | 3318 | 7918 | 5826 | 5514 |
| 2950 | 3296 | 4292 | 7756 | 5171 | 4749 |
| 2950G | 2521 | 2408 | 7532 | 5079 | 3455 |
| 2952 | 3384 | 1619 | 1269 | 168 | 911 |
| 2952G | 3358 | 1001 | 108 | 55 | 244 |
| 2954 | 2808 | 3815 | 7114 | 5039 | 3396 |
| 2954G | 2643 | 2711 | 7620 | 5406 | 3499 |
| 2955 | 1310 | 2472 | 4738 | 3765 | 1637 |
| 2955G | 1324 | 1802 | 4910 | 3755 | 1623 |
| 2964 | 5108 | 1125 | 4185 | 346 | 44 |
| 2964G | 4999 | 729 | 4646 | 534 | 91 |
| 2978 | 6986 | 2800 | 14542 | 10674 | 8049 |
| 2978G | 5464 | 3295 | 11652 | 8026 | 6452 |
| 2982 | 4955 | 2388 | 13200 | 9490 | 6772 |
| 2982G | 3222 | 2013 | 8672 | 6509 | 4949 |
| 2985 | 1358 | 832 | 4986 | 3892 | 1669 |
| 2985G | 43 | 43 | 128 | 244 | 116 |
| 2987 | 3117 | 1674 | 7646 | 5944 | 2546 |
| 2987G | 3068 | 1537 | 9202 | 6004 | 4744 |
| 2996 | 4666 | 1917 | 12875 | 9046 | 6459 |
| 2996G | 2752 | 1736 | 8742 | 6150 | 4873 |
| 2997 | 5164 | 2159 | 12167 | 8361 | 5922 |
| 2997G | 658 | 356 | 3392 | 2325 | 1020 |
| 3004 | 2794 | 1397 | 8542 | 6268 | 3083 |
| 3004G | 2753 | 1508 | 8267 | 5808 | 4345 |
| 3005 | 5683 | 2221 | 12900 | 9864 | 5868 |
| 3005G | 4344 | 2732 | 10669 | 7125 | 5880 |
| 3010 | 4829 | 1617 | 2642 | 3887 | 44 |
| 3010G | 3685 | 1097 | 2540 | 3022 | 51 |
| 3011 | 2859 | 2015 | 7855 | 5513 | 3863 |
| 3011G | 2005 | 1072 | 6194 | 4041 | 3181 |
| 3012 | 3233 | 2221 | 8543 | 5637 | 3307 |
| 3012G | 968 | 378 | 3115 | 2261 | 1198 |
| 3013 | 2343 | 1791 | 6715 | 4810 | 2528 |
| 3013G | 327 | 144 | 1333 | 1225 | 370 |
| 3014 | 1225 | 1089 | 5436 | 3621 | 1718 |
| 3014G | 1585 | 851 | 5178 | 3705 | 2411 |
| 3015 | 3202 | 2068 | 8262 | 5554 | 3796 |
| 3015G | 1243 | 531 | 4246 | 2643 | 1611 |
| 3016 | 4220 | 2543 | 8920 | 5999 | 5666 |
| 3016G | 2519 | 1277 | 6344 | 4288 | 4091 |
| 3017 | 3545 | 2553 | 8700 | 5547 | 5098 |
| 3017G | 1972 | 1081 | 5763 | 3825 | 3038 |
| 3018 | 2339 | 1971 | 6140 | 4515 | 2293 |
| 3018G | 254 | 118 | 978 | 1020 | 345 |
| 3019 | 5235 | 1882 | 7108 | 4249 | 54 |
| 3019G | 4090 | 1270 | 4769 | 3474 | 214 |
| 3020 | 3883 | 3107 | 8591 | 6602 | 4420 |
| 3020G | 2165 | 1209 | 6489 | 4295 | 2912 |
| 3021 | 1961 | 1472 | 6872 | 4641 | 2742 |
| 3021G | 2091 | 1005 | 6430 | 3988 | 2935 |
| 3022 | 2418 | 793 | 7523 | 2679 | 36 |
| 3022G | 2189 | 831 | 6182 | 3051 | 132 |
| 3023 | 1692 | 1411 | 5788 | 3898 | 2054 |
| 3023G | 1770 | 825 | 5702 | 3677 | 2648 |
| 3024 | 1819 | 1467 | 6179 | 4557 | 2450 |
| 3024G | 100 | 87 | 268 | 433 | 131 |
| 3025 | 1853 | 1233 | 6413 | 4337 | 2581 |
| 3025G | 1782 | 791 | 5773 | 3871 | 2717 |
| 3027 | 4131 | 1018 | 582 | 2510 | 22 |
| 3027G | 3492 | 814 | 1933 | 2596 | 42 |
| 3028 | 4361 | 2545 | 9884 | 5639 | 975 |
| 3028G | 2835 | 1398 | 7124 | 3885 | 597 |
| 3030 | 463 | 277 | 1266 | 1130 | 391 |
| 3030G | 943 | 302 | 3420 | 2570 | 1186 |
| 3032 | 2083 | 1496 | 6594 | 4402 | 2405 |
| 3032G | 295 | 106 | 814 | 902 | 292 |
| 3033 | 4409 | 2774 | 8971 | 6331 | 5825 |
| 3033G | 2499 | 1234 | 6745 | 4174 | 4210 |
| 3036 | 1755 | 1362 | 6137 | 4041 | 1987 |
| 3036G | 2313 | 1073 | 6387 | 4243 | 3173 |
| 3041 | 3674 | 2655 | 8629 | 5837 | 4082 |
| 3041G | 2519 | 1265 | 6468 | 4274 | 3320 |
| 3042 | 2653 | 2137 | 7277 | 5124 | 3325 |
| 3042G | 1117 | 463 | 4205 | 2762 | 1519 |
| 3043 | 3036 | 2128 | 7607 | 5532 | 3366 |
| 3043G | 2293 | 1319 | 6573 | 4403 | 3228 |

**Table 14**

| Vκ3-20Jκ1 Common Light Chain Antibodies | | | | | |
|---|---|---|---|---|---|
| Antibody | Mean Fluorescence Intensity (MFI) | | | | |
| | Antigen E-ECD | Antigen E-ΔCT | Antigen E-Ala1 | Antigen E-Ala2 | *Mf* Antigen E |
| 2968 | 6559 | 3454 | 14662 | 3388 | 29 |
| 2968G | 2149 | 375 | 9109 | 129 | 22 |
| 2969 | 2014 | 1857 | 7509 | 5671 | 3021 |
| 2969G | 1347 | 610 | 6133 | 4942 | 2513 |
| 2970 | 5518 | 1324 | 14214 | 607 | 32 |
| 2970G | 4683 | 599 | 12321 | 506 | 31 |
| 2971 | 501 | 490 | 2506 | 2017 | 754 |
| 2971G | 578 | 265 | 2457 | 2062 | 724 |
| 2972 | 2164 | 2158 | 8408 | 6409 | 3166 |
| 2972G | 1730 | 992 | 6364 | 4602 | 2146 |
| 2973 | 3527 | 1148 | 3967 | 44 | 84 |
| 2973G | 1294 | 276 | 1603 | 28 | 44 |
| 2974 | 1766 | 722 | 8821 | 241 | 19 |
| 2974G | 2036 | 228 | 8172 | 135 | 26 |
| 2975 | 1990 | 1476 | 8669 | 6134 | 2468 |
| 2975G | 890 | 315 | 4194 | 3987 | 1376 |
| 2976 | 147 | 140 | 996 | 1079 | 181 |
| 2976G | 1365 | 460 | 6024 | 3929 | 1625 |

The anti-Antigen E common light chain antibody supernatants exhibited high specific binding to the beads linked to Antigen E-ECD. For these beads, the negative control mock supernatant resulted in negligible signal (<10 MFI) when combined with the Antigen E-ECD bead sample, whereas the supernatants containing anti-Antigen E common light chain antibodies exhibited strong binding signal (average MFI of 2627 for 98 antibody supernatants; MFI > 500 for 91/98 antibody samples).

As a measure of the ability of the selected anti-Antigen E common light chain antibodies to identify different epitopes on the ECD of Antigen E, the relative binding of the antibodies to the variants were determined. All four Antigen E variants were captured to the anti-myc LUMINEX™ beads as described above for the native Antigen E-ECD binding studies, and the relative binding ratios (MFIᵥₐᵣᵢₐₙₜ/MFI_{Antigen E-ECD}) were determined. For 98 tested common light chain antibody supernatants shown in Tables 12 and 13, the average ratios (MFIᵥₐᵣᵢₐₙₜ/MFI_{Antigen E-ECD}) differed for each variant, likely reflecting different capture amounts of proteins on the beads (average ratios of 0.61, 2.9, 2.0, and 1.0 for Antigen E-ΔCT, Antigen E-Ala1, Antigen E-Ala2, and *Mf* Antigen E, respectively). For each protein variant, the binding for a subset of the 98 tested common light chain antibodies showed greatly reduced binding, indicating sensitivity to the mutation that characterized a given variant. For example, 19 of the common light chain antibody samples bound to the *Mf* Antigen E with MFIᵥₐᵣᵢₐₙₜ/MFI_{Antigen E-ECD} of <8%. Since many in this group include high or moderately high affinity antibodies (5 with K_{D} < 5nM, 15 with K_{D} < 50 nM), it is likely that the lower signal for this group results from sensitivity to the sequence (epitope) differences between native Antigen E-ECD and a given variant rather than from lower affinities.

These data establish that the common light chain antibodies described in Tables 5 and 6 represent a diverse group of Antigen-E-specific common light chain antibodies that specifically recognize more than one epitope on Antigen E.

## Claims

1. A genetically modified mouse comprising a B cell that expresses a human light chain variable (V_{L)} domain derived from a rearranged human Vκ1-39/Jκ sequence that is present in the germline of the mouse, wherein the mouse lacks an unrearranged endogenous immunoglobulin Vκ gene segment and an unrearranged endogenous immunoglobulin JK gene segment; and wherein the human V_{L} domain(s) is associated with a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4.

2. The mouse of claim 1, wherein:
(a) the human Vκ1-39/Jκ sequence is operably linked to a mouse Cκ region sequence;
(b) a sequence encoding the human V_{H} domain is operably linked to a sequence encoding a mouse heavy chain constant (C_{H}) region sequence selected from a C_{H}1, a hinge, a C_{H}2, a C_{H}3, or a combination thereof; or
(c) the human V_{H} domain is expressed from an endogenous immunoglobulin heavy chain locus.

3. The mouse of claim 1, wherein all or substantially all of the mouse Vκ and Jκ gene segments are replaced by the rearranged human Vκ1-39/Jκ sequence.

4. The mouse of claim 1, wherein the rearranged human Vκ1-39/Jκ sequence is operably linked to an immunoglobulin light chain constant region sequence selected from a mouse, rat or human sequence.

5. A genetically modified mouse comprising a B cell that expresses a human light chain variable (V_{L)} domain derived from a rearranged human Vκ3-20/Jκ sequence that is present in the germline of the mouse, wherein the mouse lacks an unrearranged endogenous immunoglobulin Vκ gene segment and an unrearranged endogenous immunoglobulin Jκ gene segment; and wherein the human V_{L} domain(s) is associated with a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4.

6. The mouse of claim 5, wherein:
(a) the human Vκ3-20/Jκ sequence is operably linked to a mouse Cκ region sequence;
(b) a sequence encoding the human V_{H} domain is operably linked to a sequence encoding a mouse C_{H} region sequence selected from a C_{H}1, a hinge, a C_{H}2, a C_{H}3, or a combination thereof; or
(c) the human V_{H} domain is expressed from an endogenous immunoglobulin heavy chain locus.

7. The mouse of claim 5, wherein all or substantially all of the mouse Vκ and Jκ gene segments are replaced by the rearranged human Vκ3-20/Jκ sequence.

8. The mouse of claim 5, wherein the rearranged human Vκ3-20/Jκ sequence is operably linked to an immunoglobulin light chain constant region sequence selected from a mouse, rat or human sequence.

9. The mouse of any of the preceding claims, wherein the mouse comprises a non-functional immunoglobulin lambda (λ) light chain locus.

10. Use of the mouse of any one of claims 1-4 in making an antibody comprising a human heavy chain variable (V_{H}) domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4 associated with a human light chain variable region derived from a rearranged human Vκ1-39/Jκ sequence.

11. The use of claim 10, wherein the antibody is a bispecific antibody.

12. Use of the mouse of any one of claims 5-8 in making an antibody comprising a human heavy chain variable (V_{H}) domain derived from a rearranged human V_{H}/D_{H}/J_{H} region selected from 3-30/3-3/3, 3-33/1-7/4, 3-33/2-15/4 or 3-53/1-1/4 associated with a human light chain variable region derived from a rearranged human Vκ3-20/Jκ sequence.

13. The use of claim 12, wherein the antibody is a bispecific antibody.

14. The use of either one of claims 11 and 13, wherein the production is by a method comprising steps of expressing the human heavy chain variable domains in a single cell.

15. The use of any one of claims 10-14, wherein the heavy chains of the antibody are fully human.

16. The use of any one of claims 10 or 11, wherein heavy chains comprising the human heavy chain variable domains pair with a light chain comprising the human V_{L} domain derived from a rearranged human Vκ1-39/Jκ sequence.

17. The use of any one of claims 12 or 13, wherein heavy chains comprising the human heavy chain variable domains pair with a light chain comprising the human V_{L} domain derived from a rearranged human Vκ3-20/Jκ sequence.

18. The use of any one of claims 10-17, wherein the human heavy chain variable domain(s) are selected by immunizing the mouse with an antigen of interest, determining the sequences of human heavy chain variable domains expressed by the mouse, and expressing the sequences in the cell.

19. A method of producing an antibody to an antigen of interest comprising a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4 associated with a human light chain variable region derived from a rearranged human Vκ1-39/Jκ sequence, the method comprising:
(a) immunizing the mouse of any one of claims 1-4 with an antigen of interest;
(b) obtaining from the mouse a human heavy chain variable domain derived from a rearranged human V_{H}/D_{H}/J_{H} region selected from 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 or 1-69/6-13/4; and
(c) employing the immunoglobulin variable region sequence obtained in (b) associated with said human light chain variable region in an antibody that specifically binds the antigen of interest.

20. A method of producing an antibody to an antigen of interest comprising a human heavy chain variable domain derived from a rearranged human V_{H}/D_{H}/J_{H} region selected from 3-30/3-3/3, 3-33/1-7/4, 3-33/2-15/4 or 3-53/1-1/4 associated with a human light chain variable region derived from a rearranged human Vκ3-20/Jκ sequence, the method comprising:
(a) immunizing the mouse of any one of claims 5-8 with an antigen of interest;
(b) obtaining from the mouse a human heavy chain variable domain derived from a rearranged human V_{H}/D_{H}/J_{H} region selected from 3-30/3-3/3, 3-33/1-7/4, 3-33/2-15/4 or 3-53/1-1/4; and
(c) employing the immunoglobulin variable region sequence obtained in (b) associated with said human light chain variable region in an antibody that specifically binds the antigen of interest.

## Patentansprüche

1. Genetisch modifizierte Maus, umfassend eine B-Zelle, die eine menschliche variable Domäne der leichten Kette (V_{L}), die von einer umgelagerten menschlichen Vκ1-39/Jκ-Sequenz abgeleitet ist, die in der Keimlinie der Maus vorhanden ist, exprimiert, wobei der Maus ein nicht umgelagertes endogenes Immunglobulin-Vκ-Gensegment und ein nicht umgelagertes endogenes Immunglobulin-Jκ-Gensegment fehlt; und wobei die menschliche(n) V_{L}-Domäne(n) mit einer menschlichen variablen Domäne der schweren Kette (V_{H}) assoziiert ist (sind), die von einer umgelagerten menschlichen V_{H}/D_{H}/J_{H}-Region, ausgewählt aus 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 oder 1-69/6-13/4, abgeleitet ist.

2. Maus nach Anspruch 1, wobei:
(a) die menschliche Vκ-39/Jκ-Sequenz operativ mit einer Maus-Cκ-Region-Sequenz verbunden ist;
(b) eine Sequenz, die die menschliche V_{H}-Domäne kodiert, operativ mit einer Sequenz verbunden ist, die eine Maus-Sequenz der konstanten Region der schweren Kette (C_{H}), ausgewählt aus einer C_{H}1, einem Gelenk, einer C_{H}2, einer C_{H}3 oder einer Kombination davon, kodiert; oder
(c) die menschliche V_{H}-Domäne von einem endogenen Immunglobulin-Locus der schweren Kette exprimiert wird.

3. Maus nach Anspruch 1, wobei alle oder im Wesentlichen alle der Maus-Vκ- und Maus-Jκ-Gensegmente durch die umgelagerte menschliche Vκ1-39/Jκ-Sequenz ersetzt werden.

4. Maus nach Anspruch 1, wobei die umgelagerte menschliche Vκ1-39/Jκ-Sequenz operativ mit einer Immunglobulin-Sequenz der konstanten Region der leichten Kette, ausgewählt aus einer Maus-, Ratten- oder menschlichen Sequenz, verbunden ist.

5. Genetisch modifizierte Maus, umfassend eine B-Zelle, die eine menschliche variable Domäne der leichten Kette (V_{L}), die von einer umgelagerten menschlichen Vκ3-20/Jκ-Sequenz abgeleitet ist, die in der Keimlinie der Maus vorhanden ist, exprimiert, wobei der Maus ein nicht umgelagertes endogenes Immunglobulin-Vκ-Gensegment und ein nicht umgelagertes endogenes Immunglobulin-Jκ-Gensegment fehlt; und wobei die menschliche(n) V_{L}-Domäne(n) mit einer menschlichen variablen Domäne der schweren Kette (V_{H}) assoziiert ist (sind), die von einer umgelagerten menschlichen V_{H}/D_{H}/J_{H}-Region, ausgewählt aus 3-30/3-3/3, 3-33/1-7/4, 3-33/2-15/4 oder 3-53/1-1/4, abgeleitet ist.

6. Maus nach Anspruch 5, wobei:
(a) die menschliche Vκ3-20/Jκ-Sequenz operativ mit einer Maus-Cκ-Region-Sequenz verbunden ist;
(b) eine Sequenz, die die menschliche V_{H}-Domäne kodiert, operativ mit einer Sequenz verbunden ist, die eine Maus-C_{H} -Region-Sequenz, ausgewählt aus einer C_{H}1, einem Gelenk, einer C_{H}2, einer C_{H}3 oder einer Kombination davon, kodiert; oder
(c) die menschliche V_{H}-Domäne von einem endogenen Immunglobulin-Locus der schweren Kette exprimiert wird.

7. Maus nach Anspruch 5, wobei alle oder im Wesentlichen alle der Maus-Vκ- und Maus-Jκ-Gensegmente durch die umgelagerte menschliche Vκ3-20/Jκ-Sequenz ersetzt werden.

8. Maus nach Anspruch 5, wobei die umgelagerte menschliche Vκ3-20/Jκ-Sequenz operativ mit einer Immunglobulin-Sequenz der konstanten Region der leichten Ketten, ausgewählt aus einer Maus-, Ratten- oder menschlichen Sequenz, verbunden ist.

9. Maus nach einem der vorhergehenden Ansprüche, wobei die Maus einen nicht-funktionellen Immunglobulin-Lambda(λ)-Locus der leichten Kette umfasst.

10. Verwendung der Maus nach einem der Ansprüche 1-4 beim Herstellen eines Antikörpers, umfassend eine menschliche variable Domäne der schweren Kette (V_{H}), die von einer umgelagerten menschlichen V_{H}/D_{H}/J_{H}-Region, ausgewählt aus 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 oder 1-69/6-13/4 abgeleitet ist, die mit einer menschlichen variablen Region der leichten Kette, die von einer umgelagerten menschlichen Vκ1-39/Jκ-Sequenz abgeleitet ist, assoziiert ist.

11. Verfahren nach Anspruch 10, wobei der Antikörper ein bispezifischer Antikörper ist.

12. Verwendung der Maus nach einem der Ansprüche 5-8 beim Herstellen eines Antikörpers, umfassend eine menschliche variable Domäne der schweren Kette (V_{H}), die von einer umgelagerten menschlichen V_{H}/D_{H}/J_{H}-Region, ausgewählt aus 3-30/3-3/3, 3-33/1-7/4, 3-33/2-15/4 oder 3-53/1-1/4, abgeleitet ist, die mit einer menschlichen variablen Region der leichten Kette, die von einer umgelagerten menschlichen Vκ3-20/Jκ-Sequenz abgeleitet ist, assoziiert ist.

13. Verfahren nach Anspruch 12, wobei der Antikörper ein bispezifischer Antikörper ist.

14. Verwendung nach einem der Ansprüche 11 und 13, wobei die Herstellung durch ein Verfahren, umfassend Schritte eines Exprimierens der menschlichen variablen Domänen der schweren Kette in einer einzelnen Zelle, stattfindet.

15. Verwendung nach einem der Ansprüche 10-14, wobei die schweren Ketten der Antikörper vollständig menschlich sind.

16. Verwendung nach einem der Ansprüche 10 oder 11, wobei sich schwere Ketten, umfassend die menschlichen variablen Domänen der schweren Kette, mit einer leichten Kette, umfassend die menschliche V_{L}-Domäne, die von einer umgelagerten menschlichen Vκ1-39/Jκ-Sequenz abgeleitet ist, paaren.

17. Verwendung nach einem der Ansprüche 12 oder 13, wobei sich schwere Ketten, umfassend die menschlichen variablen Domänen der schweren Kette, mit einer leichten Kette, umfassend die menschliche V_{L}-Domäne, die von einer umgelagerten menschlichen Vκ3-20/Jκ-Sequenz abgeleitet ist, paaren.

18. Verwendung nach einem der Ansprüche 10-17, wobei die menschliche(n) variable(n) Domäne(n) der schweren Kette durch Immunisieren der Maus mit einem Antigen von Interesse, Bestimmen der Sequenzen von menschlichen variablen Domänen der schweren Kette, die durch die Maus exprimiert werden, und Exprimieren der Sequenzen in der Zelle ausgewählt wird (werden).

19. Verfahren eines Herstellens eines Antikörpers an einem Antigen von Interesse, umfassend eine menschliche variable Domäne der schweren Kette, die von einer umgelagerten menschlichen V_{H}/D_{H}/J_{H}-Region, ausgewählt aus 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 oder 1-69/6-13/4, abgeleitet ist, die mit einer menschlichen variablen Region der leichten Kette, die von einer umgelagerten menschlichen Vκ1-39/Jκ-Sequenz abgeleitet ist, assoziiert ist, wobei das Verfahren Folgendes umfasst:
(a) Immunisieren der Maus nach einem der Ansprüche 1-4 mit einem Antigen von Interesse;
(b) Erhalten aus einer Maus einer menschlichen variablen Domäne der schweren Kette, die von einer umgelagerten menschlichen V_{H}/D_{H}/J_{H}-Region, ausgewählt aus 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 oder 1-69/6-13/4, abgeleitet ist; und
(c) Einsetzen der in (b) erhaltenen Immunglobulin-Sequenz der variablen Region, die mit der menschlichen variablen Region der leichten Kette in einem Antikörper assoziiert ist, der spezifisch an das Antigen von Interesse bindet.

20. Verfahren eines Herstellens eines Antikörpers an einem Antigen von Interesse, umfassend eine menschliche variable Domäne der schweren Kette, die von einer umgelagerten menschlichen V_{H}/D_{H}/J_{H}-Region, ausgewählt aus 3-30/3-3/3, 3-33/1-7/4, 3-33/2-15/4 oder 3-53/1-1/4, abgeleitet ist, die mit einer menschlichen variablen Region der leichten Kette, die von einer umgelagerten menschlichen Vκ3-20/Jκ-Sequenz abgeleitet ist, assoziiert ist, wobei das Verfahren Folgendes umfasst:
(a) Immunisieren der Maus nach einem der Ansprüche 5-8 mit einem Antigen von Interesse;
(b) Erhalten aus der Maus einer menschlichen variablen Domäne der schweren Kette, die von einer umgelagerten menschlichen V_{H}/D_{H}/J_{H}-Region, ausgewählt aus 3-30/3-3/3, 3-33/1-7/4, 3-33/2-15/4 oder 3-53/1-1/4, abgeleitet ist; und
(c) Einsetzen der in (b) erhaltenen Immunglobulin-Sequenz der variablen Region, die mit der menschlichen variablen Region der leichten Kette in einem Antikörper assoziiert ist, der spezifisch an das Antigen von Interesse bindet.

## Revendications

1. Souris génétiquement modifiée comprenant une cellule B qui exprime un domaine variable de chaîne légère humain (V_{L}) dérivé d'une séquence Vκ1-39/Jκ humaine réagencée dans la lignée germinale de la souris, la souris étant dépourvue d'un segment de gène Vκ d'immunoglobuline endogène non réagencé et d'un segment de gène Jκ d'immunoglobuline endogène non réagencé ; et l'au moins un domaine V_{L} humain étant associé à un domaine variable de chaîne lourde humain (V_{H}) dérivé d'une région V_{H}/D_{H}/J_{H} humaine réagencée choisie parmi 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 ou 1-69/6-13/4.

2. Souris de la revendication 1, dans laquelle :
(a) la séquence Vκ1-39/Jκ humaine est fonctionnellement liée à une séquence de région Cκ de souris ;
(b) une séquence codant pour le domaine V_{H} humain est fonctionnellement liée à une séquence codant pour une séquence de région constante de chaîne lourde (C_{H}) choisie parmi une C_{H}1, une charnière, une C_{H}2, une C_{H}3 ou une combinaison de celles-ci ; ou
(c) le domaine V_{H} humain est exprimé à partir d'un locus de chaîne lourde d'immunoglobuline endogène.

3. Souris de la revendication 1, dans laquelle tous ou sensiblement tous les segments de gène Vκ et Jκ de la souris sont remplacés par la séquence Vκ1-39/Jκ humaine réagencée.

4. Souris de la revendication 1, dans laquelle la séquence Vκ1-39/Jκ humaine réagencée est fonctionnellement liée à une séquence de région constante de chaîne légère d'immunoglobuline choisie parmi une séquence de souris, de rat ou humaine.

5. Souris génétiquement modifiée comprenant une cellule B qui exprime un domaine variable de chaîne légère humain (V_{L}) dérivé d'une séquence Vκ3-20/Jκ humaine réagencée dans la lignée germinale de la souris, la souris étant dépourvue d'un segment de gène Vκ d'immunoglobuline endogène non réagencé et d'un segment de gène Jκ d'immunoglobuline endogène non réagencé ; et l'au moins un domaine V_{L} humain étant associé à un domaine variable de chaîne lourde humain (V_{H}) dérivé d'une région V_{H}/D_{H}/J_{H} humaine réagencée choisie parmi 3-30/3-3/3, 3-33/1-7/4, 3-33/2-15/4 ou 3-53/1-1/4.

6. Souris de la revendication 5, dans laquelle :
(a) la séquence Vκ3-20/Jκ humaine et fonctionnellement liée à une séquence de région Cκ de souris ;
(b) une séquence codant pour le domaine V_{H} humain est fonctionnellement liée à une séquence codant pour une séquence de région C_{H} de souris choisie parmi une C_{H}1, une charnière, une C_{H}2, une C_{H}3 ou une combinaison de celles-ci ; ou
(c) le domaine V_{H} humain est exprimé à partir d'un locus de chaîne lourde d'immunoglobuline endogène.

7. Souris de la revendication 5, dans laquelle tous ou sensiblement tous les segments de gène Vκ et Jκ de souris sont remplacés par la séquence Vκ3-20/Jκ humaine réagencée.

8. Souris de la revendication 5, dans laquelle la séquence Vκ3-20/Jκ humaine réagencée est fonctionnellement liée à une séquence de région constante de chaîne légère d'immunoglobuline choisie parmi une séquence de souris, de rat ou humaine.

9. Souris de l'une quelconque es revendications précédentes, la souris comprenant un locus de chaîne légère lambda d'immunoglobuline non fonctionnel (λ).

10. Utilisation de la souris de l'une quelconque des revendications 1 à 4, dans la fabrication d'un anticorps comprenant un domaine variable de chaîne lourde humain (V_{H}) dérivé d'une région V_{H}/D_{H}/J_{H} humaine réagencée choisie parmi 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 ou 1-69/6-13/4 associée à une région variable de chaîne légère humaine dérivée d'une séquence Vκ1-39/Jκ humaine réagencée.

11. Utilisation de la revendication 10, dans laquelle l'anticorps est un anticorps bispécifique.

12. Utilisation de la souris de l'une quelconque des revendications 5 à 8, dans la fabrication d'un anticorps comprenant un domaine variable de chaîne lourde humain (V_{H}) dérivé d'une région V_{H}/D_{H}/J_{H} humaine réagencée choisie parmi 3-30/3-3/3, 3-33/1-7/4, 3-33/2-15/4 or 3-53/1-1/4 associée à une région variable de chaîne légère humaine dérivée d'une séquence Vκ3-20/Jκ humaine réagencée.

13. Utilisation de la revendication 12, dans laquelle l'anticorps est un anticorps bispécifique.

14. Utilisation de l'une ou l'autre des revendications 11 et 13, dans laquelle la production est effectuée par un procédé comprenant des étapes consistant à exprimer les domaines variables de chaîne lourde humaines dans une cellule unique.

15. Utilisation de l'une quelconque des revendications 10 à 14, dans laquelle les chaînes lourdes de l'anticorps sont entièrement humaines.

16. Utilisation de l'une quelconque des revendications 10 ou 11, dans laquelle des chaînes lourdes comprenant les domaines variables de chaîne lourde humains s'apparient avec une chaîne légère comprenant le domaine V_{L} humain dérivé d'une séquence Vκ1-39/Jκ humaine réagencée.

17. Utilisation de l'une quelconque des revendications 12 ou 13, dans laquelle des chaînes lourdes comprenant les domaines variables de chaîne lourde humains s'apparient avec une chaîne légère comprenant le domaine V_{L} humain dérivé d'une séquence Vκ3-20/Jκ humaine réagencée.

18. Utilisation de l'une quelconque des revendications 10 à 17, dans laquelle l'au moins un domaine variable de chaîne lourde humain est choisi par l'immunisation de la souris avec un antigène d'intérêt, la détermination des séquence des domaines variables de chaîne lourde humains exprimés par la souris et l'expression des séquences dans la cellule.

19. Procédé de production d'un anticorps pour un antigène d'intérêt comprenant un domaine variable de chaîne lourde humain dérivé d'une région V_{H}/D_{H}/J_{H} humaine réagencée choisie parmi 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 ou 1-69/6-13/4 associée à une région variable de chaîne légère humaine dérivée d'une séquence Vκ1-39/Jκ humaine réagencée, le procédé comprenant :
(a) l'immunisation de la souris de l'une quelconque des revendications 1 à 4 avec un antigène d'intérêt ;
(b) l'obtention à partir de la souris d'un domaine variable de chaîne lourde humain dérivé d'une région V_{H}/D_{H}/J_{H} humaine réagencée choisie parmi 2-5/3-22/1, 3-13/6-6/5, 3-23/2-8/4, 3-23/6-6/4, 3-23/7-27/4, 3-30/1-1/4, 3-30/3-3/4, 3-30/5-5/2, 3-30/7-27/6, 1-69/6-6/5 ou 1-69/6-13/4 ; et
(c) l'emploi de la séquence de région variable d'immunoglobuline obtenue en (b) associée à ladite région variable de chaîne légère humaine dans un anticorps qui se lie spécifiquement à l'antigène d'intérêt.

20. Procédé de production d'un anticorps pour un antigène d'intérêt comprenant un domaine variable de chaîne lourde humain dérivé d'une région V_{H}/D_{H}/J_{H} humaine réagencée choisie parmi 3-30/3-3/3, 3-33/1-7/4, 3-33/2-15/4 ou 3-53/1-1/4 associée à une région variable de chaîne légère humaine dérivée d'une séquence Vκ3-20/Jκ humaine réagencée, le procédé comprenant :
(a) l'immunisation de la souris de l'une quelconque des revendications 5 à 8 avec un antigène d'intérêt ;
(b) l'obtention à partir de la souris d'un domaine variable de chaîne lourde humain dérivé d'une région V_{H}/D_{H}/J_{H} humaine réagencée choisie parmi 3-30/3-3/3, 3-33/1-7/4, 3-33/2-15/4 ou 3- 53/1-1/4 ; et
(c) l'emploi de la séquence de région variable d'immunoglobuline obtenue en (b) associée à ladite région variable de chaîne légère humaine dans un anticorps qui se lie spécifiquement à l'antigène d'intérêt.
